(19) Europäisches Patentamt
European Patent Office
Office européen des brevets

(11) **EP 2 376 223 B1**

(12) **EUROPÄISCHE PATENTSCHRIFT**

(45) Veröffentlichungstag und Bekanntmachung des Hinweises auf die Patenterteilung:
**16.03.2016 Patentblatt 2016/11**

(21) Anmeldenummer: **09768363.5**

(22) Anmeldetag: **04.12.2009**

(51) Int Cl.:
*B01J 23/28* (2006.01)      *B01J 23/31* (2006.01)
*B01J 23/88* (2006.01)      *B01J 23/881* (2006.01)
*B01J 23/882* (2006.01)      *B01J 23/883* (2006.01)

(86) Internationale Anmeldenummer:
**PCT/EP2009/066430**

(87) Internationale Veröffentlichungsnummer:
**WO 2010/066645 (17.06.2010 Gazette 2010/24)**

(54) **VERFAHREN ZUR KONTINUIERLICHEN HERSTELLUNG VON GEOMETRISCHEN KATALYSATORFORMKÖRPERN K**

METHOD FOR CONTINUOUSLY MANUFACTURING GEOMETRIC CATALYST MOLDED BODIES K

PROCÉDÉ DE PRODUCTION EN CONTINU DE CATALYSEURS MOULÉS DE FORME GÉOMÉTRIQUE K

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **12.12.2008 DE 102008054586**
**12.12.2008 US 122129 P**

(43) Veröffentlichungstag der Anmeldung:
**19.10.2011 Patentblatt 2011/42**

(73) Patentinhaber: **BASF SE**
**67056 Ludwigshafen (DE)**

(72) Erfinder:
• **RAICHLE, Andreas**
**01109 Dresden (DE)**
• **BORCHERT, Holger**
**67591 Offstein (DE)**
• **MÜLLER-ENGEL, Klaus Joachim**
**76297 Stutensee (DE)**
• **HORSTMANN, Catharina**
**67056 Ludwigshafen (DE)**
• **MACHT, Josef**
**68159 Mannheim (DE)**

(56) Entgegenhaltungen:
EP-A1- 1 060 792      EP-A1- 1 726 358
WO-A1-2005/049200      WO-A2-02/24620
DE-A1- 19 948 523      JP-A- 10 029 097

**Beschreibung**

Vorliegende Erfindung betrifft ein Verfahren zur kontinuierlichen Herstellung von geometrischen Katalysatorformkörpern K,

die als Aktivmasse ein Multielementoxid enthalten, das als von Sauerstoff verschiedene Elemente E das Element Mo, wenigstens eines der beiden Elemente Bi und V sowie wenigstens ein weiteres Element aus der Gruppe bestehend aus Co, Ni, Fe, Cu und den Alkalimetallen enthält, in Verfahrerisstufen A) bis E), bei dem man

- in der Verfahrensstufe A) mit Hilfe von Quellen Q der Elemente E eine feinteilige Mischung M mit der Maßgabe erzeugt, dass höchstens 10 Gew.-% des Gesamtgewichts der in der feinteiligen Mischung M enthaltenen Partikel einen Partikeldurchmesser $d^M \geq 160$ μm aufweisen und der Partikeldurchmesser $d^M_{50}$ der Partikel der Mischung M die Bedingung $1\ \mathrm{\mu m} \leq d^M_{50} \leq 150\ \mathrm{\mu m}$ erfüllt;

- in der Verfahrensstufe B) das feinteilige Gemisch M*, das entweder nur aus der feinteiligen Mischung M oder aus einer Mischung aus der feinteiligen Mischung M und in der nachfolgenden Verfahrensstufe C) anfallendem und aus der Verfahrensstufe C) absatzweise oder kontinuierlich in die Verfahrensstufe B) rückgeführtem Feinkorn F besteht, durch Pressagglomeration, bei der der höchste angewandte Pressdruck P1 ist, zu Agglomeraten A verdichtet, deren Längstausdehnung L ≥ 3 mm beträgt;

- in der Verfahrensstufe C) die Agglomerate A zerkleinert und das bei der Zerkleinerung gebildete körnige Gut durch Sieben in ein Pulver P, dessen Partikeldurchmesser $d^P \leq 2$ mm und zu wenigstens 90 Gew.-%, bezogen auf das Gewicht des Pulvers P, ≥ 160 μm betragen, als Sieböberkorn, und in Feinkorn F als Siebunterkorn auftrennt und das Feinkorn F kontinuierlich oder absatzweise zur Erzeugung von feinteiligem Gemisch M* in die Verfahrensstufe B rückführt;

- in der Verfahrensstufe D) aus dem in selbige geförderten Pulver P oder aus einem Gemisch P*, bestehend aus dem in die Verfahrensstufe D) geförderten Pulver P und Formgebungshilfsmittel, durch Pressagglomeration, bei der der angewandte höchste Pressdruck P2 beträgt und die Beziehung P2 ≥ 2 · P1 erfüllt, mit der Maßgabe geometrische Formkörper V erzeugt, dass

- bei der Förderung des Pulvers P in die Verfahrensstufe D) und beim Einmischen von Formgebungshilfsmittel in das Pulver P insgesamt bei wenigstens 40 Gew.-% (vorzugsweise wenigstens 60 Gew.-%, besonders bevorzugt wenigstens 80 Gew.-% oder wenigstens 90 New.-% bzw. 100 Gew.-%), der Partikel des Pulvers P, bezogen auf dessen Gewicht, ein Partikeldurchmesser $d^P \geq 160$ μm erhalten bleibt; und

- in der Verfahrensstufe E) wenigstens eine Teilmenge der Formkörper V bei erhöhter Temperatur unter Erhalt der geometrischen Katalysatorformkörper K thermisch behandelt.

Verfahren zur Herstellung von geometrischen Katalysatorformkörpern, die als Aktivmasse ein Multielementoxid enthalten, das als von Sauerstoff verschiedene Elemente das Element Mo, wenigstens eines der beiden Elemente Bi und V sowie wenigstens ein weiteres Element aus der Gruppe bestehend aus Co, Ni, Fe, Cu und den Alkalimetallen enthält, sind bekannt (vgl. z. B. EP-A 184 790, US-A 2005/0263926 sowie die JP-A 10/29097):

In der Regel wird dabei mit Hilfe von Quellen der von Sauerstoff verschiedenen Elemente des Multielementoxids (Quellen = Ausgangsverbindungen, die wenigstens eines der Elemente enthalten und bei denen es sich entweder bereits um Oxide handelt, oder um solche Verbindungen, die durch thermische Behandlung bei erhöhter Temperatur, wenigstens im Beisein von molekularem Sauerstoff, in Oxide überführt werden) eine feinteilige innige Mischung erzeugt, die die von Sauerstoff verschiedenen Elemente des Multielementoxids in der erforderlichen Stöchiometrie enthält. Durch Pressagglomeration werden aus der feinteiligen innigen Mischung nachfolgend geometrische Formkörper (Katalysatorvorläuferformkörper) der gewünschten Geometrie geformt. Durch thermische Behandlung der erhaltenen Katalysatorvorläuferformkörper wird dann aus selbigen der erwünschte geometrische Katalysatorformkörper erhalten.

Verwendung finden solche geometrischen Katalysatorformkörper beispielsweise zur (gegebenenfalls mit inerten Formkörpern verdünnten) Beschickung des Innenraums der Reaktionsrohre eines Rohrbündelreaktors mit einem Katalysatorfestbett. Ein solches Katalysatorfestbett eignet sich unter anderem zur Durchführung von heterogen katalysierten Gasphasenreaktionen (z. B. Partialoxidationen organischer Verbindungen). An Stelle eines Rohrbündelreaktors

EP 2 376 223 B1

kann auch ein Thermoplattenreaktor beschickt werden.

Das entsprechende Reaktionsgasgemisch strömt durch das Katalysatorfestbett und während der Verweilzeit an der Katalysatoroberfläche erfolgt die gewünschte Reaktion.

Nachteilig an durch mechanisches Verdichten eines pulverförmigen Haufwerks erzeugten Formkörpern ist ganz generell, dass der Zusammenhalt des Pulverkorns im resultierenden Formkörper im Wesentlichen nicht durch intramolekulare chemische Bedingungen, sondern durch bleibende interpartikuläre Bindungen bewerkstelligt wird. Zwar resultiert aus Partikelverformungen und Bruchvorgängen beim Verdichtungsvorgang in der Regel eine Zunahme der interpartikulären Gesamtkontaktfläche, dennoch ist der Betrag der durch das Verdichten erzeugten interpartikulären Bindungskräfte ein vergleichsweise beschränkter.

Als Folgewirkung weisen wie beschrieben erzeugte Katalysatorvorläuferformkörper teilweise Beschädigungen wie z. B. Risse auf, die häufig visuell kaum wahrnehmbar sind. Bei einer nachfolgenden thermischen Behandlung solcher Vorläuferformkörper, im Rahmen derer im Katalysatorvorläuferformkörper in der Regel auch gasförmige Verbindungen freigesetzt werden, die auf bei der thermischen Behandlung sich zersetzende und/oder chemisch umsetzende Bestandteile zurückzuführen sind, nimmt eine bereits vorhandene Beschädigung wie z. B. Rissbildung in der Regel noch zu und entwickelt sich vielfach bis zum Bruch. In einem Katalysatorfestbett enthaltene Katalysatorbruchstücke bedingen jedoch eine Verdichtung desselben und verursachen beim das selbige durchströmenden Reaktionsgasgemisch letztlich eine Erhöhung des beim Durchströmen erlittenen Druckverlustes.

Eine Gegenmaßnahme, die zur Minderung des vorstehend beschriebenen Erscheinungsbildes ergriffen werden kann, besteht z. B. darin, vorab der Einbringung der erzeugten geometrischen Katalysatorformkörper K in das Katalysatorfestbett, den im Rahmen ihrer Herstellung entstandenen Bruch abzusieben (vgl. z. B. US-B 7,147,011 und die DE-A 10 2007 028 332). Nachteilig an einer solchen Verfahrensweise ist jedoch, dass die Rohstoffkosten für eine großtechnische Herstellung von Katalysatorformkörpern nicht unerheblich sind, weshalb der beim Sieben als Siebdurchgang (Siebunterkorn) anfallende Katalysatorbruch einen nicht unerheblichen materiellen Verlust bedeutet.

Darüber hinaus ist die Maßnahme der Siebabtrennung von Katalysatorbruch dann nicht anwendbar, wenn die thermische Behandlung der Katalysatorvorläuferformkörper bereits im Reaktor (z. B. im Reaktionsrohr) befindlich vorgenommen (z. B. durch Durchleiten von entsprechend erhitzten Gasen durch die mit den Vorläuferformkörpern beschickten Reaktionsrohre) wird.

Neben der möglichen Maßnahme einer Siebabtrennung von gebildetem Katalysatorbruch besteht als Abhilfe für die vorstehend beschriebene Druckverlustproblematik grundsätzlich auch die Möglichkeit, Maßnahmen zu ergreifen, die das Auftreten von Katalysatorbruch mindern. Als solche Maßnahmen werden im Stand der Technik z. B. die Mitverwendung von Formgebungshilfsmitteln wie z. B. Graphit sowie die Anwendung von geschickt ausgestalteten Matrizen bei der Formgebung empfohlen (vgl. z. B. WO 2005/049200 A1, DE-A 10 2008 040 093 sowie DE-A 10 2008 040 094).

Nachteilig an diesen Hilfsmaßnahmen ist jedoch, dass sie dem beschriebenen Problem nicht auf eine im vollen Umfang befriedigende Art und Weise abzuhelfen vermögen (das Auftreten von Katalysatorbruch wird durch die beschriebenen Maßnahmen nicht vollständig unterdrückt und außerdem bedarf es der Verwendung spezieller Formgebungsmatrizen).

Die Aufgabe der vorliegenden Erfindung bestand daher darin, ein verbessertes Verfahren zur kontinuierlichen Herstellung von geometrischen Katalysatorformkörpern K zur Verfügung zu stellen, das die beschriebenen Nachteile allenfalls noch in vermindertem Umfang aufweist.

Eingehende Untersuchungen haben zum Ergebnis geführt, dass die angestrebte Verbesserung dadurch erzielt werden kann, dass man nach der Verfahrensstufe D) und vorab der Verfahrensstufe E) die in der Verfahrensstufe D) erzeugten Formkörper V in einer Trennstufe als Verfahrensstufe F) in nicht intakte Formkörper V- und in intakte Formkörper V+ auftrennt und im Wesentlichen nur letztere der Verfahrensstufe E) zuführt. Die Vorteilhaftigkeit der genannten Trennmaßnahmen besteht zum einen darin, das der letztendlich erzeugte Anteil an Katalysatorbruch durch sie vermindert werden kann. Insbesondere besteht sie aber darin, dass, im Unterschied zu Katalysatorbruch, wie beschrieben abgetrennte Formkörper V- in das Verfahren der Herstellung von geometrischen Katalysatorformkörpern K rückgeführt werden können (ohne die Performance der dabei resultierenden geometrischen Katalysatorformkörper K nennenswert zu mindern), und somit keinen materiellen Verlust bedeuten. Nämlich dann, wenn man die nicht intakten Formkörper V- in einer Verfahrensstufe G) unter Ausbildung eines feinteiligen Haufwerks H, dessen Partikeldurchmesser $d_{50}^H$ die Bedingung

$1\ \mu m \leq d_{50}^H \leq 150\ \mu m$ erfüllt und das zu höchstens 10 Gew.-% seines Gesamtgewichts Partikel mit einem Partikeldurchmesser $d^H \geq 160\ \mu m$ enthält, zerkleinert, und das feinteilige Haufwerk H kontinuierlich oder absatzweise zur zusätzlichen Einarbeitung in die der Pressagglomeration zu unterwerfende feinteilige Mischung M* zurückführt und dabei darauf achtet, dass der Gesamtanteil des feinteiligen Haufwerks H an der feinteiligen Mischung M* einen Höchstwert von 20 Gew.-% nicht überschreitet.

Die vorstehende Gehaltsbeschränkung ist insbesondere deshalb von Relevanz, weil das im wie beschriebenen rückgeführten Haufwerk H enthaltene Material im Rahmen des Gesamtverfahrens eine Mehrfachverdichtung erfährt, die die Performance der resultierenden Katalysatorformkörper K gemäß eigener Untersuchungen nicht begünstigt.

Während es für die katalytischen Eigenschaften der in den Katalysatorformkörpern K enthaltenen Multielement-toxidaktivmasse von Vorteil ist, wenn zur Herstellung der Formkörper V von einer möglichst feinteiligen Mischung vergleichsweise homogener Ausprägung ausgegangen wird, ist es für die Fließeigenschaften der zu verdichtenden Mischung günstiger, wenn sie auch gröbere Anteile enthält (vgl. WO 2008/014839). Anwendungstechnisch zweckmäßig wird deshalb bei der Herstellung von geometrischen Katalysatorformkörpern K von vergleichsweise feinteiligen Ausgangsgemischen ausgegangen, die nachfolgend durch eine erste Pressagglomeration mit nachfolgender Zerkleinerung zunächst nur vergröbert werden, um ihre Fließeigenschaften zu verbessern. Letzteres gewährleistet beispielsweise eine reproduzierbarere Befüllung des Matrizenhohlraums ("das Pulver füllt ihn wie eine Flüssigkeit aus"), in welchem dann erst die Verdichtung zum Formkörper V erfolgt. Da der im Rahmen der Verdichtung zum Formkörper V angewandte maximale Pressdruck wesentlich größer ist, als derjenige, der zur Pulververgröberung angewendet wird, bedarf es im feinteiligen Gemisch M* keiner Beschränkung des Mengenanteiles hinsichtlich einer Materialrückführung aus der Pulververgröberung.

Als Lösung der erfindungsgemäßen Aufgabe wird somit ein Verfahren zur kontinuierlichen Herstellung von geometrischen Katalysatorformkörpern K (Vollkatalysatoren K), die als Aktivmasse ein Multielementoxid enthalten, das als von Sauerstoff verschiedene Elemente das Element Mo, wenigstens eines der beiden Elemente Bi und V sowie wenigstens ein weiteres Element aus der Gruppe bestehend aus Co, Ni, Fe, Cu und den Alkalimetallen enthält, in Verfahrensstufen A) bis G) bei dem man

- in der Verfahrensstufe A) mit Hilfe von Quellen Q der Elemente E eine feinteilige Mischung M mit der Maßgabe erzeugt, dass höchstens 10 Gew.-% des Gesamtgewichts der in der feinteiligen Mischung M enthaltenen Partikel einen Partikeldurchmesser $d^M \geq 160 \ \mu m$ aufweisen und der Partikeldurchmesser $d_{50}^M$ der Partikel der feinteiligen Mischung M die Bedingung $1 \ \mu m \leq d_{50}^M \leq 150 \ \mu m$ erfüllt;

- in der Verfahrensstufe B) das feinteilige Gemisch M*, das entweder nur aus der feinteiligen Mischung M oder aus einer Mischung aus der feinteiligen Mischung M und in der nachfolgenden Verfahrensstufe C) anfallendem und aus der Verfahrensstufe C) absatzweise oder kontinuierlich in die Verfahrensstufe B) rückgeführtem Feinkorn F besteht, durch Pressagglomeration, bei der der höchste angewandte Pressdruck P1 ist, zu Agglomeraten A verdichtet, deren Längstausdehnung $L \geq 3$ mm beträgt;

- in der Verfahrensstufe C) die Agglomerate A zerkleinert und das bei der Zerkleinerung gebildete körnige Gut durch Sieben in ein Pulver P, dessen Partikeldurchmesser $d^P \leq 2$ mm und zu wenigstens 90 Gew.-% (vorzugsweise zu wenigstens 95 Gew-% oder zu 100 Gew.-%), bezogen auf das Gewicht des Pulvers P, $\geq 160 \ \mu m$ betragen, als Siebüberkorn, und in Feinkorn F als Siebunterkorn auftrennt und das Feinkorn F kontinuierlich oder absatzweise zur Erzeugung von feinteiligem Gemisch M* in die Verfahrensstufe B rückführt;

- in der Verfahrensstufe D) aus dem in selbige geförderten Pulver P oder aus einem Gemisch P*, bestehend aus dem in die Verfahrensstufe D) geförderten Pulver P und Formgebungshilfsmittel, durch Pressagglomeration, bei der der angewandte höchste Pressdruck P2 beträgt und die Beziehung P2 ≥ 2 • P1 erfüllt, mit der Maßgabe geometrische Formkörper V erzeugt, dass

- bei der Förderung des Pulvers P in die Verfahrensstufe D) und beim Einmischen von Formgebungshilfsmittel in das Pulver P insgesamt bei wenigstens 40 Gew.-% (vorzugsweise wenigstens 60 Gew.-%, besonders bevorzugt wenigstens 80 Gew.-% oder wenigstens 90 Gew.-% bzw. 100 Gew.-%), der Partikel des Pulvers P, bezogen auf dessen Gewicht, ein Partikeldurchmesser $d^P \geq 160 \ \mu m$ erhalten bleibt; und

- in der Verfahrensstufe E) wenigstens eine Teilmenge der Formkörper V bei erhöhter Temperatur unter Erhalt der geometrischen Katalysatorformkörper K thermisch behandelt,
zur Verfügung gestellt, das dadurch gekennzeichnet ist, dass man

- vorab der Verfahrensstufe E) die in der Verfahrensstufe D) erzeugten Formkörper V in einer zusätzlichen Trennstufe als Verfahrensstufe F) in nicht intakte Formkörper V⁻ sowie in intakte (unversehrte, unbeschädigte, heile) Formkörper V⁺ auftrennt, die Formkörper V⁺ der Verfahrensstufe E) zuführt und

- in der Verfahrensstufe G) nicht intakte Formkörper V⁻ unter Ausbildung eines feinteiligen Haufwerks H, dessen Partikeldurchmesser $d_{50}^H$ die Bedingung $1 \ \mu m \leq d_{50}^H \leq 150 \ \mu m$ erfüllt und das zu höchstens 10 Gew.-% seines Gesamtgewichts Partikel mit einem Partikeldurchmesser $d^H \geq 160$ $\mu$m enthält, zerkleinert und das feinteilige Haufwerk H kontinuierlich oder absatzweise zur zusätzlichen Einarbeitung in das feinteilige Gemisch M* mit der Maßgabe in die Verfahrensstufe B) rückführt, dass der Gehalt des feinteiligen Gemischs M* an feinteiligem Haufwerk H, bezogen auf das Gesamtgewicht des feinteiligen Gemischs M*, 20 Gew.-% nicht überschreitet (und ansonsten die Verfahrensstufen A) bis G) unverändert, d. h., unter unveränderter Anwendung der in diesen anzuwendenden Verfahrensmaßnahmen, ausübt).

Die Auftrennung der Formkörper V in der Verfahrensstufe F) in nicht intakte Formkörper V- sowie in intakte Formkörper V+ kann grundsätzlich durch visuelle Betrachtung der erzeugten Formkörper V (auch "Grünlinge" V genannt) sowie sich daran anschließendes Auslesen (Aussortiern) erfolgen.

Anwendungstechnisch zweckmäßig wird die Auftrennung der Formkörper V jedoch durch eine Siebtrennung vorgenommen. Im Rahmen einer solchen Siebung zerbrechen in der Regel z. B. jene Formkörper V, die bereits eine Rissbildung aufweisen oder in denen bereits eine Rissbildung angelegt ist (die nicht "intakt" sind). Die intakten Formkörper V+ verbleiben als Siebrückstand (auch als "Überkorn" bezeichnet), während der Siebdurchgang (auch als "Unterkorn" bezeichnet) die Bruchstücke von nicht intakten Formkörpern V- umfasst.

Grundsätzlich kann der Transport der erfindungsgemäß hergestellten Formkörper V ("das Siebgut") über das Sieb (der Begriff "Sieb" wird in dieser Schrift synonym mit dem Begriff "Siebboden" verwendet; im Übrigen wird der Begriff "Sieb" oder "Siebboden" in dieser Schrift im Sinne der in der EP-A 1 726 358 in Spalte 5, Zeilen 48 bis 57 gegebenen Begriffsdefinition verwendet) durch eine kreis-, ellipsen- und/oder linearförmige Schwingbewegung des Siebbodens erfolgen. Zu diesem Zweck können für ein erfindungsgemäßes Verfahren prinzipiell alle in z. B. Chem.-Ing.-Tech. 56 (1984) Nr. 12, S. 897 bis 907 sowie in Sieben und Siebmaschinen, Grundlagen und Anwendung, Wiley VCH, Paul Schmidt (2003) empfohlenen Siebmaschinen eingesetzt werden.

Eine für die erfindungsgemäße Auftrennung der Formkörper V gut geeignete Gruppe von Siebmaschinen sind die Plansiebe, bei denen das Siebgut als Siebgutmatte linear oder kreisend auf dem Sieb (dem Siebboden) gleitet. Durch das Eigengewicht und die Reibung gegen das Sieb wird die Siebgutmatte geschert. Von Vorteil ist die sehr geringe, meist negativ wirkende Rückvermischung.

Die Schwingbewegung der Siebfläche erfolgt bei Plansieben in ihrer Siebebene. Die Schwingbewegung kann linear (hin und her) oder kreisförmig verlaufen (im ersten Fall spricht man von einem linearen Planschwingsieb). Im ersteren Fall kann sie in Förderrichtung oder quer dazu erfolgen. Durch asymmetrische Beschleunigung kann bei linearer Schwingbewegung in Förderrichtung auch beim horizontalen Sieb der Längstransport des Siebgutes bewirkt werden.

Die Kreisschwingung bietet den Vorteil, stetig eine optimale Beschleunigung aufrechtzuerhalten. Selbstverständlich ist beim erfindungsgemäßen Verfahren auch eine Kombination aus Linear- und Kreisschwinger anwendbar.

Bei Kreisschwingern wird die horizontale Kreisbewegung häufig durch einen Getriebemotor erzeugt. Bei Linearschwingern wird der ganze Siebrahmen (in welchem sich der Siebboden normalerweise ganz generell eingebracht befindet) durch gegenläufige Unwuchtmassen in eine Linearschwingung versetzt. Linearschwinger können sowohl mit horizontalem, als auch mit geneigtem Siebboden angewendet werden. Beim geneigten Siebboden wird das Siebgut durch entsprechende Neigung der Schwingungsebene gegen den Siebboden, entsprechend einer Wurfparabel, aufwärts und zugleich vorwärts geworfen. Die Neigungswinkel können z. B. von -3° bis 25° betragen. 3° bis 4° sind erfindungsgemäß bevorzugt. Erfindungsgemäß besonders bevorzugt sind zum Beispiel Linearschwingsiebe der Firma RHEWURM GmbH in D-Remscheid. Rechtecksiebmaschinen sind für einen erfindungsgemäßen Plansiebbetrieb gegenüber Rundsieben bevorzugt. Bei ihnen sind rechteckige Siebböden in einen gleichfalls rechteckigen Siebrahmen eingebracht. Häufig wird die Schwingbewegung so gestaltet, dass der Siebrückstand zum Umfang des Siebes hingetragen und dort ausgetragen wird.

Um die Sieböffnungen im Verlauf einer erfindungsgemäßen Siebung von Formkörpern V freizuhalten, kann, z. B. dann, wenn der Siebboden aus Stahl mit vergleichsweise geringem Elastizitätsmodul gefertigt ist, die Methode der Gummiballklopfung eingesetzt werden(vgl. Abbildung 12 in Chem.-Ing.-Tech. 56 (1984) Nr. 12, Seite 902). Dabei werden Gummibälle auf einen Blindboden gegeben, der sich in einem festen Abstand unter dem Sieb (dem Siebboden) befindet. Die Gummibälle springen auch bei Plansiebmaschinen während des Siebvorgangs (während der Siebung) von unten gegen das Sieb und reinigen das Sieb örtlich. Ihre Elastizität ist so bemessen, dass sie im Wesentlichen keinen zusätzlichen Bruch des intakten Siebgutes bedingen. Der Blindboden ist meist ein Lochblech, mit vorzugsweise quadratischen Lochöffnungen. In jedem Fall sind die Lochöffnungen des Blindbodens so beschaffen, dass der Siebdurchgang passieren kann.

Alternativ zu einer Gummiballklopfung kann eine Siebreinigung während des Siebvorgangs auch durch über und/oder unter dem Siebboden angeordnete Flach- oder Rollenbürsten kontinuierlich bewirkt werden.

Die Wahl der jeweiligen für die erfindungsgemäße Auftrennung anzuwendenden Sieböffnung orientiert sich an der jeweiligen Geometrie der Formkörper V. Dabei können z. B. die in den Schriften US-B 7,147,011, DE-A 102 007 028 332, EP-A 1 726 358, Aufbereitungstechnik-Nr. 11/1960, S. 457 bis 473, Chem.-Ing.-Techn. 56 (1984) Nr. 12, S.

897 bis 907 sowie in "Sieben und Siebmaschinen, Wiley-VCH GmbH & Co. KGaA, Paul Schmidt et al (2003)" gegebenen Empfehlung befolgt werden.

D. h., der verwendete Siebboden kann beispielsweise als Gitter bzw. Rost, als Loch- bzw. Spaltblech (d. h., als Blech mit gestanzten, gelaserten, wassergeschnittenen, oder gefrästen Sieböffnungen) oder als Siebgewebe (es besteht aus miteinander verwobenen Drähten, wobei die Drähte rund oder profiliert sein können) ausgebildet sein. Gitter bzw. Roste sowie Siebgewebe eignen sich insbesondere im Fall von nur eine erfindungsgemäße Sorte einer rechteckigen Sieböffnung aufweisenden Siebböden. Beliebige Sieböffnungen können auf einfache Weise in Loch- bzw. Spaltblechen verwirklicht werden. Erfindungsgemäß vorteilhafte Loch- und Spaltbleche sind solche, die lediglich eine Sorte einer rechteckigen bzw. einer Langlochform aufweisenden Sieböffnung aufweisen. Typische Blechdicken von erfindungsgemäß verwendbaren Lochblechsieben (bzw. Spaltblechsieben) betragen 1 bis 5 mm, vorzugsweise 1 bis 3 mm, besonders bevorzugt 1 bis 2 mm.

Die freie Siebfläche F (die Gesamt(querschnitts)fläche aller in einem Spaltblechsiebboden befindlichen Sieböffnungen) von erfindungsgemäß günstigen Spaltblechsiebböden wird, bezogen auf die Gesamtfläche des Spaltblechsiebbodens, in typischer Weise 10 bis 60 %, vorzugsweise 20 bis 50 % und besonders bevorzugt 30 bis 50 % betragen.

Als Werkstoff kommt insbesondere Stahl (z. B. die DIN-Werkstoffe 1.4541 oder 1.4571) in Betracht. Es kann aber auch Gummi oder Kunststoff verwendet werden.

Den Differenzierungsgrad zwischen intakten und nicht intakten Formkörpern V legt die jeweils angewandte Trennmethode fest. Der Begriff intakt ist dabei in dieser Schrift im Sinn von unversehrt, unbeschädigt oder heil zu verstehen. Wesentlich bei einer durch Sieben bewirkten Auftrennung in intakte Formkörper V+ und nicht intakte Formkörper V⁻ ist zusätzlich, dass beim Siebvorgang an den Formkörpern V herstellungsbedingt noch anhaftender Pulverstaub zusätzlich in nennenswertem Umfang mitabgetrennt wird. Enthält vorgenannter Pulverstaub entzündliche Bestandteile wie z. B. Graphit, kann es im Fall einer Nichtabtrennung des Pulverstaubs von den Formkörpern V bei deren thermischer Behandlung zu unerwünschten Entzündungserscheinungen kommen.

Erfindungsgemäß vorteilhaft wird man das erfindungsgemäße Verfahren so durchführen, dass der Gehalt des feinteiligen Gemischs M* an feinteiligem Haufwerk H, bezogen auf das Gesamtgewicht des feinteiligen Gemischs M*, 15 Gew.-% bzw. 10 Gew.-% nicht überschreitet. Anwendungstechnisch zweckmäßig ist es für das erfindungsgemäße Verfahren jedoch günstig, wenn der Gehalt des feinteiligen Gemischs M* an feinteiligem Haufwerk H wenigstens zeitweise, bezogen auf das Gesamtgewicht des feinteiligen Gemischs M*, wenigstens 1 Gew.-%, oder wenigstens 3 Gew.-%, oder wenigstens 5 Gew.-% beträgt.

Für das erfindungsgemäße Verfahren ist es weiterhin angebracht, wenn die Abluft (für den Fall, dass eine oder mehrere Verfahrensstufen nicht unter Luft, sondern unter einer anderen Gasatmosphäre wie z. B. Inertgas (z. B. $N_2$) oder mit Inertgas (z. B. $CO_2$) verdünnter Luft, oder getrocknete Luft durchgeführt wird, soll sich der Begriff "Abluft" auch auf diese Gasatmosphären erstrecken) aus den verschiedenen Verfahrensstufen B) bis G) abgesaugt und wenigstens einer mechanischen Trennoperation unterworfen wird, mit der in der abgesaugten Abluft (Gasatmosphäre) dispergierte Feststoffpartikel FP abgetrennt werden können (dies gilt insbesondere für die Abluft des zur Pressagglomeration in der Verfahrensstufe B) eingesetzten Apparats, für die Abluft des zur Pressagglomeration in der Verfahrensstufe D) eingesetzten Apparats, sowie für die Abluft der in den Verfahrensstufen C) und G) eingesetzten Zerkleinerungsapparate und der in der Verfahrensstufe B) sowie gegebenenfalls D) eingesetzten Mischvorrichtungen).

Die abgetrennten Feststoffpartikel FP, die in der Regel überwiegend von den in der jeweiligen Verfahrensstufe bearbeiteten feinteiligen Materialien abstammen, können anschließend kontinuierlich oder absatzweise in die Verfahrensstufe B) rückgeführt und ebenfalls in das feinteilige Gemisch M* eingearbeitet werden, mit der Maßgabe, dass der Gehalt des Gemischs M* an solchen rückgeführten Feststoffpartikeln FP, bezogen auf das Gesamtgewicht des Gemischs M*, 10 Gew.-%, vorzugsweise 5 Gew.-% nicht überschreitet.

Die vorgenannte Gehaltsbeschränkung ist deshalb von Relevanz, weil die rückgeführten Feststoffpartikel FP besonders feinteilig sind und gegebenenfalls die Ausbildung des Porengefüges in den geometrischen Katalysatorformkörpern K behindern können, woraus letztlich eine Beeinträchtigung der Katalysatorperformance (Aktivität, Selektivität der Zielproduktbildung) resultieren kann. Auch kann die Abluft normale Staubpartikel enthalten, die die Katalysatorperformance aufgrund ihrer stofflichen Beschaffenheit mindern können. Je nach Gestaltung der Rückführung der Feststoffpartikel FP, des feinteiligen Haufwerks H und des Feinkorns F, kann das feinteilige Gemisch M* beim erfindungsgemäßen Verfahren nur aus der feinteiligen Mischung M, oder aus der feinteiligen Mischung M und einem oder mehreren der vorgenannten, in die Verfahrensstufe B rückgeführten, Materialien (d. h. Haufwerk H, Feinkorn F und/oder Feststoffpartikel FP) bestehen.

Bei erfindungsgemäß geeigneten, eine solche mechanische Trennoperation anwendenden, Abluftreinigungsverfahren wird normalerweise durch äußere Kräfte eine Relativbewegung der dispergierten Feststoffpartikel gegenüber dem Trägergas erzeugt.

Je nach den hauptsächlich wirkenden Kräften werden dabei z. B. folgende Abscheideprinzipien unterschieden:

- Prall-, Stoß- und Fliehkräfte in Umlenkabscheidern,

- Zentrifugalkräfte in Fliehkraftabscheidern,
- Prallwirkung und Haftkräfte in filternden Abscheidern, und
- elektrische Kräfte in Elektrofiltern.

D. h., erfindungsgemäß geeignete, eine mechanische Trennoperation anwendende Abluftreinigungsapparate sind z. B. Kammer-, Prall- und Zentrifugalabscheider, die Massekräfte nutzen. Es sind für das erfindungsgemäße Verfahren aber auch akustische Abscheider anwendbar. Bevorzugt sind Aerozyklone. In einfacher Weise kann erfindungsgemäß als mechanische Trennoperation aber auch filitriert werden.

Als Filterschichten kommen z. B. Filtergewebe, poröse Filtermassen oder Papiervlies in Betracht. Auch Elektroabscheider sind erfindungsgemäß anwendbar. Selbstverständlich können erfindungsgemäß auch verschiedene hintereinandergeschaltete mechanische Trennoperationen angewendet werden.

Die erfindungsgemäß bevorzugte mechanische Trennoperation ist das Filtrieren, lassen sich damit doch auf vergleichsweise einfache Weise Teilchen mit einer Längsausdehnung von 0,001 $\mu$m und weniger noch abfangen (der Feststoffgehalt der filtrierten Abluft liegt beim erfindungsgemäßen Verfahren in der Regel bei Werten $\leq$ 0,1 mg/m$^3$). Bei entsprechender Dimensionierung und Auswahl des Filterstoffs lassen sich auf relativ kostengünstige Art und Weise Abscheidegrade von mehr als 99,9 % erreichen.

Die Abscheidewirkung basiert beim Filtrieren im Wesentlichen auf Prallwirkung (Stoß der Feinstpartikel auf das Filterelement) und Diffusion, wobei aber auch andere Faktoren wie Gravitation und elektrostatische Kräfte von Einfluss sind. Obwohl es sich bei der Filtration um keinen reinen Siebvorgang handelt (die durch Filtration abgeschiedenen Teilchen sind häufig wesentlich kleiner als die Poren des Filtermediums), haben engmaschige Filter bei der erfindungsgemäßen Filtration einen höheren Wirkungsgrad als weitmaschige. Anwendungstechnisch zweckmäßig können für das erfindungsgemäße Verfahren unter anderem Gewebefilter eingesetzt werden. Prinzipiell eignen sich für die erfindungsgemäße Filtration Filtergewebe aus Natur- oder Chemiefasern. D. h., sowohl Filtergewebe aus PVC, Polyamiden (Perlon®, Nylon®), Wolle, Polyacrylnitril (Redon®, Dralon®), Polyester und Polytetrafluorethylen (Teflon®) als auch aus siliconisiertem Glasgewebe.

Generell eignen sich für das erfindungsgemäße Verfahren Filter, wie sie auch in Klima-und Lüftungsanlagen zum Einsatz kommen. Ein günstiges Brandverhalten des Filtermaterials im Sinne der DIN 53438 ist bevorzugt. Im Übrigen kann wie in der DE-A 103 60 396 am Beispiel einer Luftfiltration beschrieben vorgegangen werden. Eigene Untersuchungen haben gezeigt, dass der Gehalt der relevanten Abluft an festen Feinstpartikel (Feststoffpartikel FP) vorab der Durchführung einer erfindungsgemäßen mechanischen Trennoperation in der Regel $\geq$ 20 mg/m$^3$ beträgt.

Bei Erreichen eines einstellbaren Druckverlustanstiegs bei der Filtration können die abgeschiedenen Feststoffpartikel FP z. B. durch Abklopfen vom Filtergewebe separiert und wie beschrieben in die Verfahrensstufe B) rückgeführt werden (vorzugsweise absatzweise). Eine mögliche Ausführungsform von Gewebe- bzw. Vliesfiltern für die erfindungsgemäße Filtration sind Schlauchfilter.

Das erfindungsgemäße Verfahren eignet sich insbesondere zur Herstellung von solchen geometrischen Katalysatorformkörpern K, die als Aktivmasse ein Multielementoxid enthalten, in welchem das Element Mo dasjenige von Sauerstoff verschiedene Element E ist, das numerisch (molar gerechnet) von allen von Sauerstoff verschiedenen Elementen E des Multielementoxids, am häufigsten im Multielementoxid enthalten ist.

Insbesondere eignet sich das erfindungsgemäße Verfahren zur Herstellung von solchen geometrischen Katalysatorformkörpern K, die als Aktivmasse ein Multielementoxid enthalten, das, bezogen auf die molare Gesamtmenge seiner von molekularem Sauerstoff verschiedenen Elemente E, zu wenigstens 30 mol-%, vorzugsweise zu wenigstens 40 mol-% und besonders bevorzugt zu wenigstens 45 mol-% das Element Mo enthält.

In der Regel werden erfindungsgemäß hergestellte geometrische Katalysatorformkörper K als Aktivmasse ein Multielementoxid enthalten, das, bezogen auf die molare Gesamtmenge seiner von molekularem Sauerstoff verschiedenen Elemente E, zu nicht mehr als 90 mol-% ($\leq$ 90 mol-%) bzw. zu nicht mehr als 80 mol-% ($\leq$ 80 mol-%) das Element Mo enthält.

Die Herstellung von für das erfindungsgemäße Verfahren geeigneten feinteiligen Mischungen M kann z. B. wie im Stand der Technik beschrieben erfolgen (vgl. z. B. die Deutsche Anmeldung mit dem Aktenzeichen 102008042064.6, die Deutsche Anmeldung mit dem Aktenzeichen 10 2008 042 061.1, die Deutsche Anmeldung mit dem Aktenzeichen 10 2008 042 060.3, die Deutsche Anmeldung mit dem Aktenzeichen 10 2008 040 093.9, die Deutsche Anmeldung mit dem Aktenzeichen 10 2008 040 094.7, WO 2005/030393, EP-A 467 144, EP-A 1 060 792, DE-A 198 55 913, WO 01/68245, EP-A 1 060 792, Research Disclosure RD 2005-497012, DE-A10 2005 035 978, DE-A 10 2005 037 678, WO 03/78059, WO 03/078310, DE-A 199 22 113, WO 02/24620, WO 02/062737, DE-A 10 2007 028 332, DE-A 10 2007 025 869, DE-A 10 2007 017 080 und US-A 2005/0131253).

Die feinteiligen Mischungen M sind dabei in einfachster Weise dadurch erhältlich, dass man mit Quellen Q der von Sauerstoff verschiedenen Elemente E des angestrebten katalytisch aktiven Multielementoxids (d. h. mit Ausgangsverbindungen, die jeweils wenigstens ein Element E chemisch gebunden enthalten) sowie nach Bedarf mitzuverwendenden Formgebungshilfsmitteln (z. B. Porosierungsmittel, Antibackmittel, Gleitmittel und Verstärkungsmittel) eine in erfindungsgemäß geforderter Weise feintei-lige Mischung M erzeugt, deren Zusammensetzung an der erwünschten Stöchiometrie des katalytisch aktiven Multielementoxids ausgerichtet ist.

Als Quellen Q der Elemente E (als Ausgangsverbindungen, die wenigstens ein Element E enthalten) können dabei (bei Normalbedingungen in der Regel im festen Aggregatzustand befindliche) Elementoxide (z. B. Metalloxide) und/oder solche wenigstens ein Element E (z. B. wenigstens ein Metall E) enthaltende chemische Verbindungen verwendet werden, die durch Erhitzen (thermisches Behandeln bei erhöhter Temperatur) in (bei Normalbedingungen in der Regel im festen Aggregatzustand befindliche) Oxide überführbar sind (wenigstens durch thermisches Behandeln in Anwesenheit von gasförmigem molekularem Sauerstoff und/oder von gasförmigem Sauerstoff freisetzenden Komponenten). Prinzipiell kann die Sauerstoffquelle z. B. in Form eines Peroxids Bestandteil der feinteiligen Mischung M sein. Ganz generell kann eine Ausgangsverbindung Quelle für mehr als ein Element E sein. Haufgut H, Feinkorn F und Feststoffpartikel FP werden normalerweise nicht als Quellen Q eingesetzt.

Auch kann die feinteilige Mischung M Verbindungen wie $NH_4OH$, $(NH_4)_2CO_3$, $NH_4NO_3$, Harnstoff, $NH_4CHO_2$, $NH_4CH_3CO_2$, Ammoniumoxalat und/oder organische Komponenten wie z. B. Stearinsäure, Stärke (z. B. Kartoffelstärke, Maisstärke), gemahlene Nussschale, feinteiliges Kunststoffgranulat (wie z. B. Polyethylen, Polypropylen), Cellulose, Graphit, Malonsäure, Salze der Stearinsäure, Salze der Malonsäure u. a. zugesetzt enthalten, die bei der thermischen Behandlung der Formkörper V$^+$ als Porenbildner fungieren, indem sie zu gasförmig entweichenden Verbindungen zerfallen und/oder zersetzt werden (z. B. zu Ammoniak, Wasserdampf, $CO_2$, CO und/oder Stickstoffoxiden).

Eine Ausbildung (Freisetzung) von porenbildenden gasförmigen Verbindungen im Rahmen der thermischen Behandlung von Formkörpern V$^+$ ist normalerweise auch dann gegeben, wenn die Quellen Q mit denen die feinteilige Mischung M erzeugt wird, teilweise organischer Natur sind (z. B. im Fall von Acetaten, Oxalaten und/oder Citraten) bzw. Hydroxidionen, Carbonationen, Hydrogencarbonationen, Ammoniumionen, Hydrogenphosphationen und/oder Nitrationen enthalten, die sich bei der erfindungsgemäßen thermischen Behandlung der Formkörper V$^+$ in der Regel wenigstens teilweise zersetzen.

In der Regel beträgt der mit der thermischen Behandlung der Formkörper V einhergehende und auf deren Ausgangsgewicht bezogene Gewichtsverlust (infolge des vorgenannten Ausgasens) 0,5 bis 40 Gew.-%, häufig 0,8 bis 35 Gew.-%, oder 2 bis 30 Gew.-%.

Desweiteren kann die feinteilige Mischung M als weitere Formgebungshilfsmittel Gleitmittel zugesetzt enthalten, deren Beisein sowohl in der Verfahrensstufe B) als auch in der Verfahrensstufe D) vorteilhaft ist, in dem sie reibungsvermindernd wirken. Als solche Gleit- bzw. Schmiermittel können z. B. Graphit, Ruß, Polyethylenglykol, Stearinsäure, Salze der Stearinsäure, Malonsäure, Salze der Malonsäure, Stärke, Polyacrylsäure, Mineralöl, Pflanzenöl, Wasser, Bornitrid, Bortrifluorid, Glyzerin, feinteiliges Teflonpulver und/oder Celluloseether verwendet werden. Erfindungsgemäß bevorzugt wird ausschließlich feinteiliger Graphit als Gleitmittel mitverwendet. Bevorzugt zugesetzte Graphite sind Asbury 3160 und Asbury 4012 der Firma Asbury Graphite Mills, Inc. New Jersey 08802, USA und Timrex® T44 der Firma Timcal Ltd., 6743 Bodio, Schweiz. Zur Gruppe der Formgebungshilfsmittel, die in feinteiliger Form in der feinteiligen Mischung M enthalten sein können, gehören auch die Antibackmittel.

Hierbei handelt es sich um feinteilige Materialien, die mitverwendet werden können, um innerhalb der feinteiligen Mischung M im Rahmen des Vermischens z. B. eine Reagglomeration (ein "Zusammenbacken") von Partikeln weitestgehend zu unterdrücken, da eine solche Reagglomeration den wirksamen Partikeldurchmesser beeinflussen könnte. Eine erfindungsgemäß bevorzugt Gruppe an feinteiligen Antibackmitteln bilden feinteilige hydrophobisierte Kieselsäuren, insbesondere feinteilige hydrophobisierte synthetische Kieselsäuren (Siliziumdioxide).

Synthetische Kieselsäuren können einerseits unmittelbar pyrogen aus Sand und andererseits durch Fällungsreaktionen aus Wasserglas erzeugt werden. Insbesondere synthetische Kieselsäuren sind aufgrund ihrer oberflächenständigen OH-Gruppen hydrophil, d. h., sie werden durch Wasser benetzt. Zum Beispiel durch Reaktion dieser oberflächenständigen OH-Gruppen mit Chlorsilanen lassen sich sowohl aus den pyrogenen als auch aus den Fällungskieselsäuren hydrophobisierte Produkte herstellen. Beispielsweise kann die Hydrophobisierung durch Umsetzung mit Dimethyldichlorsilan im Beisein von Wasserdampf bei ca. 400 °C in einem Fließbettreaktor erfolgen (wird vorzugsweise bei pyrogenen Kieselsäuren angewendet).

Insbesondere bei Fällungskieselsäuren wird das Chlorsilan der Fällsuspension bei einer Temperatur von 50 bis 90 °C unter gründlichem Rühren zugegeben. Anschließend folgen Filtration, Neutralwaschen mit Wasser, Trocknen der Filterkuchen und Tempern bei 300 bis 400 °C. In H. Brunner, D. Schutte, Chem. Ing. Techn. 89, 437 (1965) sowie in DT 24 35 860 und DT 11 17 245 wird die Herstellung hydrophobisierter feinteiliger Kieselsäuren näher beschrieben. Handelsprodukte von hydrophobisierten Fällungskieselsäuren bilden z. B. die SIPERNAT®-Marken.

Erfindungsgemäß bevorzugt wird als feinteiliges Antibackmittel Sipernat® D17 der Firma Degussa bzw. der Fa. EVONIK Industries mitverwendet. Sipernat® D17 enthält auf sein Gewicht bezogen etwa 2 Gew.-% an chemisch gebundenem Kohlenstoff und wird von Wasser nicht benetzt. Sein Rüttelgewicht (gemäß ISO 787-11) beträgt 150 g/l. Sein $d_{50}$-Wert beträgt 10 μm (Laserbeugung nach ISO 13320-1) und die spezifische Oberfläche (Stickstoffadsorption nach ISO 5794-1, Annex D) beträgt 100 m$^2$/g.

Der Zusatz von Antibackmitteln in die feinteilige Mischung M mindert auch den für eine homogene Vermischung derselben erforderlichen Energieeintrag. Um den inneren Zusammenhalt der im Verlauf des erfindungsgemäßen Verfahrens erzeugten Pressagglomerate zu fördern, können der feinteiligen Mischung M außerdem als Verstärkungsmittel feinteilige Mikrofasern aus z. B. Glas, Asbest, Siliciumcarbid und/oder Kaliumtitanat zugesetzt werden.

Die zugesetzten Formgebungshilfsmittel entweichen entweder gasförmig bei der thermischen Behandlung der Formkörper V, oder verbleiben als im Wesentlichen inerte Verdünner in den resultierenden Katalysatorformkörpern K.

Für die Bestimmung des Partikeldurchmessers $d_{50}^M$ (bzw. allgemein $d_X^M$) wird die feinteilige Mischung M über eine Dispergierrinne in den Trockendispergierer Sympatec RODOS (Sympatec GmbH, System-Partikel-Technik, Am Pulverhaus 1, D-38678 Clausfhal-Zellerfeld) geführt und dort mit Druckluft trocken dispergiert sowie im Freistrahl in die Messzelle geblasen. In dieser wird dann nach ISO 13320 mit dem Laserbeugungsspektrometer Malvern Mastersizer S (Malvern Instruments, Worcestshire WR14 1AT, United-Kingdom) die volumenbezogene Partikeldurchmesserverteilung bestimmt. Die als Messergebnis angegebenen Partikeldurchmesser $d_X^M$ sind dabei so definiert, dass X% des Partikelgesamtvolumens der feinteiligen Mischung M aus Partikeln mit diesem oder einem kleineren Durchmesser bestehen.

Das heißt, (100-X)% des vorgenannten Gesamtpartikelvolumens bestehen aus Partikeln mit einem Durchmesser > $d_X^M$. Wird in dieser Schrift nicht ausdrücklich etwas anderes erwähnt, beziehen sich Partikeldurchmesserbestimmungen an der feinteiligen Mischung M und daraus entnommene $d_X^M$ (sowie an anderen feinteiligen Gemischen) auf die vorbeschriebene Bestimmungsmethode sowie auf einen bei der Bestimmung angewandten (die Stärke der Dispergierung des Pulvers während der Messung bestimmenden) Dispergierdruck von 2 bar absolut.

Der Begriff "Multielementoxid" meint in dieser Schrift kein einfaches Gemisch verschiedener Elementoxide, sonder eine komplexe Polyoxyverbindung, die neben Sauerstoff wenigstens die erfindungsgemäß relevanten Elemente E enthält.

Rechnet man wie in dieser Schrift Halbmetalle wie Phosphor, Antimon, Arsen und Silizium den Metallen zu, so handelt es sich bei vielen Multielementoxidaktivmassen der erfindungsgemäß erhältlichen geometrischen Katalysatorformkörper K um Multimetalloxide und bei den Elementen E um Metalle. Grundsätzlich können die Multielementoxidaktivmassen der geometrischen Katalysatorformkörper K aber auch Nichtmetalle wie z. B. das Element Schwefel als Elemente E enthalten. Diese Fälle sind jedoch eher die Ausnahme.

Grundsätzlich kann die Erzeugung der feinteiligen Mischung M ausschließlich durch einfaches Vermischen feinteiliger trockener Ausgangsverbindungen erfolgen.

Erfindungsgemäß bevorzugt werden im Rahmen der Erzeugung der feinteiligen Mischung M wenigstens zwei voneinander verschiedene Quellen Q von voneinander verschiedenen Elementen E in wässrigem Medium miteinander vermischt, und dies bevorzugt mit der Maßgabe, dass dabei wenigstens eine der wenigstens zwei Quellen Q den Zustand einer wässrigen Lösung durchläuft. Nachfolgend kann das dabei resultierende wässrige Gemisch getrocknet (z. B. durch Sprühtrocknung, oder durch Gefriertrocknung, oder durch einfaches Eindampfen), die dabei resultierende Trockenmasse bei bedarf zerkleinert, und die feinteilige Trockenmasse anschließend mit den übrigen feinteiligen Bestandteilen der feinteiligen Mischung M vermischt werden.

Erfindungsgemäß besonders bevorzugt werden im Rahmen der Erzeugung der feinteiligen Mischung M wenigstens drei voneinander verschiedene Quellen Q von voneinander verschiedenen Elementen E in wässrigem Medium miteinander vermischt, und dies bevorzugt mit der Maßgabe, dass dabei wenigstens eine (besonders bevorzugt wenigstens zwei) der wenigstens drei Quellen Q den Zustand einer wässrigen Lösung durchläuft. Nachfolgend kann das dabei resultierende wässrige Gemisch getrocknet (z. B. durch Sprühtrocknung, oder durch Gefriertrocknung, oder durch einfaches Eindampfen), die dabei resultierende Trockenmasse bei Bedarf zerkleinert, und die feinteilige Trockenmasse anschließend mit den übrigen feinteiligen Bestandteilen der feinteiligen Mischung M vermischt werden.

Selbstverständlich können beim erfindungsgemäßen Verfahren auch alle zur Erzeugung der feinteiligen Mischung M verwendeten Quellen Q der Elemente E in wässrigem Medium miteinander vermischt werden. Dies wiederum bevorzugt mit der Maßgabe, dass dabei wenigstens eine (vorzugsweise wenigstens zwei) der insgesamt verwendeten Quellen Q den Zustand einer wässrigen Lösung durchläuft. Nachfolgend kann das dabei resultierende wässrige Gemisch getrocknet (z. B. durch Sprühtrocknung, oder durch Gefriertrocknung, öder durch einfaches Eindampfen), die dabei resultierende Trockenmasse bei Bedarf zerkleinert, und die feinteilige Trockenmasse anschließend gegebenenfalls mit mitzuverwendenden feinteiligen Formgebungshilfsmitteln zur feinteiligen Mischung M vermischt werden. Selbstverständlich können bei allen vorgenannten Verfahrensvarianten die feinteiligen Formgebungshilfsmittel wenigstens teilweise oder auch in ihrer Gesamtmenge schon in die wässrige Mischung der Quellen Q (vorab der Trocknung dieser wässrigen Mischung) eingearbeitet werden. Dies gilt insbesondere für in wässrigem Medium lösliche Porosierungsmittel. Antibackmittel, Gleitmittel und Verstärkungsmittel werden dagegen vorzugsweise trocken in die übrigen Bestandteile der feinteiligen Mischung M eingearbeitet.

Bezogen auf die Gesamtmenge der feinteiligen Mischung M wird die Gesamtmenge der darin enthaltenen Formgebungshilfsmittel in der Regel nicht mehr als 30 Gew.-%, meist nicht mehr als 20 Gew.-% und vielfach nicht mehr als 10 Gew.-% betragen. Üblicherweise wird der vorgenannte Gewichtsanteil jedoch ≥ 0,5 Gew.-% betragen.

Ein ergänzender Zusatz an feinteiligem Formgebungshilfsmittel kann beim erfindungsgemäßen Verfahren zum Pulver P vorab dessen Pressagglomeration zu den geometrischen Formkörpern V vorgenommen werden. Dieser Zusatz kann dabei vorab und/oder nach der Förderung des Pulvers P aus der Verfahrensstufe C) in die Verfahrensstufe D) erfolgen. D. h., das Pulver P kann vorab und/oder nach der Zugabe von feinteiligem Formgebungshilfsmittel aus der Verfahrensstufe C) in die Verfahrerisstufe D) gefördert werden.

Erfolgt der Zusatz vorab der Förderung des Pulvers aus der Verfahrensstufe C) in die Verfahrensstufe D), so erfolgt diese Förderung des Pulvers P in die Verfahrensstufe D) normalerweise im Gemisch mit dem zugesetzten feinteiligen Formgebungshilfsmittel.

Insgesamt sollte auch die in den geometrischen Formkörpern V enthaltene Gesamtmenge an Formgeburigshilfsmitteln beim erfindungsgemäßen Verfahren 30 Gew.-%, bezogen auf das Gewicht der Formkörper V, nicht überschreiten. Meist beträgt dieser Gewichtsanteil ≤ 20-Gew.-% und-vielfach ≤ 10 Gew.-%. In der Regel liegt dieser Gewichtsanteil jedoch bei ≥ 1 Gew.-%.

Der Partikeldurchmesser $d_{50}^{M}$ beträgt erfindungsgemäß bevorzugt ≥ 1 und ≤ 125 $\mu$m, besonders bevorzugt ≥ 1 und ≤ 100 $\mu$m, mit Vorteil ≥ 5 und ≤ 75 $\mu$m sowie ganz besonders bevorzugt ≥ 10 und ≤ 50 $\mu$m. In den letzteren beiden Fällen ist es ferner vorteilhaft, wenn höchstens 10 Gew.-% des Gesamtgewichts der in der feinteiligen Mischung M enthaltenen Partikel einen Partikeldurchmesser $d^M$ ≥ 125 $\mu$m bzw. ≥ 100 $\mu$m aufweisen.

Die Einstellung der Partikelgröße kann auf dem Weg der Herstellung der feinteiligen Mischung M z. B. durch Mahlen der verwendeten Ausgangsmaterialien und/oder durch Sprühtrocknen entsprechender wässriger Mischungen (z. B. Lösungen) vorgenommen werden.

Die thermische Behandlung von erfindungsgemäß hergestellten geometrischen Formkörpern V kann sowohl unter Vakuum, unter inerter Atmosphäre (z. B. $N_2$, Edelgase, Wasserdampf, $CO_2$ etc.), unter reduzierender Atmosphäre (z. B. $H_2$ oder $NH_3$) oder unter oxidierender Atmosphäre erfolgen.

In der Regel werden oxidierende Atmosphären molekularen Sauerstoff enthalten. Typische oxidierende Atmosphären sind Gemische aus Inertgas ($N_2$, Edelgase, Wasserdampf, $CO_2$ etc.) und molekularem Sauerstoff. Üblicherweise wird der Gehalt an molekularem Sauerstoff dabei wenigstens 0,1 Vol.-%, häufig wenigstens 0,2 Vol.-%, vielfach wenigstens 0,5 Vol.-%, oft wenigstens 1 Vol.-%, oder wenigstens 10 Vol.-%, oder wenigstens 20 Vol.-% betragen.

Selbstverständlich kann der Gehalt an molekularem Sauerstoff in solchen Gemischen aber auch 30 Vol.-%, oder 40 Vol.-%, oder 50 Vol.-% oder mehr betragen. Selbstverständlich kommt als oxidierende Atmosphäre für eine solche thermische Behandlung aber auch reiner molekularer Sauerstoff in Betracht. Häufig wird eine oxidierende thermische Behandlung unter Luft erfolgen.

Generell kann die thermische Behandlung unter stehender oder unter fließender Gasatmosphäre (z. B. im Luftstrom) erfolgen.

Der Begriff der Atmosphäre (bzw. der Gasatmosphäre) in welcher die thermische Behandlung erfolgt, ist dabei in dieser Schrift so zu verstehen, dass er sich aus den erfindungsgemäß hergestellten geometrischen Formkörpern V im Rahmen der thermischen Behandlung aufgrund von Zersetzungsvorgängen und/oder chemischen Reaktionsvorgängen entwickelnde Gase nicht umfasst. Selbstverständlich kann die Gasatmosphäre, in welcher die thermische Behandlung erfolgt, aber auch ausschließlich oder teilweise aus diesen Gasen bestehen. Auch können sowohl die Behandlungstemperatur als auch die Behandlungsatmosphäre über die Dauer der thermischen Behandlung zeitlich konstant oder zeitlich variabel gestaltet sein.

In der Regel erfolgt die thermische Behandlung bei Temperaturen von 150 bis 650 °C, vielfach 200 bis 600 °C, oft 250 bis 550 °C und vielfach 300 bis 500 °C.

Enthält das zu geometrischen Formkörpern V pressagglomerierte Pulver P bzw. P* Ammonium-, Formiat-, Acetat- und/oder Nitrationen, so hat es sich für die Performance der geometrischen Katalysatorformkörper K (insbesondere die Selektivität der Zielproduktbildung) als vorteilhaft erwiesen, wenn der Gehalt $G^W$ der geometrischen Formkörper V (bzw. der Pulver P, P*) an Wasser, bezogen auf das Gesamtgewicht der in den geometrischen Formkörpern V enthaltenen Gesamtmenge an Ammonium-, Formiat-, Acetat- und Nitrationen, ≤ 60 Gew.-%, mit Vorteil ≤ 50 Gew.-%, bzw. ≤ 40 Gew.-% und noch besser ≤ 35 Gew.-% beträgt. In der Regel wird der Gehalt $G^W$ ≥ 15 Gew.-% und teilweises ≥ 20 Gew.-% betragen.

Dieser Zusammenhang ist vermutlich darauf zurückzuführen, dass Salze der vorgenannten Ionen Wasser in gewissem Umfang ohne Lösungserscheinungen zu binden vermögen.

Überschreitet der Wassergehalt diese Bindekapazität, kann das Wasser Auflösungs-und/oder Chromatographeffekte (z. B. insbesondere im Anfangsstadium der thermischen Behandlung der Formkörper V) bewirken (vor allem der Elemente Co, Ni, Fe, Cu und der Alkalimetalle), mit denen lokale An- und Entreicherungen von Elementen E im Gesamtgefüge einhergehen, die die katalytische Wirksamkeit beeinträchtigen.

Dies gilt besonders dann, wenn die Multielementoxidaktivmasse Co enthält.

Aufgrund der erfindungsgemäßen Rückführungen in die Verfahrensstufe B), die die Verweilzeit von Salzen der relevanten Ionen im Herstellprozess erhöhen, ist es zur Erzielung der vorgenannten Wassergehalte anwendungstechnisch zweckmäßig, wenigstens eine der nachfolgenden Zusatzmaßnahmen anzuwenden:

- bei der Abtrennung von in der Abluft aus den verschiedenen Verfahrensstufen B) bis G) dispergierten Feststoffpartikeln FP durch z. B. Filtrieren der Abluft, wird der Filterkuchen von großen Abluftmengen durchströmt; anwendungstechnisch vorteilhaft werden die Verfahrensstufen B) bis G) daher in klimatisierten Räumen durchgeführt, deren Raumluft mittels einer Klimaanlage kontinuierlich entfeuchtet wird;

- die Förderung feinteiliger Materialien von der Verfahrensstufe C) in die Verfahrensstufe D) erfolgt pneumatisch mittels Überdruck;

- erfolgt die Rückführung in die Verfahrensstufe B) anwendungstechnisch vorteilhaft absatzweise, werden das Feinkorn F, das Haufwerk H sowie die abgetrennten Feststoffpartikel FP vorteilhaft in geschlossenen Behältern gesammelt und gelagert;

- Sprühtrocknungen von wässrigen Mischung im Rahmen der Herstellung der feinteiligen Mischung werden zweckmäßig mittels getrockneten Heißgasströmen und bei erhöhten Ausgangstemperaturen durchgeführt;

- feinteilige Mischung M, Gemisch M*, Agglomerate A, Pulver P bzw. P* und Formkörper V werden in geschlossenen Behältern zwischengelagert;

- den Anteil an rückzuführendem Feinkorn F, Haufwerk H und Feststoffpartikeln FP am feinteiligen Gemisch M* gering halten.

Die erfindungsgemäß vorteilhaft einzuhaltenden Gehalte $G^W$ der geometrischen Formkörper V (bzw. der Pulver P, P*) an Wasser sind nicht zuletzt dann von Relevanz, wenn die geometrischen Formkörper V (bzw. die Pulver P, P*) sauer sind, da eine acide Umgebung die Löseneigung erhöht. Die Eigenschaft "sauer" soll dabei dann vorliegen, wenn man 10 mg eines Formkörpers V (des Pulvers P oder P*) in 10 ml mehrfach destilliertem Wasser (pH = 7 bei 25 °C, 1 atm) bei 25 °C und 1 atm 5 min. lang rührt und der pH des wässrigen Mediums danach einen Wert von höchstens 6 (bzw. 5 oder 4) (bei 25 °C, 1 atm) aufweist (pH-Wert ≤ 6). Dies gilt vor allem dann, wenn der geometrische Formkörper V (bzw. das Pulver P bzw. P*) zusätzlich Nitrationen enthält.

Der Absolutgehalt von Formkörpern V oder Pulver P bzw. P* an Wasser kann z. B. dadurch bestimmt werden, dass das enthaltene Wasser durch Einstrahlung von Mikrowellen selektiv verdampft und der dabei einhergehende Gewichtsverlust bestimmt wird.

Ganz generell erfolgt im Verlauf der Herstellung einer feinteiligen Mischung M das Vermischen der zu dieser Herstellung verwendeten Quellen Q vorzugsweise in nasser Form. Üblicherweise werden dabei die die Elemente E enthaltenden Ausgangsverbindungen in Form einer wässrigen Lösung und/oder Suspension miteinander vermischt. Besonders innige Gemische werden dabei dann erhalten, wenn ausschließlich von in gelöster Form vorliegende Quellen Q der elementaren Konstituenten ausgegangen wird, wobei Wasser das bevorzugte Lösungsmittel ist. Anschließend wird die erhaltene Lösung oder Suspension getrocknet, wobei der Trocknungsprozess vorzugsweise durch Sprühtrocknung mit Austrittstemperaturen von 100 bis 150 °C erfolgt (in manchen Fällen kann die Trocknung aber auch durch Filtration und anschließende Trocknung des Filterkuchens erfolgen). Der Partikeldurchmesser $d_{50}$ des resultierenden Sprühpulvers beträgt in typischer Weise 10 bis 50 $\mu$m. Nach Zusatz (oder auch ohne einen solchen Zusatz) der gewünschten Formgebungshilfsmittel zur jeweiligen pulverförmig gestalteten Trockenmasse resultiert eine feinteilige Mischung M. Die feinteiligen Formgebungshilfsmittel können aber auch bereits vorab in die Sprühmaische (teilweise oder vollständig) zugesetzt werden.

Eine nur teilweise Entfernung des Lösungs- bzw. Suspendiermittels kann anwendungstechnisch zweckmäßig sein, wenn seine Mitverwendung als Formgebungshilfsmittel beabsichtigt ist.

War Wasser die Basis des flüssigen Mediums, wird das resultierende Sprühpulver normalerweise nicht mehr als 20 % seines Gewichtes, vorzugsweise nicht mehr als 15 % seines Gewichtes und besonders bevorzugt nicht mehr als 10 % seines Gewichtes an Wasser enthalten. Diese Prozentsätze gelten in der Regel auch bei Anwendung anderer flüssiger Lösungs- bzw. Suspendierhilfsmittel.

Üblicherweise ist die feinteilige Mischung M und das feinteilige Gemisch M* beim erfindungsgemäßen Verfahren anfasstrocken. Wie vorstehend erwähnt, kann es jedoch auch dann noch Substanzen enthalten, die bei Normalbedingungen (25 °C, 1 atm) flüssig sind. Es kann aber auch völlig frei von solchen Substanzen sein.

Enthält der erfindungsgemäß hergestellte geometrische Katalysatorformkörper K als Aktivmasse ein Multielementoxid, das wenigstens ein Alkalimetall enthält, so ist das wenigstens eine Alkalimetall mit Vorteil K, Na, Cs und/oder Rb, und mit besonderem Vorteil K, Na und/oder Cs, oder K und/oder Na.

Die Längstausdehnung L der in der Verfahrensstufe B) erzeugten Agglomerate A hängt nicht zuletzt vom angewandten Pressagglomerationsverfahren ab. Unter der Längstausdehnung L wird dabei die längste direkte Verbindungslinie zweier auf der Oberfläche des Agglomerats A befindlicher Punkte verstanden. In der Regel wird sie beim erfindungsgemäßen Verfahrene ≥ 0,5 cm, vielfach ≥ 1 cm, oder ≥ 3 cm, häufig ≥ 5 cm oder ≥ 10 cm betragen. In den meisten Fällen beträgt die Längstausdehnung L jedoch ≤ 1 m.

Unter Pressagglomeration wird in dieser Schrift die Agglomeration pulverförmiger Feststoffe durch Einwirkung äußerer Druckkräfte verstanden. Dies kann in der Verfahrensstufe B) z. B. dadurch geschehen, dass eine definierte Menge des feinteiligen Gemischs M* in einer Matrize mit einem Stempel verdichtet wird (Tablettieren). Erfindungsgemäß vorteilhaft erfolgt die Pressagglomeration in der Verfahrensstufe B) durch Walzenpressen. Dabei wird das feinteilige Gemisch M* zwischen zwei gegeneinander drehenden Walzen eingezogen und im Walzenspalt kompaktiert. Das feinteilige Gemisch M* kann den Walzen nur mit Hilfe der Schwerkraft durch einen Fülltrichter oder durch ein Aufgabeorgan zugeführt werden. Erfindungsgemäß bevorzugt wird als Aufgabeorgan ein Fülltrichter mit integrierter Rühreinrichtung und Schneckenförderung verwendet. Dabei muss der Massenstrom im Schneckförderer der Kapazität der nachgeschalteten Walzenpressen angepasst werden. Die Förderschnecke ist dabei so ausgestaltet, dass das in der Walzenpresse zu agglomerierende Material auf dem Weg seiner Förderung in dem Walzenspalt bereits vorentlüftet und vorverdichtet wird.

Ferner reicht die Schnecke zur Förderung des feinteiligen Gemischs M* vorzugsweise bis in den Zwickelbereich zwischen den einander gegenüberliegenden Walzen hinein. Die Walzenoberfläche kann glatt sein oder offene oder geschlossene Profile aufweisen. Erfindungsgemäß bevorzugt werden Glattwalzen und besonders bevorzugt Walzen mit offenen Profilen eingesetzt. Sie erzeugen ein bandförmiges Agglomerat, das beim Ablösen von der Walze entlang der Bandlänge in Stücke bricht, die auch als "Schülpen" bezeichnet werden.

Die Schnecke weist vorzugsweise eine im Wesentlichen vertikale Drehachse auf. Sie kann eine längs ihrer Förderrichtung abnehmende Steigung und/oder einen längs der Förderrichtung abnehmenden Durchmesser aufweisen. Die Förderkammer, in welcher die Schnecke angeordnet ist, kann eine glatte Innenwand aufweisen. Sie kann aber auch eine wendelförmig um die Förderrichtung der Schnecke verlaufende Nut aufweisen, um die Vorschubwirkung der Schnecke zu unterstützen.

Die Walzen oder zumindest die Walzenoberflächen können aus Metall (Edelstahl), Thermoplast oder Duroplast und/oder aus einem Elastomer aufgebaut sein.

Prinzipiell sind Walzenpressen in zwei verschiedenen Bauarten verfügbar: mit festgelegter Spaltweite oder mit variabler Spaltweite. Bei letzteren ist eine der beiden Walzen, die sogenannte Loswalze, verschiebbar gelagert (sie werden erfindungsgemäß bevorzugt eingesetzt). Mit Hilfe einer Anpressvorrichtung wird ein stabiler Betriebszustand der Maschine gewährleistet. In einer einfachen Ausführung besteht das Anpresssystem aus Federpaketen. Erfindungsgemäß bevorzugt ist eine hydraulische Anpressvorrichtung, die eine genaue Anpassung des Pressdrucks an das zu agglomerierende feinteilige Gemisch M* und an schwankende Eduktmassenströme ermöglicht. Die Gestaltung der Walzenoberfläche bestimmt das Einzugsverhalten des Gemischs M*, die einstellbare Drehzahl der Walzen legt die Verweildauer des Materials im Kompaktierraum fest, ein stufenlos einstellbares Hydraulikaggregat erzeugt den nötigen Pressdruck und überträgt ihn auf die Walzen, die Hydraulik hält den eingestellten Walzendruck konstant und gewährleistet so eine homogene Schülpe, und Abstreifer halten die Walzen sauber.

Für die Verfahrensstufe C) des erfindungsgemäßen Verfahrens besonders geeignet ist der Zweiwalzenkompaktor vom Typ 200/100 der Fa. Hoskawa Bepex GmbH, D-74211 Leingarten, mit konkave, geriffelten Glattwalzen und variabler Spaltweite.

In typischer Weise weisen die beim erfindungsgemäßen Verfahren in der Verfahrensstufe B) produzierten Schülpen eine Dicke von wenigen mm (z. B. 3 mm) und eine Breite von einigen cm (z. B. 10 cm) auf. Im Übrigen kann z. B. wie in der WO 2008/014839 oder in "Modellierung der Pressagglomeration feinkörniger, kohäsiver und kompressibler Schüttgüter", Dissertation, Lilla Grossmann, Otto-von-Gueicke-Universität Magdeburg, 13.06.2006, empfohlen vorgegangen werden. Selbstverständlich kommen in der Verfahrensstufe B) auch alle anderen im Stand der Technik bekannten Verfahren der Pressagglomeration in Betracht. Die in der Verfahrensstufe B) angewandten maximalen (höchsten) Pressdrucke P1 richten sich nach der individuellen Beschaffenheit des jeweiligen feinteiligen Gemischs M*.

In der Regel werden sie so gewählt, dass das Material um das 1,5- bis 3-fache verdichtet wird (Verhältnis der Massendichte). Typische maximale Pressdrucke P1 betragen 0,1 bis 5, häufig 2 bis 4 kN/cm$^2$.

Erfindungsgemäß wesentlich ist, dass der maximale (höchste) Pressdruck P2 die Beziehung P2 ≥ 2 • P1 erfüllt. Das erfindungsgemäße Verfahren ist aber auch dann anwendbar, wenn P2 ≥ 3 • P1, oder P2 ≥ 4 • P1, oder P2 ≥ 6 • P1, oder P2 ≥ 8 • P1 erfüllt ist. In der Regel wird P2 jedoch nicht mehr als 20 • P1, häufig nicht mehr als 15 • P1 betragen.

Die in der Verfahrensstufe B) erzeugten Agglomerate A werden beim erfindungsgemäßen Verfahren anschließend in der Verfahrensstufe C) auf die gewünschte Korngröße zerkleinert (grundsätzlich können dazu alle bekannten Zerkleinerungsvorrichtungen verwendet werden). Erfindungsgemäß vorteilhaft erfolgt die Zerkleinerung feinkornarm.

Wurde in der Verfahrensstufe B) mittels Walzenpressen agglomeriert, werden die dabei erzeugten Schülpen anwendungstechnisch zweckmäßig zunächst gebrochen. Vorteilhaft wird dazu beim erfindungsgemäßen Verfahren eine Stiftbrechermaschine vom Typ GBM-406 der Fa. Frewitt-Maschinenfabrik AG in CH-1700 Fribourg verwendet.

Sie besteht im Inneren aus einer sich links herum drehenden (mit Stiften bestückten) Walze, der sogenannten Stiftwalze. Diese ist beiderseits von gelochten oder geschlitzten Prallplatten umgeben. Die Korngröße nach dem Brechvorgang wird durch die Größen der Löcher bzw. Schlitze (allgemein Öffnungen) in der Prallplatte und durch die Abstände der Prallplatten zur Stiftwalze eingestellt.

Nach der vorgenannten Grobzerkleinerung, bei der vergleichsweise scharfkantige Bruchstücke erhalten werden, werden diese anwendungstechnisch zweckmäßig einer Siebmühle zugeführt, vorzugsweise einer Rotorsiebmühle. Erfindungsgemäß bevorzugt wird für diesen Zweck eine Siebmühle vom Typ MGR-803 der Fa. Frewitt-Maschinenfabrik AG in CH-1700 Fribourg eingesetzt.

Diese Schlagsiebmaschine besteht aus einem Rotor und einem Lochsieb (die Grundstruktur einer solchen Schlagsiebmaschine zeigt die Figur 3 der WO 2008/014839 beispielhaft). Über rotierende Rotorarme werden die vorgebrochenen Schülpen durch die Öffnung eines konisch gekrümmten Siebes gedrückt und dabei mechanisch auf die durch die Sieböffnungen vorgegebene Korngröße zerkleinert. Das Sieb besteht anwendungstechnisch aus Viereckdraht und weist quadratische Maschen als Sieblöcher (Sieböffnungen) auf.

Die Antriebslagerung des Rotors bewirkt eine Erwärmung der produktberührenden Teile und somit einen Temperaturanstieg der Pulvermasse. Dieser sollte in der Regel 70 °C nicht übersteigen.

Als Nebenprodukt der beschriebenen Zerkleinerung fällt auch unerwünschtes Feinkorn an.

Dieses Feinkorn F (das normalerweise keinen unterhalb des Zerteilungsgrads der feinteiligen Mischung M liegenden Zerteilungsgrad aufweist) kann z. B. mittels eines Schwingsiebes als Unterkorn abgetrennt werden, wobei die Maschenweite des Schwingsiebs die Überkorn-/Unterkorn-Durchmessergrenze festlegt. Beispielsweise eigenen sich für diesen Trennschritt Schwingsiebe der Fa. Allgaier in D-73062 Uhingen, z. B. solche vom Typ ATS 600.

Das so abgetrennte Feinkorn F (es kann bis zu 50 Gew.-% oder mehr der Gesamtkornmenge betragen; erfindungsgemäß bevorzugt beträgt es ≤ 40 Gew.-%, oder ≤ 30 Gew.-% der Gesamtmenge) wird beim erfindungsgemäßen Verfahren absatzweise oder kontinuierlich zur Einarbeitung in feinteiliges Gemisch M* in die Verfahrensstufe B) rückgeführt, während das bei der Siebabtrennung anfallende Überkorn in die Verfahrensstufe D) gefördert wird. Vorab oder nach dieser Förderung können dem Pulver P feinteilige Formgebungshilfsmittel zugesetzt werden (z. B. Graphit als Schmiermittel für die Pressagglomeration in der nachfolgenden Verfahrensstufe D)). Die Förderung des Pulvers P aus der Verfahrensstufe C) für sich, oder im Gemisch mit feinteiligem Formgebungshilfsmittel (als Pulver P*) wird erfindungsgemäß vorteilhaft pneumatisch durchgeführt. Hierunter versteht man den Transport des Pulvers mit Gas (z. B. mit Luft (vorzugsweise getrocknete Luft; die Lufttrocknung kann z. B. mittels BASF Sorbeat® durchgeführt werden, durch dessen Schüttung die Luft geführt wird) oder mit Inertgas wie z. B. $N_2$ und/oder $CO_2$) mittels Überdruck ("druckpneumatisch") oder mittels Unterdruck ("saugpneumatisch").

Prinzipbedingt findet die Förderung durch Rohre oder Schläuche hindurch statt.

Grundsätzlich kann die Förderung sowohl als Flugförderung (Gasgeschwindigkeit ≥ 20 m/s, bezogen auf das leere Rohr; Fördergut wird schwebend durch die Förderleitung geblasen oder gesaugt; Verhältnis von Fördergut zu Fördergas (die Beladung) ist < 15 kg/kg, der Kontakt mit der Rohrwand ist besonders in Umlenkungen so intensiv, dass Verschleiß und Kornbruch auftritt), Strähnenförderung (Gasgeschwindigkeit 15 bis 20 m/s, Teil des Förderguts rutscht als Strähne über den Boden des Rohres und wird von den darüberfliegenden Partikeln angetrieben, Beladung (liegt im Bereich 20 bis 40 kg/kg), Pfropfenförderung (die Förderleitung ist soweit mit Fördergut gefüllt, dass das Fördergut als ein oder mehrere Materialpropfen durch die Leitung geschoben wird; Beladung > 40 kg/kg bis 200 kg/kg, Gasgeschwindigkeit 3 bis 10 m/s) als auch als Fließförderung (Gas-Feststoffgemisch verhält sich wie eine Kontinum, Beladungen bis 300 kg/kg oder 400 kg/kg, Gasgeschwindigkeit 7 bis 15 m/s) ausgeführt werden. Erfindungsgemäß bevorzugt wird die Flugförderung bei weitestgehender Vermeidung von Umlenkungen angewendet. Bevorzugtes Förderrohrmaterial ist Edelstahl.

Grundsätzlich unterliegt die angestrebte Geometrie der resultierenden Formkörper V beim erfindungsgemäßen Verfahren keiner Beschränkung. D. h., die geometrischen Formkörper V können sowohl regelmäßig als auch unregelmäßig geformt sein, wobei regelmäßig geformte Formkörper V erfindungsgemäß bevorzugt sind.

Beispielsweise kann der Formkörper V beim erfindungsgemäßen Verfahren Kugelgeometrie aufweisen. Dabei kann der Kugeldurchmesser z. B. 2 bis 10 mm, oder 4 bis 8 mm betragen.

Die Geometrie des Formkörpers V (des Katalysatorvorläuferformkörpers) kann aber auch vollzylindrisch oder hohlzyliridrisch (ringförmig) sein. In beiden Fällen können Außendurchmesser (A) und Höhe (H) z. B. 2 bis 10 mm, oder 2 bzw. 3 bis 8 mm betragen. Im Fall von Hohlzylindern (Ringen) ist in der Regel eine Wandstärke von 1 bis 3 mm zweckmäßig. Selbstverständlich kommen als Katalysatorvorläufergeometrie aber auch alle diejenigen Betracht, die in der WO 02/062737 offenbart und empfohlen werden.

Die in der Verfahrensstufe D) angewandten Formgebungsdrucke richten sich beim erfindungsgemäßen Verfahren nach der jeweiligen spezifischen Beschaffenheit des zu formenden Pulvers P bzw. P*. Im Allgemeinen betragen die in der Verfahrensstufe D) angewandten maximalen Formgebungsdrucke P2 500 bis 50 000 N/cm², vorzugsweise 2000 bis 35 000 N/cm², und besonders bevorzugt 6000 bis 25 000 N/cm².

Erfindungsgemäß bevorzugt erfolgt die Pressagglomeration in der Verfahrensstufe D) durch Tablettieren. Die Tablettierung kann dabei z. B. wie in den Schriften EP-A 184790, US 2005/0263926, JP-A 10/29097 und WO 2005/030393 beschrieben durchgeführt werden. Erfindungsgemäß bevorzugt wird die Tablettierung beim erfindungsgemäßen Verfahren wie in den Schriften DE-A 10 2008 040 093 und DE-A 10 2008 040 094 beschrieben durchgeführt, insbesondere bezüglich der Seitendruckfestigkeit SD des resultierenden ringförmigen bzw. ringähnlichen Formkörpers V.

Die experimentelle Bestimmung der Seitendruckfestigkeit wird dabei wie in den Schriften WO 2005/030393 sowie WO 2007/017431 beschrieben durchgeführt. Selbstverständlich sind ringähnliche Formkörper V, wie sie die DE-A 10 2008 040 093 empfiehlt, erfindungsgemäß ganz besonders bevorzugt. Die Stirnfläche von ringförmigen oder ringähnlichen Formkörpern V kann beim erfindungsgemäßen Verfahren sowohl gekrümmt als auch nicht gekrümmt sein (vgl. insbesondere DE-A 10 2007 004 961, EP-A 184790 und DE-A 10 2008 040 093). Bei der Ermittlung der Höhe solcher geometrischen Formkörper V wird eine solche Krümmjung nicht berücksichtigt.

Prinzipiell kann die thermische Behandlung von Formkörpern $V^+$ zum Erhalt der geometrischen Katalysatorformkörper K in den unterschiedlichsten Ofentypen wie z. B. beheizbare Umluftkammern (Umluftöfen), Hordenöfen, Drehrohröfen, Bandkalzinierern oder Schachtöfen durchgeführt werden. Erfindungsgemäß vorteilhaft erfolgt die thermische Behandlung der Formkörper $V^+$ in einer Bandkalzinierungsvorrichtung, wie sie die DE-A 100 46 957 und die WO 02/24620 empfehlen.

Eine Heißpunktausbildung innerhalb des Kalzinationsgutes wird dabei weitestgehend dadurch vermieden, dass mit Hilfe von Ventilatoren durch ein das Kalzinationsgut tragendes gasdurchlässiges Förderband erhöhte Volumenströme an Kalzinationsatmosphäre durch das Kalzinationsgut gefördert werden. Den Abschluss der Bandkalzinierung bildet in der Regel eine Kühlzone. In der Kühlzone befinden sich oberhalb und unterhalb des gasdurchlässigen Förderbandes von einem Kühlmedium durchströmte Kühlrippen. Mit Hilfe von Ventilatoren wird die abgeschlossene Gasatmosphäre der Kühlzone umgewälzt und die Kühlung derselben beim Kontakt mit den Kühlrippen bewirkt.

Die Zerkleinerung nicht intakter Formkörper V- auf die für das erfindungsgemäße Verfahren erforderliche Partikelgröße wird anwendungstechnisch zweckmäßig mit Hilfe einer Hammermühle (auch "Schlagmühle" genannt) durchgeführt (grundsätzlich können aber auch sonstige Mühlen verwendet werden). Die Zerkleinerung des Mahlguts geschieht dabei durch kinetische Schlagwirkung. In einem Metallgehäuse dreht sich ein Rotor, an dessen äußeren Umfang eine Anzahl beweglicher Stahlhämmer angebracht sind, die auf Umfangsgeschwindigkeiten von bis zu 120 m/s gebracht werden. Bei Eintritt in den Schlagkreis des Rotors trifft das Mahlgut auf die rotierenden Hämmer.

Durch den Schlag der Hämmer wird der größte Zerkleinerungseffekt erzielt. Die Hämmer schleudern die Stücke außerdem auf die Mahlwand, wo sie durch den Aufprall weiter gebrochen werden. Eine weitere Zerkleinerung erfolgt im unteren Bereich zwischen Rotor und Mahlwand. Das Mahlgut verbleibt so lange in der Zerkleinerungszone, bis es so klein ist, dass es durch ein Loch-(Öffnungs-)Sieb am äußeren Umfang der Maschine passt. Dem Sieb kommt die Funktion einer Begrenzung der Oberkrongröße zu.

Im Normalfall wird in der Hammermühle kein Zerkleinerungsgrad erreicht, der unterhalb desjenigen der feinteiligen Mischung M liegt. D. h., im Wesentlichen erfolgt in der Hammermühle nur ein Aufbrechen von kohäsiven Kontaktstellen der Primärpartikel in ihren durch das Pressagglomerieren gebildeten Agglomeraten.

Erfindungsgemäß bevorzugt wird vorstehende Zerkleinerung mit einer Hammermühle der Fa. Hosokava Alpine AG in D-86199 Augsburg durchgeführt.

Für den Fall, dass in der Verfahrensstufe B) für den Brechvorgang eine Siebmühle verwendet wird, kann das Sieb der Siebmühle aufgrund seiner hohen mechanischen Beanspruchung von Zeit zu Zeit brechen. Mittels eines über dem Feinkorn F-Schwingsieb angebrachten Schutzsieb werden für diesen Fall aus der Mühle austretende grobe Bruchstücke abgefangen. Sie können ebenfalls der Verfahrensstufe G) zugeführt und dort gemeinsam mit den Formkörpern V- zu dem in die Verfahrensstufe B) rückzuführenden Haufwerk H) zerkleinert werden (z. B. wie beschrieben in einer Hammermühle).

Die Partikeldurchmesser $d^P$ betragen beim erfindungsgemäßen Verfahren häufig auch ≤ 1,5 mm und vielfach ≤ 1 mm. Darüber hinaus betragen bei wenigstens 90 Gew.-% (vorzugsweise bei wenigstens 95 Gew.-% oder bei 100 Gew.-%), bezogen auf das Gesamtgewicht des Pulvers P, der Partikel des Pulvers P die Partikeldurchmesser $d^P$ ≥ 200 $\mu$m, oder ≥ 300 $\mu$m, oder sogar ≥ 400 $\mu$m.

Für das erfindungsgemäße Verfahren ist darüber hinaus von Vorteil, wenn die Partikeldurchmesser $d^H$ ≤ 150 $\mu$m, vorzugsweise ≤ 130 $\mu$m, besonders bevorzugt ≤ 110 $\mu$m und ganz besonders bevorzugt ≤ 100 $\mu$m betragen.

Es sei an dieser Stelle noch festgehalten, dass bei einer absatzweisen Rückführung von feinteiligem Gut bei erfindungsgemäßen Verfahren dieses feinteilige Gut zunächst standgeregelt in diesbezüglich vorgesehenen Sammelbehältern gesammelt wird. Beim Erreichen des festgelegten Füllstandes wird der jeweilige Sammelbehälter dann rückführend entleert.

Erfindungsgemäß hergestellte geometrische Katalysatorformkörper K eignen sich vor allem als Katalysatoren für heterogen katalysierte Partialoxidationen organischer Verbindungen. Im Besonderen sind dies die Partialoxidationen von Propen zu Acrolein, von iso-Buten bzw. tert.-Butanol bzw. dessen Methylether zu Methacrolein oder zu Methacrylnitril, von Propen zu Acrylnitril, von Acrolein zu Acrylsäure und von Methacrolein zu Methacrylsäure (Partialoxidationen im Beisein von Ammoniak, sogenannte Ammoxidationen, sollen in dieser Schrift ebenfalls unter dem Begriff Partialoxidationen subsumieren.

Alles bisher in dieser Schrift Gesagte hat vor allem Gültigkeit für die Herstellung der in den Schriften der Deutschen Anmeldungen mit den Aktenzeichen 102008040094.7, 102008040093.9, 102008042060.3, 102008042061.1 und 102008042064.6 beschriebenen geometrischen Katalysatorformkörper K.

Insbesondere hat es dann Gültigkeit, wenn das katalytisch wirksame (aktive) Multielementoxid des geometrischen (z. B. ringförmigen oder ringähnlichen) Multielementoxid-Vollkatalysators (Katalysatorformkörpers K) eine Stöchiometrie der allgemeinen Formel I,

$$Mo_{12}Bi_aFe_bX^1_cX^2_dX^3_eX^4_fO_n \qquad (I),$$

mit

| | | |
|---|---|---|
| $X^1$ = | Nickel und/oder Kobalt, |
| $X^2$ = | Thallium, Samarium, ein Alkalimetall und/oder ein Erdalkalimetall, |
| $X^3$ = | Zink, Phosphor, Arsen, Bor, Antimon, Zinn, Cer, Blei, Vanadium, Chrom, Niob und/oder Wolfram, |
| $X^4$ = | Silicium, Aluminium, Titan und/oder Zirkonium, |

$a$ =     0,2 bis 5,
$b$ =     0,01 bis 5,
$c$ =     0 bis 10,
$d$ =     0 bis 2,
$e$ =     0 bis 8,
$f$ =     0 bis 10, und
$n$ =     eine Zahl, die durch die Wertigkeit und Häufigkeit der von Sauerstoff verschiedenen Elemente in I bestimmt wird,

oder eine Stöchiometrie der allgemeinen Formel II,

$$[Y^1_{a'}Y^2_{b'}O_{x'}]_p[Y^3_{c'}Y^4_{d'}Y^5_{e'}Y^6_{f'}Y^7_{g'}Y^8_{h'}O_{y'}]_q \qquad (II),$$

mit

$Y^1$ =     nur Wismut oder Wismut und wenigstens eines der Elemente Tellur, Antimon, Zinn und Kupfer,
$Y^2$ =     Molybdän oder Wolfram, oder Molybdän und Wolfram,
$Y^3$ =     ein Alkalimetall, Thallium und/oder Samarium,
$Y^4$ =     ein Erdalkalimetall, Nickel, Kobalt, Kupfer, Mangan, Zink, Zinn, Cadmium und/oder Quecksilber,
$Y^5$ =     Eisen oder Eisen und wenigstens eines der Elemente Vanadium, Chrom und Cer,
$Y^6$ =     Phosphor, Arsen, Bor und/oder Antimon,
$Y^7$ =     ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,
$Y^8$ =     Molybdän oder Wolfram, oder Molybdän und Wolfram,
$a'$ =     0,01 bis 8,
$b'$ =     0,1 bis 30,
$c'$ =     0 bis 4,
$d'$ =     0 bis 20,
$e'$ >     0 bis 20,
$f'$ =     0 bis 6,
$g'$ =     0 bis 15,
$h'$ =     8 bis 16,
$x', y'$ =     Zahlen, die durch die Wertigkeit und Häufigkeit der von Sauerstoff verschiedenen Elemente in II bestimmt werden, und
$p, q$ =     Zahlen, deren Verhältnis $p/q$ 0,1 bis 10 beträgt,

aufweist.

Solche z. B. ringförmigen oder ringähnlichen Multielementoxid-Vollkatalysatoren eignen sich vor allem als Katalysatoren mit erhöhter Selektivität und Aktivität für die gasphasenkatalytische Partialoxidation von Propen zu Acrolein sowie von iso-Buten bzw. tert.-Butanol bzw. dessen Methylether zu Methacrolein.

Die Partialoxidation kann dabei z. B. wie in den Schriften WO 00/53557, WO 00/53558, deutsche Anmeldung Nr. 10 2008 040 093.9, deutsche Anmeldung Nr. 10 2008 040 094.7, DE-A 199 10 506, EP-A 1 106 598, WO 01/36364, DE-A 199 27 624, DE-A 199 48 248, DE-A 199 48 523, DE-A 199 48 241, EP-A 700 714, DE-A 103 13 213, DE-A 103 13 209, DE-A 10 2004 003 212 und DE-A 10 2005 013 039 beschrieben erfolgen.

Die Katalysatorbeschickung kann dabei nur geometrische Katalysatorformkörper K oder mit inerten Formkörpern verdünnte Katalysatorformkörper K umfassen. Im letzteren Fall wird die Katalysatorbeschickung vorteilhaft so gestaltet, dass ihre volumenspezifische Aktivität in Strömungsrichtung des Reaktionsgasgemischs zunimmt.

Die Herstellung solcher Katalysatorformkörper K findet sich z. B. beschrieben in den Schriften DE-A 10 2005 037 678, DE-A 102 007 003 778, DE-A 102 007 028 332 und in den deutschen Anmeldungen mit den Aktenzeichen 102008040094.7, 102008040093.9, 102008042060.3, 102008042061.1 und 102008042064.6.

In der erfindungsgemäßen Verfahrensstufe D) erfolgt die Formgebung dabei vorteilhaft so, dass die Seitendruckfestigkeit des resultierenden ringförmigen oder ringähnlichen Katalysatorformkörpers K $\geq 10$ und $\leq 40$ N, besser $\geq 10$ und $\leq 35$ N, noch besser $\geq 12$ N und $\leq 30$ N beträgt. Vorzugsweise beträgt die Seitendruckfestigkeit der ringähnlichen Katalysatorformkörper K $\geq 13$ N und $\leq 27$ N, bzw. $\geq 14$ N und $\leq 25$ N. Ganz besonders bevorzugt beträgt die Seitendruckfestigkeit der ringähnlichen Katalysatorformkörper K $\geq 15$ N und $\leq 22$ N.

Betreffend die Aktivmassen der Stöchiometrie II betragen der stöchiometrische Koeffizient b vorzugsweise 2 bis 4, der stöchiometrische Koeffizient c vorzugsweise 3 bis 10, der stöchiometrische Koeffizient d vorzugsweise 0,02 bis 2, der stöchiometrische Koeffizient e vorzugsweise 0 bis 5 und der stöchiometrische Koeffizient a vorzugsweise 0,4 bis 2. Der stöchiometrische Koeffizient f beträgt vorteilhaft 0,5 oder 1 bis 10. Besonders bevorzugt liegen die vorgenannten stöchiometrischen Koeffizienten gleichzeitig in den genannten Vorzugsbereichen.

Ferner ist $X^1$ vorzugsweise Kobalt, $X^2$ ist vorzugsweise K, Cs und/oder Sr, besonders bevorzugt K, $X^3$ ist bevorzugt Wolfram, Zink und/oder Phosphor und $X^4$ ist bevorzugt Si. Besonders bevorzugt weisen die Variablen $X^1$ bis $X^4$ gleichzeitig die vorgenannten Bedeutungen auf.

Besonders bevorzugt weisen alle stöchiometrischen Koeffizienten a bis f und alle Variablen $X^1$ bis $X^4$ gleichzeitig ihre vorgenannten vorteilhaften Bedeutungen auf.

Innerhalb der Stöchiometrien der allgemeinen Formel II sind jene bevorzugt, die der allgemeinen Formel III

$$[Bi_{a''}Z^2_{b''}O_{x''}]_{p''}[Z^8_{12}Z^3_{c''}Z^4_{d''}Fe_{e''}Z^5_{f''}Z^6_{g''}Z^7_{h''}O_{y''}]_{q''} \qquad (III),$$

mit

| | | |
|---|---|---|
| $Z^2 =$ | Molybdän oder Wolfram, oder Molybdän und Wolfram, | |
| $Z^3 =$ | Nickel und/oder Kobalt, | |
| $Z^4 =$ | Thallium, ein Alkalimetall und/oder ein Erdalkalimetall, vorzugsweise K, Cs und/oder Sr, | |
| $Z^5 =$ | Phosphor, Arsen, Bor, Antimon, Zinn, Cer, Vanadium, Chrom und/oder Bi, | |
| $Z^6 =$ | Silicium, Aluminium, Titan und/oder Zirkonium, vorzugsweise Si, | |
| $Z^7 =$ | Kupfer, Silber und/oder Gold, | |
| $Z^8 =$ | Molybdän oder Wolfram, oder Wolfram und Molybdän, | |
| $a'' =$ | 0,1 bis 1, | |
| $b'' =$ | 0,2 bis 2, | |
| $c'' =$ | 3 bis 10, | |
| $d'' =$ | 0,02 bis 2, | |
| $e'' =$ | 0,01 bis 5, vorzugsweise 0,1 bis 3, | |
| $f'' =$ | 0 bis 5, | |
| $g'' =$ | 0 bis 10, vorzugsweise > 0 bis 10, besonders bevorzugt 0,2 bis 10 und ganz besonders bevorzugt 0,4 bis 3, | |
| $h'' =$ | 0 bis 1, | |
| $x'', y'' =$ | Zahlen, die durch die Wertigkeit und Häufigkeit der von Sauerstoff verschiedenen Elemente in III bestimmt werden, und | |
| $p'', q'' =$ | Zahlen, deren Verhältnis $p''/q''$ 0,1 bis 5, vorzugsweise 0,5 bis 2 beträgt, | |

entsprechen.

Ferner sind katalytisch aktive Multielementoxide der Stöchiometrie II bevorzugt, die dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1_a \cdot Y^2_b \cdot O_{x'}$ enthalten, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Besonders vorteilhafte katalytisch aktive Multielementoxide der Stöchiometrie II sind solche, in denen $Y^1$ nur Wismut ist.

Innerhalb der katalytisch aktiven Multielementoxide der Stöchiometrie III sind diejenigen erfindungsgemäß bevorzugt, in denen $Z^2_{b''} = (Wolfram)_{b''}$ und $Z^8_{12} = (Molybdän)_{12}$ ist.

Ferner sind katalytisch aktive Multielemeritoxide der Stöchiometrie III bevorzugt, die dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Bi_{a''}Z^2_{b''}O_{x''}$ enthalten, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Auch ist es von Vorteil, wenn wenigstens 25 mol-% (bevorzugt wenigstens 50 mol-% und besonders bevorzugt wenigstens 100 mol-%) des gesamten Anteils $[Y^1_{a'}Y^2_{b'}O_{x'}]_p$ ($[Bi_{a''}Z^2_{b''}O_{x''}]_p$) der wie beschrieben erhältlichen katalytisch aktiven Multielementoxide der Stöchiometrie II (der Stöchiometrie III) in den katalytisch aktiven Multielementoxiden der Stöchiometrie II (der Stöchiometrie III) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung $Y^1_{a'}Y^2_{b'}O_{x'}$ ($[Bi_{a''}Z^2_{b''}O_{x''}]$) vorliegen, deren Größtdurchmesser im Bereich 1 nm bis 100 $\mu$m liegt.

Das in dieser Schrift Gesagte hat aber auch dann Gültigkeit, wenn das katalytisch wirksame (aktive) Multielementoxid des z. B. ringförmigen oder ringähnlichen Multielementoxid-Vollkatalysators (Katalysatorformkörpers K) eine Stöchiometrie der allgemeinen Formel IV,

$$Mo_{12}P_aV_bX^1_cX^2_dX^3_eSb_fRe_gS_hO_n \qquad (IV),$$

aufweist, in der Variablen folgende Bedeutung haben:

$X^1 =$     Kalium, Rubidium und/oder Cäsium,
$X^2 =$     Kupfer und/oder Silber,
$X^3 =$     Cer, Bor, Zirkonium, Mangan und/oder Wismut,
$a =$     0,5 bis 3,
$b =$     0,01 bis 3,
$c =$     0,2 bis 3,
$d =$     0,01 bis 2,
$e =$     0 bis 2,
$f =$     0 bis 2, vorzugsweise 0,01 bis 2,
$g =$     0 bis 1,
$h =$     0 bis 0,5, vorzugsweise 0,001 bis 0,5, und
$n =$     eine Zahl, die durch die Wertigkeit und Häufigkeit der von Sauerstoff verschiedenen Elemente in IV bestimmt wird.

Bevorzugt sind Multielementoxide IV, in denen h 0,03 bis 0,5 ist.

Besonders bevorzugte Stöchiometrien der allgemeinen Formel IV sind jene der Ausführungsbeispiele B1 bis B15 aus der EP-A 467 144 und dies auch dann, wenn diese beispielhaften Multielementoxide kein K und/oder kein Re enthalten.

Vorgenannte EP-A 467 144 und die Deutsche Anmeldung mit dem Aktenzeichen 102007003778.5 sowie die deutschen Anmeldungen mit den Aktenzeichen 102008040094.7 und 102008040093.9 beschreiben auch die Herstellung von ringförmigen Multielementoxid (IV)-Vollkatalysatorformkörpern und deren bevorzugte Verwendung als Katalysatoren für die heterogen katalysierte Gasphasenpartialoxidation von Methacrolein zu Methacrylsäure.

In überraschender Weise wirkt sich die erfindungsgemäße Rückführung im Wesentlichen auch nicht nachteilig auf die Langzeitstabilität der geometrischen Katalysatorformkörper im Partialoxidationsbetrieb aus.

Damit umfasst die vorliegende Anmeldung insbesondere die nachfolgenden Ausführungsformen:

1. Verfahren zur kontinuierlichen Herstellung von geometrischen Katalysatorformkörpern K, die als Aktivmasse ein Multielementoxid enthalten, das als von Sauerstoff verschiedene Elemente E das Element Mo, wenigstens eines der beiden Elemente Bi und V sowie wenigstens ein weiteres Element aus der Gruppe bestehend aus Co, Ni, Fe, Cu und den Alkalimetallen enthält, in Verfahrensstufen A) bis G), bei dem man

- in der Verfahrensstufe A) mit Hilfe von Quellen Q der Elemente E eine feinteilige Mischung M mit der Maßgabe erzeugt, dass höchstens 10 Gew.-% des Gesamtgewichts der in der feinteiligen Mischung M enthaltenen Partikel einen Partikeldurchmesser $d^M \geq 160$ $\mu$m aufweisen und der Partikeldurchmesser $d^M_{50}$ der Partikel der feinteiligen Mischung M die Bedingung 1 $\mu$m $\leq d^M_{50} \leq 150$ µm erfüllt;

- in der Verfahrensstufe B) das feinteilige Gemisch M*, das entweder nur aus der feinteiligen Mischung M oder aus einer Mischung aus der feinteiligen Mischung M und in der nachfolgenden Verfahrensstufe C) anfallendem und aus der Verfahrensstufe C) absatzweise oder kontinuierlich in die Verfahrensstufe B) rückgeführtem Feinkorn F besteht, durch Pressagglomeration, bei der der höchste angewandte Pressdruck P1 ist, zu Agglomeraten A verdichtet, deren Längstausdehnung ≥ 3 mm beträgt;

- in der Verfahrensstufe C) die Agglomerate A zerkleinert und das bei der Zerkleinerung gebildete körnige Gut durch Sieben in ein Pulver P, dessen Partikeldurchmesser $d^P \leq 2$ mm und zu wenigstens 90 Gew.-%, bezogen auf das Gewicht des Pulvers P, ≥ 160 $\mu$m betragen, als Siebüberkorn, und in Feinkorn F als Siebunterkorn auftrennt und das Feinkorn F kontinuierlich oder absatzweise zur Erzeugung von feinteiligem Gemisch M* in die Verfahrensstufe B) rückführt;

- in der Verfahrensstufe D) aus dem in selbige geförderten Pulver P oder aus einem Gemisch P*, bestehend aus dem in die Verfahrensstufe D) geförderten Pulver P und Formgebungshilfsmittel, durch Pressagglomeration, bei der der angewandte höchste Pressdruck P2 beträgt und die Beziehung P2 ≥ 2 • P1 erfüllt, mit der Maßgabe geometrische Formkörper V erzeugt, dass

- bei der Förderung des Pulvers P in die Verfahrensstufe D) und beim Einmischen von Formgebungshilfsmittel in das Pulver P insgesamt bei wenigstens 40 Gew.-% der Partikel des Pulvers P, bezogen auf dessen Gewicht, ein Partikeldurchmesser $d^P \geq 160$ $\mu$m erhalten bleibt; und

- in der Verfahrensstufe E) wenigstens eine Teilmenge der Formkörper V bei erhöhter Temperatur unter Erhalt der geometrischen Katalysatorformkörper K thermisch behandelt, dadurch gekennzeichnet, dass man

- vorab der Verfahrensstufe E) die in der Verfahrensstufe D) erzeugten Formkörper V in einer zusätzlichen Trennstufe als Verfahrensstufe F) in nicht intakte Formkörper V⁻ sowie in intakte Formkörper V⁺ auftrennt, die Formkörper V⁺ der Verfahrensstufe E) zuführt und

- in der Verfahrensstufe G) nicht intakte Formkörper V⁻ unter Ausbildung eines feinteiligen Haufwerks H, dessen Partikeldurchmesser $d_{50}^H$ die Bedingung $1\ \mu m \leq d_{50}^H \leq 150\ \mu m$ erfüllt und das zu höchstens 10 Gew.-% seines Gesamtgewichts Partikel mit einem Partikeldurchmesser $d^H \geq 160$ $\mu$m enthält, zerkleinert und das feinteilige Haufwerk H kontinuierlich oder absatzweise zur zusätzlichen Einarbeitung in das feinteilige Gemisch M* mit der Maßgabe in die Verfahrensstufe B) rückführt, dass der Gehalt des feinteiligen Gemischs M* an feinteiligem Haufwerk H, bezogen auf das Gesamtgewicht des feinteiligen Gemischs M*, 20 Gew.-% nicht überschreitet.

2. Verfahren gemäß Ausführungsform 1, dadurch gekennzeichnet, dass die Auftrennung der Formkörper V in der Verfahrensstufe F durch eine Siebtrennung vorgenommen wird, bei der die intakten Formkörper V⁺ als Siebrückstand verbleiben und der Siebdurchgang die Bruchstücke von nicht intakten Formkörpern V⁻ umfasst.

3. Verfahren gemäß einer der Ausführungsformen 1 oder 2, dadurch gekennzeichnet, dass in wenigstens einer der verschiedenen Verfahrensstufen B) bis G) die in dieser wenigstens einen Verfahrensstufe herrschende Gasatmosphäre abgesaugt und wenigstens einer mechanischen Trennoperation unterworfen wird, mit der in der Gasatmosphäre enthaltene Feststoffpartikel FP abgetrennt, und kontinuierlich oder absatzweise in die Verfahrensstufe B) rückgeführt und mit der Maßgabe in das feinteilige Gemisch M* eingearbeitet werden, dass der Gehalt des Gemischs M* an solchen rückgeführten Feststoffpartikeln FP, bezogen auf das Gesamtgewicht des Gemischs M*, 10 Gew.-% nicht überschreitet.

4. Verfahren gemäß Ausführungsform 3, dadurch gekennzeichnet, dass der Gehalt des Gemischs M* an rückgeführten Feststoffpartikeln FP 5 Gew.-% nicht überschreitet.

5. Verfahren gemäß einer der Ausführungsformen 3 oder 4, dadurch gekennzeichnet, dass die wenigstens eine mechanische Trennoperation die Filtration ist.

6. Verfahren gemäß einer der Ausführungsformen 1 bis 5, dadurch gekennzeichnet, dass die geometrischen Katalysatorformkörper K als Aktivmasse ein Multielementoxid enthalten, in welchem das Element Mo dasjenige von Sauerstoff verschiedene Element E ist, das von allen von Sauerstoff verschiedenen Elementen E des Multielementoxids mit der größten molaren Häufigkeit im Multielementoxid enthalten ist.

7. Verfahren gemäß einer der Ausführungsformen 1 bis 6, dadurch gekennzeichnet, dass die geometrischen Katalysatorformkörper K als Aktivmasse ein Multielementoxid enthalten, das, bezogen auf die molare Gesamtmenge seiner von molekularem Sauerstoff verschiedenen Elemente E, zu wenigstens 30 mol-% das Element Mo enthält.

8. Verfahren gemäß einer der Ausführungsformen 1 bis 7, dadurch gekennzeichnet, dass die geometrischen Katalysatorformkörper K als Aktivmasse ein Multielementoxid enthalten, das wenigstens eines der Alkalimetalle K, Na und Cs als Element E enthält.

9. Verfahren gemäß einer der Ausführungsformen 1 bis 8, dadurch gekennzeichnet, dass $1\ \mu m \leq d_{50}^{M} \leq 100\ \mu m$ ist.

10. Verfahren gemäß einer der Ausführungsformen 1 bis 9, dadurch gekennzeichnet; dass im Rahmen der Erzeugung der feinteiligen Mischung M wenigstens drei voneinander verschiedene Quellen Q von voneinander verschiedenen Elementen E in wässrigem Medium miteinander vermischt werden.

11. Verfahren gemäß einer der Ausführungsformen 1 bis 10, dadurch gekennzeichnet, dass das zu geometrischen Formkörpern V pressagglomerierte Pulver P oder P* wenigstens ein Ion aus der Gruppe bestehend aus Ammonium-, Formiat-, Acetat- und Nitrationen enthält, und der Gehalt $G^W$ des Pulvers oder P* an Wasser, bezogen auf das Gesamtgewicht der in ihm enthaltenen Gesamtmenge an Ammonium-, Formiat-, Acetat- und Nitrationen ≤ 60 Gew.-% beträgt.

12. Verfahren gemäß Ausführungsform 11, dadurch gekennzeichnet, dass $G^W$ ≤ 50 Gew.-% beträgt.

13. Verfahren gemäß Ausführungsform 11, dadurch gekennzeichnet, dass $G^W$ ≤ 40 Gew.-% beträgt.

14. Verfahren gemäß einer der Ausführungsformen 11 bis 13, dadurch gekennzeichnet, dass das Pulver P oder P* sauer ist.

15. Verfahren gemäß Ausführungsform 14, dadurch gekennzeichnet, dass das Pulver P oder P* Nitrationen enthält.

16. Verfahren gemäß einer der Ausführungsformen 1 bis 15, dadurch gekennzeichnet, dass die Längstausdehnung L der in der Verfahrensstufe B) erzeugten Agglomerate ≥ 0,5 cm beträgt.

17. Verfahren gemäß einer der Ausführungsformen 1 bis 15. dadurch gekennzeichnet, dass die Längstausdehnung L der in der Verfahrensstufe B) erzeugten Agglomerate ≥ 1 cm beträgt.

18. Verfahren gemäß einer der Ausführungsformen 1 bis 17. dadurch gekennzeichnet, dass die Pressagglomeration in der Verfahrensstufe B) mit einer Walzenpresse durchgeführt wird.

19. Verfahren gemäß einer der Ausführungsformen 1 bis 18, dadurch gekennzeichnet, dass P2 ≥ 3 • P1 ist.

20. Verfahren gemäß einer der Ausführungsformen 1 bis 18, dadurch gekennzeichnet, dass P2 ≥ 4 • P1 ist.

21. Verfahren gemäß einer der Ausführungsformen 1 bis 20, dadurch gekennzeichnet, dass der Formkörper V eine ringförmige Geometrie aufweist mit einem Außehdurchmesser A und einer Höhe H von 2 bis 10 mm und einer Wandstärke von 1 bis 3 mm.

22. Verfahren gemäß einer der Ausführungsformen 1 bis 21, dadurch gekennzeichnet, dass die Partikeldurchmesser $d^P$ ≤ 1 mm betragen.

23. Verfahren gemäß einer der Ausführungsformen 1 bis 22, dadurch gekennzeichnet, dass bei wenigstens 90 Gew.-%, bezogen auf das Gesamtgewicht des Pulvers P, der Partikel des Pulvers P der Partikeldurchmesser $d^P$ ≥ 200 $\mu$m ist.

24. Verfahren gemäß einer der Ausführungsformen 1 bis 23, dadurch gekennzeichnet, dass die Partikeldurchmesser $d^H$ ≤ 100 $\mu$m betragen.

25. Verfahren gemäß einer der Ausführungsformen 1 bis 24, dadurch gekennzeichnet, dass der Pressdruck P1 0,1 bis 5 k N/cm$^2$ ist.

26. Verfahren gemäß einer der Ausführungsformen 1 bis 25, dadurch gekennzeichnet, dass die Förderung des Pulvers P aus der Verfahrensstufe C) in die Verfahrensstufe D) saugpneumatisch oder druckpneumatisch erfolgt.

27. Verfahren gemäß einer der Ausführungsformen 1 bis 26, dadurch gekennzeichnet, dass der Gehalt des feinteiligen Gemisches M* an feinteiligem Haufwerk H, bezogen auf das Gesamtgewicht des feinteiligen Gemischs M*, 15 Gew.-% nicht überschreitet.

28. Verfahren gemäß einer der Ausführungsformen 1 bis 26, dadurch gekennzeichnet, dass der Gehalt des feinteiligen Gemisches M* an feinteiligem Haufwerk H, bezogen auf das Gesamtgewicht des feinteiligen Gemischs M*, 10 Gew.-% nicht überschreitet.

29. Verfahren gemäß einer der Ausführungsformen 1 bis 28, dadurch gekennzeichnet, dass der Gehalt des feinteiligen Gemisches M* an feinteiligem Haufwerk H, bezogen auf das Gesamtgewicht des feinteiligen Gemischs M*, wenigstens 1 Gew.-% beträgt.

30. Verfahren gemäß einer der Ausführungsformen 1 bis 28, dadurch gekennzeichnet, dass der Gehalt des feinteiligen Gemisches M* an feinteiligem Haufwerk H, bezogen auf das Gesamtgewicht des feinteiligen Gemischs M*, wenigstens 3 Gew.-% beträgt.

31. Verfahren gemäß einer der Ausführungsformen 1 bis 28, dadurch gekennzeichnet, dass der Gehalt des feinteiligen Gemisches M* an feinteiligem Haufwerk H, bezogen auf das Gesamtgewicht des feinteiligen Gemischs M*, wenigstens 5 Gew.-% beträgt.

32. Verfahren gemäß einer der Ausführungsformen 1 bis 31, dadurch gekennzeichnet, dass sich an ein Verfahren gemäß einer der Ausführungsförmen 1 bis 31 ein Verfahren der heterogen katalysierten partiellen Gasphasenoxidation einer organischen Verbindung, anschließt, bei dem als Katalysator wenigstens ein geometrischer Katalysatorformkörper K erhalten nach dem Verfahren gemäß einer der Ausführungsformen 1 bis 31 eingesetzt wird.

33. Verfahren gemäß Ausführungsform 32, dadurch gekennzeichnet, dass das Verfahren der heterogen katalysierten partiellen Gasphasenoxidation die Partial-oxidation von Propen zu Acrolein, von iso-Buten zu Methacrolein, von Propen zu Acrylnitril, von iso-Buten zu Methacrylnitril, von Acrolein zu Acrylsäure oder von Methacrolein zu Methacrylsäure ist.

Beispiele und Vergleichsbeispiele

I. Herstellung von ringförmigen Katalysatorformkörpern K und ringförmigen Vergleichskatalysatorformkörpern VK, deren Multielementoxidaktivmasse jeweils nachfolgende Stöchiometrie aufweist:

$$[Bi_2W_2O_9 \cdot 2WO_3]_{0,50}[Mo_{12}Co_{5,5}Fe_{3,0}Si_{1,5}K_{0,08}O_x]_1.$$

1. Ringförmige Vergleichskatalysatorformkörper VK1-1 (in den Verfahrensstufen B), C), D) und F) herrschte Luftatmosphäre (26° C und relative Luftfeuchtigkeit von 65 %)

Verfahrensstufe A)

a) Herstellung einer feinteiligen Ausgangsmasse 1 ( als Quelle der Elemente Bi und W)

In einem wassertemperierten 1,75-m$^3$-Doppelmantelbehälter (der Zwischenraum wurde von Temperierwasser durchströmt) aus Edelstahl (D (Durchmesser) = 1,3 m, h (Höhe) = 1,9 m) mit einem stufenlos regelbaren Balkenrührer (D = 0,8 m, h = 1,68 m) wurden in 780 kg einer eine Temperatur von 25°C aufweisenden wässrigen salpetersauren Wismutnitratlösung (11,2 Gew.-% Bi; freie Salpetersäure 3 bis 5 Gew.-%; Massendichte: 1,22 bis 1,27 g/ml, hergestellt mit Salpetersäure aus Wismutmetall der Firma Sidech S.A., 1495 Tilly, Belgien, Reinheit: > 99,997 Gew.-% Bi, < 7 mg/kg Pb, je < 5 mg/kg Ni; Ag, Fe, je < 3 mg/kg Cu, Sb und je < 1 mg/kg Cd, Zn) bei 25°C innerhalb von 20 min portionsweise 214,7 kg einer 25°C aufweisenden Wolframsäure (74,1 Gew.-% W, H.C. Starck, D-38615 Goslar, Reinheit > 99,9 Gew.-

% $WO_3$ nach Glühen bei 750 °C, 0,4 µm < $d_{50}$ < 0,8 µm) eingerührt (70 U/min). Das resultierende wässrige Gemisch wurde anschließend noch 3 h bei 25°C gerührt und dann sprühgetrocknet.

Die Sprühtrocknung erfolgte in einem Drehscheibensprühturm vom Typ FS 15 der Firma Niro A/S, DK-2860 Soeborg, im Heißluftgleichstrom bei einer Gaseintrittstemperatur von 300 ± 10 °C, einer Gasaustrittstemperatur von 100 ± 10°C, einer Scheibendrehzahl von 18000 U/min, einem Durchsatz von 200 l/h, einer Luftmenge von 1800 Nm³/h und einer Verweilzeit von 2,2 Minuten. Das resultierende Sprühpulver wies einen Glühverlust von 12,8 Gew.-% (3 h bei 600°C im Porzellantiegel (der bei 900 °C bis zur Gewichtskonstanz geglüht worden war) unter Luft glühen) und (bei einem Dispergierdruck von 1,1 bar absolut) einen $d_{50}$ von 28,0 µm ($d_{10}$ = 9,1 µm, $d_{90}$ = 55,2 µm) auf. Die nachfolgende Tabelle 1 gibt einen Überblick über repräsentative $d_x$-Werte des Sprühpulvers in µm in Abhängigkeit vom angewandten absoluten Dispergierdruck in bar:

Tabelle 1

|  | 2 bar | 1,5 bar | 1,2 bar | 1,1 bar |
|---|---|---|---|---|
| $d_{10}$ (µm) | 0,91 | 1,17 | 3,4 | 9,1 |
| $d_{50}$ (µm) | 5,8 | 8,5 | 19,7 | 28,0 |
| $d_{90}$ (µm) | 27,5 | 34,3 | 47,2 | 55,2 |

Das erhaltene Sprühpulver wurde anschließend mit 16,7 Gew.-% (bezogen auf das Sprühpulvergewicht) an 25 °C aufweisendem Wasser in einem Auspresskneter vom Typ VIU-160 der Firma AMK (Aachener Misch- und Knetfabrik, Peter Küpper GmbH & Co KG, 20 U/min) für 30 min angeteigt und mittels eines Extruders vom Typ G 103-10/D7A-752K der Firma Bonnot Company (Uniontown, Ohio, USA, Drehmoment: ≤ 50 Nm) zu Strängen des Durchmessers 6 mm extrudiert. Diese wurden in Abschnitte von 6 cm geschnitten, auf einem 3-zonigen Bandtrockner (Firma: Grenzebach BSH GmbH, D-36222 Bad Hersfeld) bei einer Verweilzeit von 120 min je Zone bei Lufttemperaturen von 90-95°C (Zone 1), 115 °C (Zone 2) und 125 °C (Zone 3) an Luft getrocknet und dann bei einer Temperatur von ca. 830°C (Außentemperatur des elektrisch beheizten Drehrohrofens) thermisch behandelt (kalziniert; im luftdurchströmten Drehrohrofen (im Gegenstrom, 0,3 mbar Unterdruck, 1,54 m³ Innenvolumen, 0,7 m Innendurchmesser, bei 4 m Länge des Ofens 7 cm Neigung, 8 mm Wanddicke, 200 Nm³/h Luft, 50 kg/h Extrudat, Drehzahl: 1 U/min)). Wesentlich bei der genauen Einstellung der Kalzinationstemperatur ist hier, dass sie an der angestrebten Phasenzusammensetzung des Kalzinationsprodukts orientiert erfolgt, das kalzinierte Material aber andererseits eine BET-Oberfläche ≥ 0,2 m²/g aufweist. Gewünscht sind hier die Phasen $WO_3$ (monoklin) und $Bi_2W_2O_9$ (orthorhombisch), unerwünscht ist das Vorhandensein von $\gamma$-$Bi_2WO_6$ (Russellit). Sollte daher nach der Kalzination der Gehalt der Verbindung $\gamma$-$Bi_2WO_6$ mehr als 5 Intensitäts-% betragen (berechnet als Verhältnis der Intensität des Reflexes von $\gamma$-$Bi_2WO_6$ im Röntgenpulverdiffraktogramm bei 2$\Theta$ = 28,4° (CuK$\alpha$-Strahlung) zur Intensität des Refleflexes von $Bi_2W_2O_9$ bei 20 = 30,0°), so ist die Präparation zu wiederholen und die Kalzinationstemperatur oder die Verweilzeit bei gleichbleibender Kalzinationstemperatur zu erhöhen, bis der Grenzwert erreicht wird. Das so erhaltene vorgebildete kalzinierte Mischoxid wurde mit einer Biplex-Querstromsichtmühle Typ 500 BQ der Firma Hosokawa Alpine AG, D-86199 Augsburg mit 2500 U/min gemahlen, so dass der $d_{50}$-Wert (bei einem Dispergierdruck von 2,0 bar absolut gemessen) 2,5 µm ($d_{10}$ = 1,1 µm, $d_{90}$ = 6,0 µm) und die BET-Oberfläche (DIN 66131, Stickstoffadsorption) 0,7 m²/g betrug. Der $\gamma$-$Bi_2WO_6$-Gehalt lag bei 3 Intensitäts-%.

Das Mahlgut wurde dann in Portionen von 20 kg in einem Schräglagen-Mischer (Typ VIS, Füllvolumen: 60 l, Aachener Misch- und Knetmaschinenfabrik) mit Misch- und Schneidflügel (Drehzahl Mischflügel:60 U/min, Drehzahl Schneidflügel: 3000 U/min) innerhalb von 5 min homogen mit 0,5 Gew.-% (bezogen auf das Mahlgut) feinteiligem hydrophobisiertem $SiO_2$ (Antibackmittel) der Fa. Degussa vom Typ Sipernat® D17 (Rüttelgewicht 150 g/l; $d_{50}$-Wert der $SiO_2$-Partikel (Laserbeugung nach ISO 13320-1) betrug 10 µm, die spezifische Oberfläche (Stickstoffadsorption nach ISO 5794-1, Annex D) betrug 100 m²/g) vermischt.

b) Herstellung einer feinteiligen Ausgangsmasse 2 aus verschiedenen Quellen Q

Eine Lösung A wurde hergestellt, indem man in einem wassertemperierten 1,75-m³-Doppelmantelbehälter (der Zwischenraum wurde von Temperierwasser durchströmt) aus Edelstahl (D = 1,3 m, h = 1,9 m) mit einem stufenlos regelbaren Balkenrührer (D = 0,8 m, h = 1,68 m) bei 60 °C unter Rühren (70 U/min) zu 660 l eine Temperatur von 60 °C aufweisendem Wasser innerhalb einer Minute 1,075 kg einer eine Temperatur von 60°C aufweisenden wässrigen Kaliumhydroxidlösung (47,5 Gew.-% KOH) und anschließend über eine Differenzialdosierwaage mit einer Dosiergeschwindigkeit von 600 kg/h 237,1 kg Ammoniumheptamolybdattetrahydrat (weiße Kristalle mit einer Körnung d < 1 mm, 81,5 Gew.-% $MoO_3$, 7,0-8,5 Gew.-% $NH_3$, max. 150 mg/kg Alkalimetalle, H.C. Stärck, D-38642 Goslar) dosierte und die resultierende leicht trübe Lösung bei 60 °C 60 min rührte (70 U/min).

Eine Lösung B wurde hergestellt, indem man in einem wassertemperierten 1,75-m³-Doppelmantelbehälter (der Zwischenraum wurde von Temperierwasser durchströmt) aus Edelstahl (D = 1,3 m, h = 1,9 m) mit einem stufenlos regelbaren Balkenrührer (D = 0,8 m, h = 1,68 m) bei 60 °C in 282,0 kg einer eine Temperatur von 60 °C aufweisenden wässrigen salpetersauren Kobalt(-II)-nitratlösung (pH (bei 25°C und 1 bar) = 4, 12,5 Gew.-% Co, hergestellt mit Salpetersäure aus Kobaltmetall der Firma MFT Metals & Ferro-Alloys Trading GmbH, D-41747 Viersen, Reinheit, >99,6 Gew.-%, < 0,3 Gew.-% Ni, < 100 mg/kg Fe, < 50 mg/kg Cu) vorlegte und zu dieser unter Rühren (70 U/min) 142,0 kg einer 60 °C warmen Eisen-(III)-nitrat-nonahydrat-Schmelze (13,8 Gew.-% Fe, < 0,4 Gew.-% Alkalimetalle, < 0,01 Gew.-% Chlorid, < 0,02 Gew.-%,Sulfat, Dr. Paul Lohmann GmbH, D-81857 Emmerthal) dosierte. Anschließend wurde unter Aufrechterhaltung der 60 °C 30 Minuten nachgerührt. Dann wurde unter Beibehalt der 60 °C die Lösung B innerhalb von 10 Minuten in die vorgelegte Lösung A abgelassen und weitere 15 Minuten mit 70 U/min bei 60 °C gerührt. Anschließend wurden dem resultierenden wässrigen Gemisch als Si-Quelle 19,9 kg eines Kieselgels der Fa. Grace vom Typ Ludox TM 50 (50,1 Gew.-% $SiO_2$, Dichte: 1,29 g/ml, pH 8,5 bis 9,5, Alkaligehalt max. 0,5 Gew.-%) zugegeben und danach noch weitere 15 Minuten mit 70 U/min bei 60 °C gerührt.

Anschließend wurde in einem Drehscheibensprühturm vom Typ FS-15 der Firma Niro A/S, DK-2860 Soeborg im Heißluftgegenstrom sprühgetrocknet (Gaseintrittstemperatur: 350 ± 10 °C, Gasaustrittstemperatur: 140 ± 5 °C, Scheibendrehzahl: 16000 U/min, Durchsatz: 270 kg/h, Luftmenge: 2100 $Nm^3$/h, Verweilzeit: 1,9 Minuten). Das resultierende Sprühpulver wies einen Glühverlust von 31,0 Gew.-% (3 h bei 600 °C unter Luft glühen), eine Restfeuchte (Wassergehalt) von 6,0 Gew.-% (gemessen (wie in allen Fällen dieser Schrift) mit dem Gerät Smart System 5 der Firma CEM Corporation (3100 Smith Farm Road, PO Box 200, Matthews, NC 28106, USA) als Gewichtsverlust beim Aufheizen mittels Mikrowellen (Maximalleistung der Geräts: 300 W; Messprogramm: Leistung: 45 % der Maximalleistung, Trocknungsdauer: 3 min., keine BIAS-Korrektur (gerätespezifisch), max. Temperatur (würde diese Probentemperatur erreicht, schaltet das Messgerät automatisch ab): 250 °C, Probengewicht: 0,06-0,10 g)), einen Stickstoffgehalt von 9,2 Gew.-%, einen Ammoniumgehalt von 5,4 Gew.-% und einen $d_{50}$ (bei einem Dispergierdruck von 2,0 bar absolut gemessen) von 36 $\mu$m ($d_{10}$ = 10 $\mu$m, $d_{90}$ = 72 $\mu$m) auf.

c) Herstellung der feinteiligen Mischung M

110 kg der feinteiligen Ausgangsmasse 2 wurden dann in einem aus Edelstahl 1.4541 gefertigten Schräglagen-Mischer (Typ VIL, Füllvolumen: 200 I, Aachener Misch- und Knetmaschinenfabrik) mit Misch- und Schneidflügel (Drehzahl Mischflügel: 39 U/min, Drehzahl Schneidflügel: 3000 U/min) vorgelegt und 1 min vorgemischt. Innerhalb von 10 min wurde hierzu bei fortgesetztem Mischen über eine Zellenradschleuse feinteilige Ausgangsmasse 1 in der für eine Multielementoxidaktivmasse der Stöchiometrie:

$$[Bi_2W_2O_9 \cdot 2WO_3]_{0,50}[Mo_{12}Co_{5,5}Fe_{3,0}Si_{1,5}K_{0,08}O_x]_1$$

erforderlichen Menge zudosiert. Dann wurde der Mischvorgang weitere 15 min fortgesetzt um eine (zur Erreichung einer hohen Aktivität und einer hohen Selektivität der Acroleinbildung der späteren ringförmigen Katalysatorformkörper erforderliche) intensive und vollständige Homogenisierung (einschließlich der Rückbildung gegebenenfalls entstandener Agglomerate) der beiden Ausgangsmassen zu erreichen. Bezogen auf die vorgenannte Gesamtmasse wurde unter Erhalt der feinteiligen Mischung M innerhalb von weiteren 2 min 1 Gew.-% Graphit TIMREX T44 der Firma Timcal AG ($d_{50}$ = 20,8 $\mu$m) untergemischt. Die feinteilige Mischung M wies keine Partikel mit einem Partikeldurchmesser $d^M$ ≥ 160 $\mu$m auf.

Verfahrensstufe B)

Herstellung der Agglömerate A

Die feinteilige Mischung M wurde dann in einer aus Edelstahl 1.4541 gefertigten Zweiwalzenpresse vom Typ Zweiwalzenkompaktor K200/100 der Fa. Hosokawa Bepex GmbH in D-74211 Leingarten mit konkaven (Tiefe = 2 mm), geriffelten (quer) Glattwalzen (Spaltweite: 2,8 mm, Walzendurchmesser: 20 cm, Walzendrehzahl: 9 UPM, Presskraftsollwert: ca. 75 kN, maximaler Pressdruck P1: 3,75 kN/cm$^2$) zu ca. 10 cm breiten und ca. 2,8 mm hohen Schülpen (Agglomerate A) pressagglomeriert.

Verfahrensstufe C)

Herstellung der Pulver P / P*

Die Schülpen wurden über einen aus Edelstahl 1.4541 gefertigten Stiftwalzenbrecher vom Typ GBM-406 und eine nachgeschaltete, aus Edelstahl 1.4541 gefertigte Schlagsiebmaschine vom Typ MGR-803 (beide Firma Frewitt

**EP 2 376 223 B1**

Maschinenfabrik AG, CH-1700 Fribourg) mit einem Rotor und einem Frewittsieb mit einer Maschenweite (quadratische Maschen aus Viereckdraht) von 1 mm zerkleinert. Über integrierte Schwingsiebe der Fa. Allgaier (Siebweite Überkorn (nur relevant bei defektem Frewittsieb): 1,5 mm, Siebweite Unterkorn: 400 $\mu$m) mit Gummiball-Klopfung (Gummiball-durchmesser = 22 mm) wurde ein Pulver P isoliert, dessen Partikeldurchmesser $d^P$ 400 $\mu$m $\leq d^P \leq$ 1 mm betrugen. Die Mengenverteilung zwischen dem Überkorn, dem gewünschten Pulver P und dem Feinkorn F (Unterkorn) betrug <1 Gew.-%: ca. 50 Gew.-%: ca. 50 Gew.-%. Das Feinkorn F wurde mittels saugpneumatischer Förderung vor den Zweiwalzenkompaktor zurückgeführt und im Gemisch M* mit neu zugeführter feinteiliger Mischung M erneut zu Schülpen pressagglomeriert.

Zur Herstellung der ringförmigen Formkörper V in der Verfahrensstufe D) wurden dem Pulver P in einem Bandschnecken-Mischer vom Typ S 5 der Firma Draiswerke GmbH in D-68305 Mannheim, innerhalb von 2 min weitere 2,5 Gew.-% des Graphits TIMREX T44 der Firma Timcal AG zugemischt und das dabei resultierende Gemisch P* mittels saugpneumatischer Förderung zur Tablettiermaschine transportiert. Im mit der Tablettiermaschine tablettierten Gemisch lag der Gewichtsanteil mit $d^P \geq$ 160 $\mu$m bei 85 Gew.-% (Bestimmung durch Siebanalyse; Bestimmung wie bei allen entsprechenden Siebanalysen dieser Schrift durch 5-minütiges Sieben bei maximaler Amplitude mit einem Prüfsieb der Maschenweite 160 $\mu$m und dem Siebapparat AF 200 der Fa. Retsch GmbH in 42781 Haan, Reinische Strasse 36), bei einer Rütteldichte des Gemischs von 1250 g/l (zur Bestimmung werden ca. 0,75 l Gemisch innerhalb von ca. 90 s über eine Dosierrinne (Firma Retsch, Modell DR 1000) und einen Schüttrichter in einen auf einem Stampfvolumeter (Firma JEL, Modell STAV 2003) befindlichen 1-l-Messzylinder aus Polymethylpenten (Firma Nalgene, Buddenberg-Katalog 95/96 Best.-Nr. 9.274 927) dosiert und für 3,08 min mit 700 Schlägen verdichtet, die Rütteldichte ergab sich dann als Quotient aus eingefüllter Masse und im Messzylinder eingenommenem Volumen) und einer Restfeuchte (einem Wassergehalt) von 5,7 Gew.-%. Das Gewichtsverhältnis von Restfeuchte zu Gewichtsverlust bei 450 °C (3 h bei 450°C im Porzellantiegel (der bei 900°C bis zur Gewichtskonstanz geglüht worden war) unter Luft erhitzen) betrug 22 %. $G^w$ betrug 28 Gew.-%.

Verfahrensstufe D)

Herstellung der ringförmigen Formkörper V

Anschließend wurde das zur Tablettiermaschine geförderte Pulver P* in derselben Tablettiermaschine (ein Korsch-Rundläufer vom Typ PH 865 der Fa. Korsch) wie im Beispiel II. der deutschen Anmeldung Nr. 102008040093.9 beschrieben bei einer Drehzahl des Tablettiertellers von ca. 40 UPM unter Luftatmosphäre zu ringförmigen Formkörpern V der Geometrie 5 mm x 3 mm x 2 mm (Außendurchmesser x Höhe x Innendurchmesser) mit einer Seitendruckfestigkeit von 19 N tablettiert. Die Hauptpresskraft betrug ca. 4,2 kN. Der maximale Pressdruck P2 betrug 25,5 kN/cm². Um Staubfreisetzung zu vermeiden wurde die Tablettiermaschiene abgesaugt (300 bis 400 Nm³/h). Die Abluft wurde über einen Filter vom Typ HSL 900-8/8 SZ der Firma Herding in D-92224 Amberg geführt, der unter Gewinnung des aus Feststoffpartikeln FP bestehenden Filterkuchens periodisch abgereinigt wurde.

Verfahrensstufe E)

Thermische Behandlung der ringförmigen Formkörper V unter Erhalt der ringförmigen Vergleichskatalysatorformkörper VK1-1

Zur abschließenden thermischen Behandlung wurden jeweils 1000 g einer repräsentativen Mischprobe der wie beschrieben hergestellten ringförmigen Formkörper V gleichmäßig aufgeteilt auf nebeneinander angeordnete 4 Gitternetze mit einer quadratischen Grundfläche von jeweils 150 mm x 150 mm (Schütthöhe: ca. 15 mm) in einem mit 1000 Nl/h Luft durchströmten Umluftofen (Firma Heraeus Instruments GmbH, D-63450 Hanau, Typ: K 750/2) zunächst mit einer Aufheizrate von 80°C/h von Raumtemperatur (25 °C) auf 185 °C aufgeheizt. Diese Temperatur wurde für 1 h aufrechterhalten und dann mit einer Aufheizrate von 48 °C/h auf 225 °C erhöht. Die 225 °C wurden für 2 h gehalten, bevor sie mit einer Aufheizrate von 120 °C/h auf 270 °C erhöht wurde. Diese Temperatur wurde ebenfalls 1 h aufrechterhalten, bevor sie, mit einer Aufheizrate von 60°C/h, auf 464°C erhöht wurde. Diese Endtemperatur wurde für 10 Stunden gehalten. Danach wurde auf Raumtemperatur abgekühlt und die ringförmigen Vergleichskatalysatorformkörper VK1-1 mit einer Seitendruckfestigkeit von 6,8 N und einer Massendichte von 1,92 g/ml erhalten.

Bei einer anschließenden Unterkornsiebung mit einem Langlochsieb aus Edelstahl 1.4541 (gerade Kantenlänge: 20 mm, Kantenabstand: 1,8 mm) fiel, bezogen auf das Gewicht des insgesamt zur Siebung aufgegebenen Siebgutes, ein Unterkornanteil von 2,3 Gew.-% an.

Anstatt die thermische Behandlung wie vorstehend beschrieben durchzuführen, kann man sie auch wie in Beispiel 1 der DE-A 100 46 957 (die Schütthöhe in der Zersetzung (Kammern 1 bis 4) beläuft sich dabei jedoch vorteilhaft auf 42 mm bei einer Verweilzeit pro Kammer von 1,23 h und in der Kalzination (Kammern 5 bis 8) beläuft sie sich vorteilhaft auf 130 mm bei einer Verweilzeit von 3,89 h) beschrieben mittels einer Bandkalziniervorrichtung durchführen. Die Kammern besitzen eine Grundfläche (bei einer einheitlichen Kammerlänge von 1,40 m) von 1,29 m² (Zersetzung) und 1,40 m² (Kalzination) und werden von unten durch das grobmaschige Förderband von 50-150 Nm³/h auf 100 °C (Zersetzung) bzw. 450 °C (Kalzination) vorgeheizte Zuluft durchströmt. Zusätzlich wird die Luft durch rotierende Ventilatoren (900 bis 1500 U/min) umgewälzt. Innerhalb der Kammern ist die zeitliche und örtliche Abweichung der Temperatur vom Sollwert (typische Werte für die Zonen 1-8 sind: 140 °C, 190 °C, 220 °C, 265 °C, 380 °C, 425 °C, 460 °C, 460 °C) stets ≤ 2°C. Hinter Kammer 8 schließt sich vorteilhafterweise eine (mittels Wasserkühlung in Kühlrippen) auf 70 °C temperierte 2 m lange Kühlzone an. Im Übrigen wird wie in Beispiel 1 der DE-A 100 46 957 beschrieben verfahren. Die resultierenden Vergleichskatalysatorformkörper VK1-1 eignen sich in gleicher Weise als Katalysatoren für die Partialoxidation von Propylen zu Acrolein. Sie können zur Abtrennung von Agglomeraten noch über ein 5 mm x 20 mm LanglochSchlitzsieb und zur Abtrennung von gebildetem Bruch über ein (bevorzugt zwei) Schlitzsiebe (1,8 mm x 20 mm Langloch-Schlitz) geführt werden.

In einer alternativen Ausführungsform wurden alle Verfahrensstufen identisch wiederholt. Nach der Verfahrensstufe D) wurden die in selbiger erzeugten ringförmigen Formkörper V mittels einer Schwingsiebmaschine E.A. Typ 36-2 von der Engelsmann AG in D-67059 Ludwigshafen am Rhein, über ein Edelstahl 1.4541 Schlitzsieb (rechteckige Schlitze der Länge 30 mm und der Breite 1,8 mm) geführt und als Verfahrensstufe F in intakte Formkörper V⁺ (ca. 98 Gew.-%) als Überkorn und nicht intakte Formkörper V-(ca. 2 Gew.-%) aufgetrennt. Wie bereits beschrieben durchgeführte thermische Behandlung der ringförmigen Formkörper V⁺ im Umluftofen lieferte wieder die Vergleichskatalysatorformkörper VK1-1. Bei einer anschließenden Unterkornsiebung derselben mit einem Langlochsieb aus Edelstahl 1.4541 (gerade Kantenlänge: 20 mm, Kantenabstand: 1,8 mm) fiel, bezogen auf das Gewicht des insgesamt zur Siebung aufgegebenen Siebgutes, nur noch ein Unterkornanteil von 0,2 Gew.-% an.

2. Ringförmige Katalysatorformkörper K1-1 (in den Verfahrensstufen B), C), D), F) und G) herrschte Luftatmosphäre (26° und relative Luftfeuchtigkeit von 65 %)

Das Herstellverfahren entsprach jenem zur Herstellung der Vergleichskatalysatorformkörper VK1-1, jedoch mit folgenden Unterschieden:

a) Die in der Verfahrensstufe F) abgetrennten Formkörper V- wurden als Verfahrensstufe G) mit einer Hammermühle der Fa. Hosokawa Alpine AG, D-86199 Augsburg aufgemahlen (Partikeldurchmesser 1 μm < d$^H$ < 100 μm; Siebanalyse). Das resultierende feinteilige Haufwerk H wurde in einem geschlossenen Sammelbehälter zwischengelagert und von dort saugpneumatisch vor die Verfahrensstufe B) rückgeführt und in das Gemisch M* eingearbeitet (mit einem Gewichtsanteil von 20 Gew.-% bezogen auf das Gesamtgewicht).

b) Der in der Verfahrensstufe D) gewonnene Filterkuchen aus Feststoffpartikeln FP wurde in einem geschlossenen Sammelbehälter gesammelt und von dort saugpneumatisch vor die Verfahrensstufe B) rückgeführt und ebenfalls in das Gemisch M* eingearbeitet (mit einem Gewichtsanteil von 2 Gew.-% bezogen auf das Gesamtgewicht) und das Gemisch M* zu Schülpen pressagglomeriert.

Im mit der Tablettiermaschine tablettierten Gemisch lag der Gewichtsanteil mit d$^P$ ≥ 160 μm bei 79 Gew.-% (Bestimmung durch Siebanalyse), bei einer Rütteldichte des Gemischs von 1300 g/l und einer Restfeuchte (einem Wassergehalt) von 6,5 Gew.-%. Das Gewichtsverhältnis von Restfeuchte zu Gewichtsverlust bei 450 °C (3 h bei 450°C im Porzellantiegel (der bei 900°C bis zur Gewichtskonstanz geglüht worden war) unter Luft erhitzen) betrug 25 %. G$^W$ betrug 33 Gew.-%. Die Seitendruckfestigkeit der resultierenden Formkörper V betrug 22 N. Die resultierenden Katalysatorformkörper K1-1 wiesen eine Seitendruckfestigkeit von 6,3 N und eine Massendichte von 1,93 g/ml auf.

3. Ringförmige Katalysatorformkörper K1-2 (in den Verfahrensstufen B), C), D), F) und G) herrschte Luftatmosphäre mit erhöhter Luftfeuchtigkeit (31° und relative Luftfeuchtigkeit von 89 %)

Das Herstellverfahren entsprach jenem zur Herstellung der Katalysatorformkörper K1-1, jedoch mit dem Unterschied einer erhöhten Luftfeuchtigkeit in den Verfahrensstufen B), C), D), F) und G). Im mit der Tablettiermaschine tablettierten Gemisch lag der Gewichtsanteil mit d$^P$ ≥ 160 μm bei 83 Gew.-% (Bestimmung durch Siebanalyse), bei einer Restfeuchte (einem Wassergehalt) von 9,9 Gew.-%. Das Gewichtsverhältnis von Restfeuchte zu Gewichtsverlust bei 450 °C (3 h bei 450°C im Porzellantiegel (der bei 900°C bis zur Gewichtskonstanz geglüht worden war) unter Luft erhitzen) betrug 35 %. G$^W$ betrug 54 Gew.-%. Die Seitendruckfestigkeit der resultierenden Formkörper V betrug 23 N. Die resultierenden Katalysatorformkörper K1-2 wiesen eine Seitendruckfestigkeit von 6,1 N und eine Massendichte von 1,91 g/ml auf.

4. Ringförmige Katalysatorformkörper K1-3 (in den Verfahrensstufen B), C), D), F) und G) herrschte Luftatmosphäre mit erhöhter Luftfeuchtigkeit (31 ° und relative Luftfeuchtigkeit von 89 %)

Das Herstellverfahren entsprach jenem zur Herstellung der Katalysatorformkörper K1-2, jedoch mit dem Unterschied, dass das feinteilige Haufwerk H vor seiner Rückführung vor die Verfahrensstufe B) 24 Stunden in einem offenen Sammelbehälter zwischengelagert worden war. Im mit der Tablettiermaschine tablettierten Gemisch lag der Gewichtsanteil mit $d^P \geq 160$ μm bei 86 Gew.-% (Bestimmung durch Siebanalyse), bei einer Restfeuchte (einem Wassergehalt) von 12,5 Gew.-%. Das Gewichtsverhältnis von Restfeuchte zu Gewichtsverlust bei 450 °C (3 h bei 450°C im Porzellantiegel (der bei 900°C bis zur Gewichtskonstanz geglüht worden war) unter Luft erhitzen) betrug 42 %. $G^W$ betrug 72 Gew.-%. Die Seitendruckfestigkeit der resultierenden Formkörper V betrug 24 N. Die resultierenden Katalysatorformkörper K1-3 wiesen eine Seitendruckfestigkeit von 5,9 N und eine Massendichte von 1,90 g/ml auf.

II. Testung der in I. hergestellten ringförmigen Katalysatoren in einer heterogen katalysierten partiellen Gasphasenoxidation von Propen zu Acrolein

Ein Reaktionsrohr (V2A Stahl; 21 mm Außendurchmesser, 3 mm Wandstärke, 15 mm Innendurchmesser, Länge 120 cm) wurde von oben nach unten in Strömungsrichtung wie folgt beschickt:

Abschnitt 1: ca. 30 cm Länge
40 g Steatitkugeln (C220 Steatit der Fa. Ceram Tec) mit einem Durchmesser von 1,5 bis 2,0 mm als inerte Vorschüttung (Äufheizzone).

Abschnitt 2: ca. 70 cm Länge
100 g des jeweiligen in I. hergestellten ringförmigen Katalysators.

Die Temperierung des Reaktionsrohres erfolgte jeweils mittels eines mit Stickstoff geperlten Salzbades (53 Gew.-% Kaliumnitrat, 40 Gew.-% Natriumnitrit und 7 Gew.-% Natriumnitrat).
Der Reaktor wurde kontinuierlich mit einem Reaktionsgasausgangsgemisch (Beschickungsgasgemisch aus Luft, polymer grade Propylen und Stickstoff) der nachfolgenden Zusammensetzung beschickt:

5 Vol.-% Propen (polymer grade),
9,5 Vol-% molekularer Sauerstoff und
85,5 Vol.-% molekularer Stickstoff.

Bei einem in das Reaktionsrohr geführten Volumenstrom des Reaktionsgasausgangsgemischs (dessen Eintrittstemperatur in das Reaktionsrohr ca. 30°C betrug) von 100 Nl/h (5 Nl/h Propen (polymer grade)) erfolgte die Thermostatisierung des Reaktionsrohres durch Variation der Salzbadtemperatur $T^S$ (°C) in allen Fällen so, dass der Propenumsatz $U^P$ (mol-%) bei einmaligem Durchgang des Beschickungsgasgemischs durch das Reaktionsrohr kontinuierlich ca. 95 mol-% betrug.
Die nachfolgende Tabelle 2 zeigt (jeweils bezogen auf einen Einmaldurchgang des Reaktionsgasgemischs durch das Reaktionsrohr) den nach einer Betriebszeit von 60 h für die jeweilige Katalysatorbeschickung des Reaktionsrohres bei der jeweiligen Temperatur $T^S$ exakt vorliegenden Propylenumsatz $U^P$, die zugehörige Selektivität $S^{AC}$ der Acroleinbildung (mol-%), und die für den Fall einer in einer nachfolgenden Reaktionsstufe durchgeführten partiellen Oxidation des gebildeten Acroleins zu Acrylsäure wesentliche Selektivität $S^{AC + AA}$ der Gesamtbildung von Acrolein und Acrylsäure (mol-%).

Tabelle 2

| Katalysator | $T^S$ (°C) | $U^P$ (mol-%) | $S^{AC}$ (mol-%) | $S^{AC + AA}$ (mol-%) |
|---|---|---|---|---|
| VK1-1 | 331 | 95,3 | 86,3 mol-% | 93,9 mol-% |
| K1-1 | 329 | 95,2 | 85,6 mol-% | 93,5 mol-% |
| K1-2 | 332 | 95,1 | 85,1 mol-% | 93,3 mol-% |
| K1-3 | 335 | 95,1 | 84,3 mol-% | 92,6 mol-% |

In überraschender Weise führt die erfindungsgemäße Rückführung bei der Herstellung der ringförmigen Katalysatoren insbesondere bei $S^{AC + AA}$ nur zu marginalen Verlusten, die sich bei einer geringen Restfeuchte (geringem Wassergehalt) des Rückführgutes und damit einer geringen Restfeuchte sowie einem geringen $G^W$ der Formkörper V am Rande der Messgenauigkeit bewegen.

III. Herstellung von ringförmigen Katalysatorformkörpern K und ringförmigen Vergleichskatalysatorformkörpern VK, deren Multielementoxidaktivmasse jeweils nachfolgende Stöchiometrie aufweist:

$$Mo_{12}Bi_{0,6}Co_7Fe_{3,0}Si_{1,6}K_{0,08}O_x.$$

1. Ringförmige Vergleichskatalysatorformkörper VK2-1 (in den Verfahrensstufen B), C), D) und F) herrschte Luftatmosphäre (26°C und relative Luftfeuchtigkeit von 60%)

Verfahrensstufe A)

Herstellung der feinteiligen Mischung M aus verschiedenen Quellen Q

Eine Lösung A wurde hergestellt, indem man in einem wassertemperierten 1,75-m$^3$-Doppelmantelbehälter (der Zwischenraum wurde von Temperierwasser durchströmt) aus Edelstahl (D = 1,3 m, h = 1,9 m) mit einem stufenlos regelbaren Balkenrührer (D = 0,8 m, h = 1,68 m) bei 60 °C unter Rühren (70 U/min) zu 432 l eine Temperatur von 65 °C aufweisendem Wasser innerhalb einer Minute 0,62 kg einer eine Temperatur von 25 °C aufweisenden wässrigen Kaliumhydroxidlösung (47,5 Gew.-% KOH) und anschließend über eine Differenzialdosierwaage mit einer Dosiergeschwindigkeit von 600 kg/h 139,7 kg 25°C aufweisendes Ammoniumheptamolybdattetrahydrat (weiße Kristalle mit einer Körnung d < 1 mm, 81,5 Gew:-% MoO$_3$, 7,0-8,5 Gew.-% NH$_3$, max. 150 mg/kg Alkalimetalle, H.C. Starck, D-38642 Goslar) dosierte und die resultierende Lösung anschließend noch 90 min bei 60 °C rührte (70 U/min).

Eine Lösung B wurde hergestellt, indem man in einem wassertemperierten 1,75-m$^3$-Doppelmantelbehälter (der Zwischenraum wurde von Temperierwasser durchströmt) aus Edelstahl (D = 1,3 m, h = 1,9 m) mit einem stufenlos regelbaren Balkenrührer (D = 0,8 m, h = 1,68 m) bei 60°C in 217,5 kg einer eine Temperatur von 60 °C aufweisenden wässrigen salpetersauren Kobalt(-II)nitratlösung (pH = 4 (25 °C, 1 atm), 12,5 Gew.-% Co, hergestellt mit Salpetersäure aus Kobaltmetall der Firma MFT Metals & Ferro-Alloys Trading GmbH, D-41747 Viersen, Reinheit, >99,6 Gew.-%, < 0,3 Gew.-% Ni, < 100 mg/kg Fe, < 50 mg/kg Cu) vorlegte, und zu dieser unter Rühren (70 U/min) 80,0 kg einer 60 °C warmen Eisen-(III)-nitrat-nonahydrat-Schmelze (13,8 Gew.-% Fe, < 0,4 Gew.-% Alkalimetalle, < 0,01 Gew.-% Chlorid, < 0,02 Gew.-% Sulfat, Dr. Paul Lohmann GmbH, D-81857 Emmerthal) dosierte. Anschließend wurde unter Aufrechterhaltung der 60 °C noch 30 Minuten nachgerührt. Dann wurden unter fortgesetztem Rühren und unter Beibehalt dieser Temperatur 74,5 kg wässriger salpetersaurer Wismutnitratlösung (11,1 Gew.-% Bi; freie Salpetersäure 3 bis 5 Gew.-%; Massendichte: 1,22 bis 1,27 g/ml, hergestellt mit Salpetersäure aus Wismutmetall der Firma Sidech S.A., 1495 Tilly, Belgien, Reinheit: > 99,997 Gew.-% Bi, < 7 mg/kg Pb, je < 5 mg/kg Ni, Ag, Fe, je < 3 mg/kg Cu, Sb und je < 1 mg/kg Cd, Zn) eingerührt und anschließend unter Aufrechterhaltung der 60 °C nochmals 30 Minuten nachgerührt. Dann wurde unter Beibehalt der 60 °C und unter fortgesetztem Rühren innerhalb von 15 Minuten die Lösung B in die vorgelegte Lösung A abgelassen, der Kessel mit 5 l Wasser nachgespült und die gebildete Suspension weitere 15 Minuten mit 70 U/min bei 60 °C gerührt. Anschließend wurden dem resultierenden wässrigen Gemisch als Si-Quelle 10 l eines Kieselgels der Fa. Grace vom Typ Ludox TM 50 (49,1 Gew.-% SiO$_2$, Dichte: 1,29 g/ml, pH 8,5 bis 9,5, Alkaligehalt max. 0,5 Gew.-%) zugegeben und danach noch weitere 15 Minuten mit 70 U/min bei 60 °C gerührt.

Anschließend wurde in einem Drehscheibensprühturm vom Typ FS-15 der Firma Niro A/S, DK-2860 Soeborg im Heißluftgegenstrom sprühgetrocknet (Gaseintrittstemperatur: 350 ± 10 °C, Gasaustrittstemperatur: 140 ± 5 °C, Scheibendrehzahl: 18000 U/min, Durchsatz: 270 kg/h, Luftmenge: 2100 Nm$^3$/h, Verweilzeit: 1,9 Minuten). Das resultierende Sprühpulver wies einen Glühverlust von 30,0 Gew.-% (3 h bei 600°C unter Luft glühen), eine Restfeuchte von 6,5 Gew.-% und einen $d_{50}$ (bei einem Dispergierdruck von 2,0 bar absolut gemessen) von 27 $\mu$m ($d_{10}$ = 4,7 $\mu$m, $d_{90}$ = 59 $\mu$m) auf.

100 kg dieses Sprühpulvers und 1 kg Graphit Asbury 3160 (Firma Asbury Graphite Mills, Inc. New Jersey 08802, USA) mit einem $d_{50}$ von 123 $\mu$m wurden dann in einem Schräglagen-Mischer (Typ VIL, Füllvolumen: 200 l, Aachener Misch- und Knetmaschinenfabrik) mit Misch- aber ohne Schneidflügel (Drehzahl Mischflügel: 39 U/min) 5 Minuten unter Erhalt der feinteiligen Mischung M gemischt. Die feinteilige Mischung M enthielt weniger als 1 Gew.-% an Partikeln mit einem Partikeldurchmesser $d^M \geq 160$ $\mu$m.

Verfahrensstufe B)

Herstellung der Agglomerate A

Die feinteilige Mischung M wurde dann in einer aus Edelstahl 1.4541 gefertigten Zweiwalzenpresse vom Typ Zweiwalzenkompaktor K200/100 der Fa. Hosokawa Bepex GmbH mit konkaven (Tiefe = 2 mm), geriffelten (quer) Glattwalzen (Spaltweite: 2,8 mm, Walzendrehzahl: 10 UPM, Presskraftsollwert: ca. 35 kN, maximaler Pressdruck P1: 1,75 kN/cm$^2$) zu ca. 10 cm breiten und ca. 2,8 mm hohen Schülpen (Agglomeraten A) pressagglomeriert.

Verfahrensstufe C)

Herstellung der Pulver P/P*

Die Schülpen wurden über einen aus Edelstahl 1.4541 gefertigten Stiftwalzenbrecher vom Typ GBM-406 und eine nachgeschaltete, aus Edelstahl 1.4541 gefertigte Schlagsiebmachiene vom Typ MGR-803 (beide Firma Frewitt Maschinenfabrik AG, CH-1700 Fribourg) mit einem Rotor und einem Frewittsieb mit einer Maschenweite (quadratische Maschen aus Viereckdraht) von 1 mm zerkleinert. Über integrierte Schwingsiebe der Fa. Allgaier (Siebweite Überkorn (nur relevant bei defektem Frewittsieb): 1,5 mm, Siebweite Unterkorn: 200 $\mu$m) mit Gummiball-Klopfung (Gummiballdurchmesser = 22 mm) wurde ein Pulver P isoliert, dessen Partikeldurchmesser d$^P$ 200 $\mu$m $\leq$ d$^P$ $\leq$ 1 mm betrugen. Die Mengenverteilung zwischen dem Überkorn, dem gewünschten Pulver P und dem Feinkorn F (Unterkorn) betrug <1 Gew.-%: ca. 60 Gew.-%: ca. 40 Gew.-%. Das Feinkorn F wurde mittels saugpneumatischer Förderung vor den Zweiwalzenkompaktor zurückgeführt und im Gemisch M* mit neu zugeführter feinteiliger Mischung M erneut zu Schülpen pressagglomeriert.

Zur Herstellung der ringförmigen Formkörper V in der Verfahrensstufe D) wurden dem Pulver P in einem Turbulent-Mischer vom Typ S5 der Firma Draiswerke GmbH in D-68305 Mannheim, innerhalb von 2 min weitere 2 Gew.-% des Graphits Asbury 3160 zugemischt und das dabei resultierende Pulver P* mittels saugpneumatischer Förderung zur Tablettiermaschine transportiert. Im mit der Tablettiermaschine tablettierten Gemisch lag der Gewichtsanteil mit d$^P$ $\geq$ 160 $\mu$m bei 78,5 Gew.-% (Bestimmung durch Siebanalyse) und die Restfeuchte betrug 6,9 Gew.-%. Das Gewichtsverhältnis von Restfeuchte zu Gewichtsverlust bei 450 °C (3 h bei 450°C im Porzellantiegel (der bei 900 °C bis zur Gewichtskonstanz geglüht worden war) unter Luft erhitzen) betrug 23 %. G$^w$ betrug 30 Gew.-%.

Verfahrensstufe D)

Herstellung der ringförmigen Formkörper V

Anschließend wurde das zur Tablettiermaschine geförderte Pulver P* in derselben Tablettiermaschine (ein Kilian Rundläufer vom Typ RX 73, der Fa. Kilian, D-50735 Köln) wie in Beispiel III. der deutschen Anmeldung Nr. 102008040093.9 unter Luftatmosphäre zu ringförmigen Formkörpern V der Geometrie 5 mm x 3 mm x 2 mm (Außendurchmesser x Höhe x Innendurchmesser) mit einer Seitendruckfestigkeit von 19 N tablettiert. Der maximale Pressdruck P2 betrug 21,6 kN/cm$^2$. Der Rundläufertisch ist mit auswechselbaren Matrizen bestückt. Um Staubfreisetzung zu vermeiden wurde die Tablettiermaschiene abgesaugt (300 bis 400 Nm$^3$/h). Die Abluft wurde über einen Filter vom Typ HSL 900-8/8 SZ der Firma Herding in D-92224 Amberg geführt, der unter Gewinnung des aus Feststoffpartikeln FP bestehenden Filterkuchens periodisch abgereinigt wurde.

Verfahrensstufe E)

Thermische Behandlung der ringförmigen Formkörper V unter Erhalt der ringförmigen Vergleichskatalysatorformkörper VK2-1

Zur abschließenden thermischen Behandlung wurden jeweils 1000 g einer repräsentativen Mischprobe der wie beschrieben hergestellten Formkörper V gleichmäßig aufgeteilt auf nebeneinander angeordnete 4 Gitternetze mit einer quadratischen Grundfläche von jeweils 150 mm x 150 mm (Schütthöhe: ca. 15 mm) in einem mit 1200 Nl/h Luft durchströmten Umluft-Schachtofen (Firma Nabertherm GmbH, Bahnhofstraße 20, D-28865 Lilienthal/Bremen, Typ S60/65A mit Siemens SPS7 und Bedienpanel H 1700 sowie Innentemperaturregelung) zunächst in 72 min von Raumtemperatur (25 °C) auf 130 °C aufgeheizt. Diese Temperatur wurde für 72 min aufrechterhalten und dann in 36 min auf 190 °C erhöht. Nach einer Haltezeit von 72 min wurde in 36 min auf 220 °C aufgeheizt. Die 220 °C wurden für 72 min gehalten, bevor die Temperatur innerhalb von 36 min auf 265 °C erhöht wurde. Diese Temperatur wurde ebenfalls 72 min aufrechterhalten, bevor sie innerhalb von 93 min auf 380 °C erhöht wurde. Nach einer Haltezeit von 187 min wurde in 93 min auf 430 °C aufgeheizt und die Temperatur dann nach einer weiteren Haltezeit von 187 min in 93 min auf 500 °C erhöht. Diese Endtemperatur wurde für 467 min gehalten. Danach wurde auf Raumtemperatur abgekühlt und die ringförmigen Vergleichskatalysatorformkörper VK2-1 mit einer Seitendruckfestigkeit von 6,3 N erhalten.

Bei einer anschließenden Unterkornsiebung mit einem Langlochsieb aus Edelstahl 1.4541 (gerade Kantenlänge: 20 mm, Kantenabstand: 1,8 mm) fiel, bezogen auf das Gewicht des insgesamt zur Siebung aufgegebenen Siebgutes, ein Unterkornanteil von 3,4 Gew.-% an.

Anstatt die thermische Behandlung wie vorstehend beschrieben durchzuführen, kann man sie auch wie in Beispiel 1 der DE-A 100 46 957 (die Schütthöhe in der Zersetzung (Kammern 1 bis 4) beläuft sich dabei jedoch vorteilhaft auf 32 mm bei einer Verweilzeit pro Kammer von 1,8 h und in der Kalzination (Kammern 5 bis 8) beläuft sie sich vorteilhaft auf 77 mm bei einer Verweilzeit von 4,7 h) beschrieben mittels einer Bandkalziniervorrichtung durchführen. Die Kammern besitzen eine Grundfläche (bei einer einheitlichen Kammerlänge von 1,40 m) von 1,29 $m^2$ (Zersetzung) und 1,40 $m^2$ (Kalzination) und werden von unten durch das grobmaschige Förderband von 50-150 $Nm^3$/h auf 100 °C (Zersetzung) bzw. 470 °C (Kalzination) vorgeheizte Zuluft durchströmt. Zusätzlich wird die Luft durch rotierende Ventilatoren (900 bis 1400 U/min) umgewälzt. Innerhalb der Kammern ist die zeitliche und örtliche Abweichung der Temperatur vom Sollwert (typische Werte für die Zonen 1-8 sind: 150 °C, 190 °C, 220 °C, 265 °C, 380 °C, 430 °C, 500 °C, 500 °C) stets ≤ 2°C. Hinter Kammer 8 schließt sich vorteilhafterweise eine auf 90 °C temperierte 2 m lange Kühlzone an. Im Übrigen wird wie in Beispiel 1 der DE-A 100 46 957 beschrieben verfahren. Die resultierenden Vergleichskatalysatorformkörper VK2-1 eignen sich als Katalysatoren für die Partialoxidation von Propylen zu Acrolein. Sie können zur Abtrennung von Agglomeraten noch über ein 5 mm x 20 mm Langlochschlitzsieb und zur Abtrennung von kalziniertem Bruch über ein bevorzugt zwei Langlochschlitzsiebe (1,8 mm x 20 mm Schlitz) geführt werden.

In einer alternativen Ausführungsform wurden alle Verfahrensstufen identisch wiederholt. Nach der Verfahrensstufe D) wurden die in selbiger erzeugten ringförmigen Formkörper V mittels einer Schwingsiebmaschine E.A. Typ 36-2 von der Engelsmann AG in D-67059 Ludwigshafen am Rhein, über ein Edelstahl 1.4541 Schlitzsieb (rechteckige Schlitze der Länge 30 mm und der Breite 1,8 mm) geführt und als Verfahrensstufe F in intakte Formkörper $V^+$ (ca. 97 Gew.-%) als Überkorn und nicht intakte Formkörper $V^-$ (ca. 3 Gew.-%) aufgetrennt. Die wie bereits beschrieben durchgeführte thermische Behandlung der ringförmigen Formkörper $V^+$ im Umluftofen lieferte wieder die Vergleichskatalysatorformkörper VK2-1. Bei einer anschließenden Unterkornsiebung derselben mit einem Langlochsieb aus Edelstahl 1.4541 (gerade Kantenlänge: 20 mm, Kantenabstand: 1,8 mm) fiel, bezogen auf das Gewicht des insgesamt zur Siebung aufgegebenen Siebgutes, nur noch ein Unterkornanteil von 0,4 Gew.-% an.

2. Ringförmige Katalysatorformkörper K2-1 (in den Verfahrensstufen B), C), D), F) und G) herrschte Luftatmosphäre (26° und relative Luftfeuchtigkeit von 60 %)

Das Herstellverfahren entsprach jenem zur Herstellung der Vergleichskatalysatorformkörper VK2-1, jedoch mit folgenden Unterschieden:

a) Die in der Verfahrensstufe F) abgetrennten Formkörper V- wurden als Verfahrensstufe G) mit einer Hammermühle der Fa. Hosokawa Alpine AG, D-86199 Augsburg aufgemahlen (Partikeldurchmesser 1 μm < $d^H$ < 100 μm; Siebanalyse). Das resultierende feinteilige Haufwerk H wurde in einem geschlossenen Sammelbehälter zwischengelagert und von dort saugpneumatisch vor die Verfahrensstufe B) rückgeführt und in das Gemisch M* eingearbeitet (mit einem Gewichtsanteil von 20 Gew.-% bezogen auf das Gesamtgewicht).

b) Der in der Verfahrensstufe D) gewonnene Filterkuchen aus Feststoffpartikeln FP wurde in einem geschlossenen Sammelbehälter gesammelt und von dort saugpneumatisch vor die Verfahrensstufe B) rückgeführt und ebenfalls in das Gemisch M* eingearbeitet (mit einem Gewichtsanteil von 2 Gew.-% bezogen auf das Gesamtgewicht) und das Gemisch M* zu Schülpen pressagglomeriert.

Im mit der Tablettiermaschine tablettierten Gemisch lag der Gewichtsanteil mit $d^P$ ≥ 160 μm bei 77,5 Gew.-% und die Restfeuchte (der Wassergehalt) betrug 7,9 Gew.-%. Das Gewichtsverhältnis von Restfeuchte zu Gewichtsverlust bei 450 °C (3 h bei 450°C im Porzellantiegel (der bei 900 °C bis zur Gewichtskonstanz geglüht worden war) unter Luft erhitzen) betrug 26 %. $G^W$ betrug 35 Gew.-%. Die Seitendruckfestigkeit der resultierenden Formkörper V betrug 20 N. Die resultierenden Katalysatorformkörper K2-1 wiesen eine Seitendruckfestigkeit von 6,1 N auf.

3. Ringförmige Katalysatorformkörper K2-2 (in den Verfahrensstufen B), C), D), F) und G) herrschte Luftatmosphäre mit erhöhter Luftfeuchtigkeit (32° und relative Luftfeuchtigkeit von 91 %)

Das Herstellverfahren entsprach jenem zur Herstellung der Katalysatorformkörper K2-1, jedoch mit dem Unterschied einer erhöhten Luftfeuchtigkeit in den Verfahrensstufen B), C), D), F) und G). Im mit der Tablettiermaschine tablettierten Gemisch lag der Gewichtsanteil mit $d^P \geq 160$ μm bei 95,7 Gew.-% (Bestimmung durch Siebanalyse), bei einer Restfeuchte (einem Wassergehalt) von 11,5 Gew.-%. Das Gewichtsverhältnis von Restfeuchte zu Gewichtsverlust bei 450 °C (3 h bei 450°C im Porzellantiegel (der bei 900°C bis zur Gewichtskonstanz geglüht worden war) unter Luft erhitzen) betrug 35 %. $G^w$ betrug 54 Gew.-%. Die Seitendruckfestigkeit der resultierenden Formkörper V betrug 22 N. Die resultierenden Katalysatorformkörper K2-2 wiesen eine Seitendruckfestigkeit von 5,9 N auf.

4. Ringförmige Katalysatorformkörper K2-3 (in den Verfahrensstufen B), C), D), F) und G) herrschte Luftatmosphäre mit erhöhter Luftfeuchtigkeit (32° und relative Luftfeuchtigkeit von 91 %)

Das Herstellverfahren entsprach jenem zur Herstellung der Katalysatorformkörper K2-2, jedoch mit dem Unterschied, dass das feinteilige Haufwerk H vor seiner Rückführung vor die Verfahrensstufe B) 24 Stunden in einem offenen Sammelbehälter zwischengelagert worden war. Im mit der Tablettiermaschine tablettierten Gemisch lag der Gewichtsanteil mit $d^P \geq 160$ μm bei 98,8 Gew.-% (Bestimmung durch Siebanalyse; geringerer Zerfall bzw. Reagglomeration durch erhöhte Restfeuchte), bei einer Restfeuchte (einem Wassergehalt) von 14,1 Gew.-%. Das Gewichtsverhältnis von Restfeuchte zu Gewichtsverlust bei 450 °C (3 h bei 450°C im Porzellantiegel (der bei 900°C bis zur Gewichtskonstanz geglüht worden war) unter Luft erhitzen) betrug 41 %. $G^W$ betrug 69 Gew.-%. Die Seitendruckfestigkeit der resultierenden Formkörper V betrug 24 N. Die resultierenden Katalysatorformkörper K2-3 wiesen eine Seitendruckfestigkeit von 5,5 N auf.

5. Ringförmige Katalysatorformkörper K2-4 (in den Verfahrensstufen B), C), D), F) und G) herrschte Luftatmosphäre mit erhöhter Luftfeuchtigkeit (32° und relative Luftfeuchtigkeit von 92 %) und der Anteil des rückgeführten Feinkorns F ist erhöht

Das Herstellverfahren entsprach jenem zur Herstellung der Katalysatorformkörper K2-2, jedoch mit dem Unterschied, dass in der Verfahrensstufe C) die Siebweite des Unterkornschwingsiebs 400 μm betrug. Dadurch erhöhte sich der Gewichtsanteil des Feinkorns F auf ca. 70 Gew.-%. Der Gewichtsanteil mit $d^P \geq 160$ μm lag bei 98,9 Gew.-% (Bestimmung durch Siebanalyse; geringerer Zerfall bzw. Reagglomeration durch erhöhte Restfeuchte), bei einer Restfeuchte (einem Wassergehalt) von 13,9 Gew.-%. Das Gewichtsverhältnis von Restfeuchte zu Gewichtsverlust bei 450 °C (3 h bei 450 °C im Porzellantiegel (der bei 900°C bis zur Gewichtskonstanz geglüht worden war) unter Luft erhitzen) betrug 41 %. $G^W$ betrug 69 Gew.-%. Die Seitendruckfestigkeit der resultierenden Formkörper V betrug 23 N. Die resultierenden Katalysatorformkörper K2-4 wiesen eine Seitendruckfestigkeit von 5,7 N auf.

IV. Testung der in III. hergestellten ringförmigen Katalysatoren in einer heterogen katalysierten partiellen Gasphasenoxidation von Propen zu Acrolein

Die katalytische Testung erfolgte wie für die ringförmigen Katalysatoren unter II beschrieben. Die nachfolgende Tabelle 3 zeigt in entsprechender Weise wie die Tabelle 2 die erzielten Ergebnisse.

Tabelle 3

| Katalysatoren | $T^S$ (°C) | $U^P$ (mol-%) | $S^{AC}$ (mol-%) | $S^{AC + AA}$ (mol-%) |
|---|---|---|---|---|
| VK2-1 | 319 | 95,0 | 91,0 | 95,7 |
| K2-1 | 318 | 95,0 | 90,8 | 95,5 |
| K2-2 | 319 | 95,1 | 90,6 | 95,1 |
| K2-3 | 321 | 95,1 | 89,5 | 93,8 |
| K2-4 | 319 | 95,0 | 90,0 | 94,4 |

In überraschender Weise führt die erfindungsgemäße Rückführung bei der Herstellung der ringförmigen Katalysatoren insbesondere bei $S^{AC + AA}$ nur zu marginalen Verlusten, die sich bei einer geringen Restfeuchte (geringem Wassergehalt) des Rückführgutes und damit einer geringen Restfeuchte sowie einem geringen $G^w$ der Formkörper V am Rande der Messgenauigkeit bewegen.

V. Herstellung von ringförmigen Katalysatorformkörpern K und ringförmigen Vergleichskatalysatorformkörpern VK, deren Multielementoxidaktivmasse jeweils nachfolgende Stöchiometrie aufweist:

$$Mo_{12}P_{1,5}V_{0,6}CS_{1,0}Cu_{0,5}Sb_1S_{0,04}O_x.$$

1. Ringförmige Vergleichskatalysatorformkörper VK3-1 (in den Verfahrensstufen B), C), D) und F) herrschte Luftatmosphäre (27°C und relative Luftfeuchtigkeit von 71 %)

Verfahrensstufe A)

Herstellung der feinteiligen Mischung M aus verschiedenen Quellen Q

In einem wassertemperierten 1,75-m³-Doppelmantelbehälter (der Zwischenraum wurde von Temperierwasser durchströmt) aus Edelstahl (D = 1,3 m, h = 1,9 m) mit einem stufenlos regelbaren Balkenrührer (D = 0,8 m, h = 1,68 m) wurden 619 l auf 45 °C temperiertes Wasser vorgelegt und während der nachfolgenden Schritte mit 70 Umdrehungen pro Minute (UPM) gerührt. Innerhalb von ca. 40 Minuten wurden hierzu 537,5 kg eine Temperatur von 25°C aufweisendes Ammoniumheptamolybdattetrahydrat $((NH_4)_6Mo_7O_{24} \cdot 4\ H_2O$ (81 Gew.-% $MoO_3$, 8 Gew.-% $NH_3$, $\leq$ 50 Gew.-ppm Na und $\leq$ 100 Gew.-ppm K) dosiert. Die Temperatur der Lösung sank dabei auf 37 °C ab. Um ein sicheres Auflösen des Ammoniumheptamolybdats zu gewährleisten, wurde nach Ende der Zudosierung noch 15 Minuten nachgerührt, wobei die Temperatur von 37 °C beibehalten wurde. Bei derselben Temperatur wurden nun über eine Differenzialdosierwaage innerhalb von 3 Minuten (jeweils 25 °C aufweisend) 17,82 kg Ammoniummetavanadat ($NH_4VO_3$, 77 Gew.-% $V_2O_5$, 14,5 Gew.-% $NH_3$, $\leq$ 150 Gew.-ppm Na und $\leq$ 500 Gew.-ppm K) zudosiert. Es wurde 2 Minuten nachgerührt. Dann wurde innerhalb einer Minute eine in einem separaten Lösebehälter (gerührter 0,20-m³-Doppelmantelbehälter aus Edelstahl (D = 0,7 m, h = 0,78 m)) hergestellte, farblose, klare, 60 °C warme Lösung von 49,6 kg Cäsiumnitrat ($CsNO_3$ mit 72 Gew.-% $Cs_2O$ und $\leq$ 50 Gew.-ppm Na, $\leq$ 100 Gew.-ppm K, $\leq$ 10 Gew.-ppm Al sowie $\leq$ 20 Gew.-ppm Fe, Lösedauer ca. 30 min, während der anschließenden Dosierung wurde der Vorlagebehälter mit $CsNO_3$-Lösung nicht gerührt) in 106 l Wasser über einen Rohrstutzen (D = 25 mm) zugegeben. Hierbei stieg die Temperatur der resultierenden Suspension auf 39 °C. Nach einminütigem Nachrühren wurde innerhalb einer weiteren Minute aus einem 310-l-Vorratsbehälter aus 1.4571 Stahl über einen Rohrstutzen (D = 25 mm) 31,66 l 75 gew.-%ige Phosphorsäure (Dichte bei 25 °C und 1 atm: 1,57 g/ml, Viskosität bei 25 °C und 1 atm: 0,147 $cm^2$/S) zudosiert. Aufgrund der exothermen Reaktion stieg die Temperatur hierbei auf 42°C. Erneut wurde 1 Minute nachgerührt. Dann wurden innerhalb einer Minute 1,34 kg Ammoniumsulfat ($(NH_4)_2SO_4$ (> 99 Gew.-%)) eingerührt und 1 weitere Minute nachgerührt. Währenddessen wurde das Warmwasser aus dem Doppelmantel des Ansatzkessels ausgeblasen. Bei identischer Temperatur wurden über eine Dosierwaage innerhalb von 3 Minuten 37,04 kg Antimontrioxid ($Sb_2O_3$, Partikeldurchmesser $d_{50}$ = ca. 2 $\mu$m, Kristallstruktur laut XRD: > 75 % Senarmontit, < 25 % Valentinit, Reinheit: > 99,3 Gew.-%, $\leq$ 0,3 Gew.-% $As_2O_3$, $\leq$ 0,3 Gew.-% PbO und $\leq$ 300 Gew.-ppm FeO) zugegeben (käuflich erhältlich als Triox White, Code No. 639000 der Fa. Antraco, D-10407 Berlin). Nun wurde die Rührerdrehzahl von 70 auf 50 UPM zurückgenommen. Anschließend wurde die gerührte Suspension mittels Dampf im Doppelmantel innerhalb von 30 Minuten linear auf 95 °C aufgeheizt. Bei dieser Temperatur und 50 UPM wurden aus einem Vorlagebehälter aus Edelstahl innerhalb von 4 Minuten 51,64 kg Kupfernitratlösung (wässrige $Cu(NO_3)_2$-Lösung mit 15,6 Gew.-% Cu) zugegeben. Nach 56-minütigem Nachrühren bei 95 °C wurde die Rührgeschwindigkeit weiter von 50 auf 35 UPM zurückgenommen. Anschließend wurde die gesamte Suspension innerhalb von 4 Minuten in einen mit 10 Nm³/h Stickstoff überlagerten, auf 85 °C temperierten und mit 35 UPM gerührten Sprühturmvorlagenbehälter (1,75-m³-Doppelmantelbehälter aus Edelstahl (D = 1,3 m, h = 1,9 m) mit einem stufenlos regelbaren Balkenrührer (D = 0,8 m, h = 1,68 m)) abgelassen und es wurde mit 20 l Wasser (25 °C) nachgespült (später wurde mit 5 bis 25 gew.-%iger wässriger $NH_3$-Lösung gereinigt). Aus diesem heraus wurde die Suspension in einem Drehscheibensprühturm vom Typ FS-15 der Firma Niro A/S, DK-2860 Soeborg im Heißluftgleichstrom mit einer Eintrittstemperatur von 300 °C, einer Austrittstemperatur von 110 °C, einer Scheibendrehzahl von 18000 U/min, einem Durchsatz von 270 kg/h, einer Luftmenge von 1800 Nm³/h und einer Verweilzeit von 2,2 Minuten innerhalb von 3,5 h sprühgetrocknet, wobei das resultierende Sprühpulver einen Glühverlust (1 h bei 500 °C in Luft) von 17,2 Gew.-% und einen $d_{50}$ von 35,9 $\mu$m aufwies ($d_{10}$ = 14,3 $\mu$m, $d_{90}$ = 65,6 $\mu$m, gemessen bei einem Dispergierdruck von 2 bar absolut).

Das Sprühpulver wurde in einem Schräglagen-Mischer (Typ VIL, Füllvolumen: 200 l, Aachener Misch- und Knetmaschinenfabrik) mit Misch- und Schneidflügel (Drehzahl Mischflügel: 39 U/min, Drehzahl Schneidflügel: 3000 U/min) innerhalb von 9 Minuten mit 1,5 Gew.-% Graphit TIMREX T44 ($d_{50}$ = 20,8 $\mu$m) der Firma Timcal AG unter Erhalt der feinteiligen Mischung M vermischt. Die feinteilige Mischung M enthielt keine Partikel mit einem Partikeldurchmesser $d^M \geq$ 160 $\mu$m.

Verfahrensstufe B)

Herstellung der Agglomerate A

Die feinteilige Mischung M wurde dann in einer aus Edelstahl 1.4541 gefertigten Zweiwalzenpresse vom Typ Zweiwalzenkompaktor Typ K200/100 der Fa. Hosokawa Bepex GmbH, D-74211 Leingarten) mit konkaven (Tiefe = 2 mm), geriffelten Glattwalzen (Spaltweite: 2,8 mm, Walzendurchmesser: 20 cm, Walzendrehzahl: 13 UPM, Presskraftsollwert: ca. 30 kN, maximaler Pressdruck P1: 1,5 kN/cm$^2$) zu ca. 10 cm breiten und ca. 2,8 mm hohen Schülpen (Agglomeraten A) pressägglomeriert.

Verfahrensstufe C)

Herstellung der Pulver P/P*

Die Schülpen wurden über einen aus Edelstahl 1.4541 gefertigten Stiftwalzenbrecher vom Typ GBM-406 und eine nachgeschaltete, aus Edelstahl 1.4541 gefertigte Schlagsiebmaschine vom Typ MGR-803 (beide Firma Frewitt Maschinenfabrik AG, CH-1700 Fribourg) mit einem Rotor und einem Frewittsieb mit einer Maschenweite (quadratische Maschen aus Viereckdräht) von 1 mm zerkleinert. Über integrierte Schwingsiebe der Fa. Allgaier (Siebweite Überkorn (nur relevant bei defektem Frewittsieb): 1,25 mm, Siebweite Unterkorn: 400 $\mu$m) mit Gummiball-Klopfung (Gummiball-durchmesser = 22 mm) wurde ein Pulver P isoliert, dessen Partikeldurchmesser $d^P$ 400 $\mu$m $\leq d^P \leq$ 1 mm betrugen. Die Mengenverteilung zwischen dem Überkorn, dem gewünschten Pulver P und dem Feinkorn F (Unterkorn) betrug <1 Gew.-%: ca. 55 Gew.-%: ca. 45 Gew.-%. Das Feinkorn F wurde mittels saugpneumatischer Förderung vor den Zweiwalzenkompaktor zurückgeführt und im Gemisch M* mit neu zugeführter feinteiliger Mischung M erneut zu Schülpen pressagglomeriert. Der Presskraftsollwert in der Verfahrensstufe B) wurde so nachgeregelt, dass sich ein Rüttelgewicht des Pulvers P zwischen 1350 und 1410 g/l einstellte.

Zur Herstellung der ringförmigen Formkörper V in der Verfahrensstufe D) wurden dem Pulver P in einem Bandschnecken-Mischer vom Typ S5 der Firma Draiswerke GmbH in D-68305 Mannheim innerhalb von 2 Min weitere 1 Gew.-% des Graphits TIMREX T44 der Firma Timcal AG zugemischt und das dabei resultierende Gemisch P* mittels saugpneumatischer Förderung zur Tablettiermaschine transportiert. Im mit der Tablettiermaschine tablettierten Gemisch lag der Gewichtsanteil mit $d^P \geq$ 160 $\mu$m bei 84 Gew.-% (Bestimmung durch Siebanalyse) und die Restfeuchte betrug 3,9 Gew.-%. Das Gewichtsverhältnis von Restfeuchte zu Gewichtsverlust bei 450°C (3 h bei 450°C im Porzellantiegel (der bei 900 °C bis zur Gewichtskonstanz geglüht worden war) unter Luft erhitzen) betrug 24 %. $G^W$ betrug 32 Gew.-%.

Verfahrensstufe D)

Herstellung der ringförmigen Formkörper V

Anschließend wurde das zur Tablettiermaschine geförderte Pulver P* in derselben Tablettiermaschine (ein Korsch-Rundläufer vom Typ PH 865 der Fa. Korsch) wie im Beispiel IV. der deutschen Anmeldung Nr. 102008040093.9 beschrieben mit einer Drehzahl des Tablettiertellers von ca. 20 UPM unter Luftatmosphäre zu ringförmigen Formkörpern V der Geometrie 7 mm x 7 mm x 3 mm (Außendurchmesser x Höhe x Innendurchmesser) mit einer Seitendruckfestigkeit von 37 $\pm$ 2 N tablettiert. Die Hauptresskraft betrug ca. 3-5 kN. Der maximale Pressdruck P2 betrug 16 kN/cm$^2$: Um Staubfreisetzung zu vermeiden wurde die Tablettiermaschiene abgesaugt (300 bis 400 Nm$^3$/h). Die Abluft wurde über einen Filter vom Typ HSL 900-8/8 SZ der Firma Herding in D-92224 Amberg geführt, der unter Gewinnung des aus Feststoffpartikeln FP bestehenden Filterkuchens periodisch abgereinigt wurde.

Verfahrensstufe E)

Thermische Behandlung der ringförmigen Formkörper V unter Erhalt der ringförmigen Vergleichskatalysatorformkörper VK3-1

Die thermische Behandlung erfolgte in zwei hintereinander geschalteten und miteinander direkt gekoppelten (der "output" des ersten bildete den "input" des zweiten Bandkalzinierers) Bandkalzinierern wie sie die DE-A 100 46 957 näher beschreibt (die verschiedenen Zonen eines Bandkalzinieres kommunizieren gasseitig), die jeweils 4 Heizzonen (Kammern) enthielten. Die Kammern besaßen eine Grundfläche (bei einer einheitlichen Kammerlänge von 1,40 m) von 1,29 m$^2$ (Zersetzung; 1. Bankalzinierer) und 1,40 m$^2$ (Kalzination; 2. Bandkalzinierer) und wurden von unten durch das grobmaschige Förderband von auf 150 °C (Zersetzung) bzw. 320 °C (Kalzination) vorerhitzter Zuluft durchströmt. Diese Luft wurde in den Kammern zusätzlich durch rotierende Ventilatoren umgewälzt. Die charakteristischen Kenngrößen für den Betrieb der beiden Bandkalzinierer sind in Tabelle 5 zusammengefasst. Im 1. Bandkalzinierer (Bandbreite = 92 cm) erfolgte die Zersetzung der in den geometrischen Formkörpern V enthaltenen Ammoniumsalze ("Salzzersetzung"). Die Konzentration des hierbei freigesetzten $NH_3$ wurde in allen 4 Heizzonen des 1. Bandkalzinierers kontinuierlich durch FTIR-Spektroskopie überwacht (Spektrometer der Fa. Nicolet, Typ "Impact", IR-Edelstahlzelle mit $CaF_2$-Fenster, 10 cm Schichtdicke, Temperierung auf 120 °C, Bestimmung der Konzentration anhand der Intensität der Bande bei 3.333 $cm^{-1}$). Die Messwerte sind in Tabelle 5 ebenfalls angegeben. Die Temperaturen in den Zonen 2 bis 4 des 1. Bandkalzinierers beeinflussen (zusammen mit den Temperaturen im 2. Bandkalzinierer) den Restammoniumgehalt der resultierenden ringförmigen Vergleichskatalysatorformkörper VK3-1 (sie beeinflussen jedoch nicht deren Anteil an orthorhombischem $MoO_3$). Bei einer Bandgeschwindigkeit von 2,3 cm/min betrug die Schütthöhe im 1. Bandkalzinierer 50 mm. Im 2. Bandkalzinierer erfolgte die Endkalzination der geometrischen Formkörper V. Analog zu den 4 Heizzonen des 1. Bandkalzinierers wurden im 2. Bandkalzinierer die ersten beiden Zonen bezüglich des freiwerdenden Ammoniaks überwacht. Die Temperaturen in den Zonen 6 bis 8 dienten primär als Steuergröße für den Anteil an orthorhombischem $MoO_3$ in den resultierenden ringförmigen Vergleichskatalysatorformkörpern VK3-1, beeinflussten aber auch deren Restammoniumgehalt. Im 2. Bandkalzinierer (Bandbreite = 100 cm) betrug die Bandgeschwindigkeit 1,0 cm/min, so dass eine Schütthöhe von 105 mm resultierte. Die so erhaltenen ringförmigen Vergleichskatalysatorformkörper VK3-1 wurden zur Abtrennung von Agglomeraten noch über ein 9 mm (Kantenabstand) x 20 mm (Kantenlänge) Langlochschlitzsieb und zum Zweck der Unterkornsiebung über zwei Langlochschlitzsiebe (jeweils 6 mm (Kantenabstand) x 20 mm (Kantenlänge)) geführt. Der anfallende Unterkornanteil betrug, bezogen auf die Gesamtmenge des aufgegebenen Siebgutes, 23 Gew.-%.

Die ringförmigen Vergleichskatalysatorformkörper- VK3-1 wiesen eine Seitendruckfestigkeit von 15 N, einen Ammonium-Gehalt (bestimmt durch Titration nach Kjeldahl) von 0,52 Gew.-% $NH_4^+$ und einen $MoO_3$-Gehait von 2,1 Intensitäts-% auf. Letzterer berechnet sich als Verhältnis der Intensität (die Definition der Intensität eines Beugungsreflexes im Röntgendiffraktogramm bezieht sich in dieser Schrift stets auf die in der DE-A 198 35 247, sowie die in der DE-A 100 51 419 und in der DE-A 100 46 672 niedergelegte Definition) des $(021)$-$MoO_3$-Reflexes bei 20 = 27,3° zur Intensität des $(222)$-Reflexes der Heteropolyverbindung bei $2\Theta = 26,5°$ im Röntgenpulverdiffraktogramm (mit Cu-$K\alpha$-Strahlung).

In einer alternativen Ausführungsform wurden alle Verfahrensstufen identisch wiederholt. Nach der Verfahrensstufe D) wurden die in selbiger erzeugten ringförmigen Formkörper V mittels einer Schwingsiebmaschine E.A. Typ 36-2 von der Engelsmann AG in D-67059 Ludwigshafen am Rhein, über ein Edelstahl 1.4541 Schlitzsieb (rechteckige Schlitze der Länge 20 mm und der Breite 5 mm) geführt und als Verfahrensstufe F in intakte Formkörper $V^+$ (ca. 82 Gew.-%) als Überkorn und nicht intakte Formkörper V-(ca. 18 Gew.-%) aufgetrennt. Wie bereits beschrieben durchgeführte thermische Behandlung der ringförmigen Formkörper $V^+$ in den zwei hintereinander geschalteten Bandkalzinierern lieferte wieder die ringförmigen Vergleichskatalysatorformkörper VK3-1. Bei einer anschließenden Unterkornsiebung derselben mit zwei Langlochsieben aus Edelstahl 1.4541 (gerade Kantenlänge: 20 mm, Kantenabstand: 6 mm) fiel, bezogen auf das Gewicht des insgesamt zur Siebung aufgegebenen Siebgutes, nur noch ein Unterkornanteil von 5,2 Gew.-% an.

2. Ringförmige Katalysatorformkörper K3-1 (in den Verfahrensstufen B), C), D), F) und G) herrschte Luftatmosphäre (27° und relative Luftfeuchtigkeit von 71 %)

Das Herstellverfahren entsprach jenem zur Herstellung der Vergleichskatalysatorformkörper VK3-1, jedoch mit folgenden Unterschieden:

a) Die in der Verfahrensstufe F) abgetrennten Formkörper V- wurden als Verfahrensstufe G) mit einer Hammermühle der Fa. Hosokawa Alpine AG, D-86199 Augsburg aufgemahlen (Partikeldurchmesser 1 $\mu m < d^H < 100$ $\mu m$; Siebanalyse). Das resultierende feinteilige Haufwerk H wurde in einem geschlossenen Sammelbehälter zwischengelagert und von dort saugpneumatisch vor die Verfahrensstufe B) rückgeführt und in das Gemisch M* eingearbeitet (mit einem Gewichtsanteil von 20 Gew.-% bezogen auf das Gesamtgewicht).

b) Der in der Verfahrensstufe D) gewonnene Filterkuchen aus Feststoffpartikeln FP wurde in einem geschlossenen Sammelbehälter gesammelt und von dort saugpneumatisch vor die Verfahrensstufe B) rückgeführt und ebenfalls in das Gemisch M* eingearbeitet (mit einem Gewichtsanteil von 2 Gew.-% bezogen auf das Gesamtgewicht) und das Gemisch M* zu Schülpen pressagglomeriert.

Im mit der Tablettiermaschine tablettierten Gemisch lag der Gewichtsanteil mit $d^P \geq 160$ $\mu m$ bei 80 Gew.-% und die Restfeuchte (der Wassergehalt) betrug 4,1 Gew.-%. Das Gewichtsverhältnis von Restfeuchte zu Gewichtsverlust bei 450 °C (3 h bei 450°C im Porzellantiegel (der bei 900°C bis zur Gewichtskonstanz geglüht worden war) unter Luft erhitzen) betrug 26 %. $G^W$ betrug 35 Gew.-%. Die Seitendruckfestigkeit der resultierenden Formkörper V betrug.37 $\pm$

2 N. Die resultierenden ringförmigen Katalysatorformkörper K3-1 wiesen eine Seitendruckfestigkeit von 15 N, einen Ammonium-Gehalt (bestimmt durch Titration nach Kjeldahl) von 0,53 Gew.-% $NH_4^+$ und einen $MoO_3$-Gehalt von 2,1 Intensitäts-% auf.

3. Ringförmige Katalysatorformkörper 3-2 (in den Verfahrensstufen B), C), D), F) und G) herrschte Luftatmosphäre (27°C° und relative Luftfeuchtigkeit von 71 %)

Das Herstellverfahren entsprach jenem zur Herstellung der Katalysatorformkörper K3-1, jedoch mit dem Unterschied, dass das feinteilige Haufwerk H vor seiner Rückführung vor die Verfahrensstufe B) 24 Stunden in einem offenen Sammelbehälter zwischengelagert worden war. Im mit der Tablettiermaschine tablettierten Gemisch lag der Gewichtsanteil mit $d^P \geq 160 \, \mu$m bei 83 Gew.-% (Bestimmung durch Siebanalyse; geringerer Zerfall bzw. Reagglomeration durch erhöhte Restfeuchte), bei einer Restfeuchte (einem Wassergehalt) von 6,9 Gew.-%. Das Gewichtsverhältnis von Restfeuchte zu Gewichtsverlust bei 450 °C (3 h bei 450°C im Porzellantiegel (der bei 900 °C bis zur Gewichtskonstanz geglüht worden war) unter Luft erhitzen) betrug 41 %. $G^W$ betrug 69 Gew.-%. Die Seitendruckfestigkeit der resultierenden Formkörper V betrug 37 $\pm$ 2 N. Die resultierenden Katalysatorformkörper K3-2 wiesen eine Seitendruckfestigkeit von 15 N, einen Ammonium-Gehalt (bestimmt durch Titration nach Kjeldahl) von 0,53 Gew.-% $NH_4^+$ und einen $MoO_3$-Gehalt von 2,2 Intensitäts-% auf.

VI. Testung der in V. hergestellten ringförmigen Katalysatoren in einer heterogen katalysierten partiellen Gasphasenoxidation von Methacrolein zu Methacrylsäure

2 kg der jeweiligen ringförmigen Katalysatorformkörper wurden mit einer Vor- und einer Nachschüttung von jeweils 50 g Steatitringen (Steatit C220 der Fa. CeramTec) der Geometrie 7 mm x 7 mm x 4 mm (Außendurchmesser x Länge x Innendurchmesser) in einen Modellrohrreaktor aus Edelstahl (Außendurchmesser = 30 mm, Innendurchmesser = 26 mm, Länge = 4,15 m) gefüllt (Füllhöhe: 397 cm). Dieses befand sich in einem mit Stickstoff geperlten, auf etwa 287 °C temperierten Salzbad (53 Gew.-% Kaliumnitrat, 40 Gew.-% Natriumnitrit und 7 Gew.-% Natriumnitrat). Die katalytische Testung erfolgte unter Kreisgasfahrweise. Dabei resultierte eine Eduktgaszusammensetzung (eine Zusammensetzung des Reaktionsgasäusgangsgemischs) von ca. 5 Vol.-% Methacrolein, 9 Vol.-% $O_2$, 17 Vol.-% Wasserdampf, 1,5 Vol.-% CO, 1,8 Vot.-% $CO_2$ und als Restgasmenge bis 100 Vol.-% im Wesentlichen molekularer Stickstoff. Die massenbezogene Raumgeschwindigkeit (WHSV) betrug 0,17 h$^{-1}$.

Während der jeweils 5-tägigen Testung wurde der Methacrolein-Umsatz $U^{MAC}$ im einfachen Durchgang bei ca. 64 mol-% gehalten. Hierzu wurde die Salzbadtemperatur schrittweise erhöht. Die am 5. Tag erzielten Ergebnisse zeigt die nachfolgende Tabelle 4. Dabei ist $S^{MAS}$ die Selektivität der Methacrylsäurebildung und $S^{COx}$ die Selektivität der Kohlenoxidnebenproduktbildung.

Tabelle 4

| Katalysator | $T^S$ (°C) | $U^{MAC}$ (mol-%) | $S^{MAS}$ (mol-%) | $S^{Cox}$ (mol-%) |
|---|---|---|---|---|
| VK3-1 | 293 | 64,0 | 84,1 | 9,9 |
| K3-1 | 291 | 64,4 | 83,8 | 10,1 |
| K3-2 | 293 | 64,4 | 83,1 | 10,6 |

In überraschender Weise führt die erfindungsgemäße Rückführung bei der Herstellung der ringförmigen Katalysatoren bei $S^{MAS}$ nur zu marginalen Verlusten, die sich bei einer geringen Restfeuchte (geringem Wassergehalt) des Rückführgutes und damit einer geringen Restfeuchte sowie einem geringeren $G^W$ der Formkörper V am Rande der Messgenauigkeit bewegen.

Tabelle 5:

| 1.Bandkalzinierer | Schütthöhe | | | | | | 5,0 cm | | |
|---|---|---|---|---|---|---|---|---|---|
| | Bandgeschwindigkeit | | | | | | 2,3 cm/min | | |
| | | | | | | | | | |
| 2. Bandkkalzinierer | Schütthöhe | | | | | | 10,5 cm | | |
| | Bandgeschwindigkeit | | | | | | 1,0 cm/min | | |
| | | Zonenlänge [cm] | Verweilzeit [min] | Zonentemperatur [°C] | Zulufttemperatur [°C] | Zuluftstrom [Nm$^3$/h] | Abluftstrom [Nm$^3$/h] | Ventilatoren [UPM] | NH$_3$ [Vol.-%] |
| 1. Bandkkalzinierer | Einlauf | 50 | 25 | offen, nicht temperiert | 150 | 35 | | | |
| | Zone 1 | 145 | 72,5 | 164 | 150 | 100 | 70 | 900 | 1,3-1,9 |
| | Zone 2 | 145 | 72,5 | 228 | 150 | 80 | 195 | 500 | 2,3-2,9 |
| | Zone 3 | 145 | 72,5 | 260 | 150 | 115 | 220 | 500 | 0,6-0,9 |
| | Zone 4 | 145 | 72,5 | 286 | 150 | 115 | 240 | 500 | 0,25-0,4 |
| | Übergabestelle zum 2. Bandkalzinierer | 120 | 60 | nicht temperiert | 150 | 35 | | | |
| 2. Bandkkalzinierer | Einlauf | 125 | 125 | | | 35 | | | |
| | Zone 5 | 145 | 145 | 350 | 320 | 170 | 220 | 500 | 0,14-0,25 |
| | Zone 6 | 145 | 145 | 381 | 320 | 125 | 190 | 500 | 0,05-0,17 |
| | Zone 7 | 145 | 145 | 381 | 320 | 125 | 190 | 500 | |
| | Zone 8 | 145 | 145 | 382 | 320 | 160 | 210 | 500 | |
| | Kühlzone | 200 | 200 | 70 | 320 | 35 | | eingeschaltet | |
| | Auslauf | 50 | 50 | | | | | | |
| | | | | | | | | | |
| | Gesamtverweilzeit | | 1330 | | | | | | |

US-Provisional Application No. 61/122129, eingereicht am 12. Dezember 2008, ist in die vorliegende Anmeldung durch Literaturhinweis eingefügt.

Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

**Patentansprüche**

1. Verfahren zur kontinuierlichen Herstellung von geometrischen Katalysatorformkörpern K, die als Aktivmasse ein Multielementoxid enthalten, das als von Sauerstoff verschiedene Elemente E das Element Mo, wenigstens eines der beiden Elemente Bi und V sowie wenigstens ein weiteres Element aus der Gruppe bestehend aus Co, Ni, Fe, Cu und den Alkalimetallen enthält, in Verfahrensstufen A) bis G), bei dem man

- in der Verfahrensstufe A) mit Hilfe von Quellen Q der Elemente E eine feinteilige Mischung M mit der Maßgabe erzeugt, dass höchstens 10 Gew.-% des Gesamtgewichts der in der feinteiligen Mischung M enthaltenen Partikel einen Partikeldurchmesser $d^M \geq 160\ \mu m$ aufweisen und der Partikeldurchmesser $d_{50}^M$ der Partikel der feinteiligen Mischung M die Bedingung $1\ \mu m \leq d_{50}^M \leq 150\ \mu m$ erfüllt;

- in der Verfahrensstufe B) das feinteilige Gemisch M*, das entweder nur aus der feinteiligen Mischung M oder aus einer Mischung aus der feinteiligen Mischung M und in der nachfolgenden Verfahrensstufe C) anfallendem und aus der Verfahrensstufe C) absatzweise oder kontinuierlich in die Verfahrensstufe B) rückgeführtem Feinkorn F besteht, durch Pressagglomeration, bei der der höchste angewandte Pressdruck P1 ist, zu Agglomeraten A verdichtet, deren Längstausdehnung $\geq 3$ mm beträgt;

- in der Verfahrensstufe C) die Agglomerate A zerkleinert und das bei der Zerkleinerung gebildete körnige Gut durch Sieben in ein Pulver P, dessen Partikeldurchmesser $d^P \leq 2$ mm und zu wenigstens 90 Gew.-%, bezogen auf das Gewicht des Pulvers P, $\geq 160\ \mu m$ betragen, als Siebüberkorn, und in Feinkorn F als Siebunterkorri auftrennt und das Feinkorn F kontinuierlich oder absatzweise zur Erzeugung von feinteiligem Gemisch M* in die Verfahrensstufe B rückführt;

- in der Verfahrensstufe D) aus dem in selbige geförderten Pulver P oder aus einem Gemisch P*, bestehend aus dem in die Verfahrensstufe D) geförderten Pulver P und Formgebungshilfsmittel, durch Pressagglomeration, bei der der angewandte höchste Pressdruck P2 beträgt und die Beziehung P2 $\geq 2 \cdot$ P1 erfüllt, mit der Maßgabe geometrische Formkörper V erzeugt, dass

- bei der Förderung des Pulvers P in die Verfahrensstufe D) und beim Einmischen von Formgebungshilfsmittel in das Pulver P insgesamt bei wenigstens 40 Gew.-% der Partikel des Pulvers P, bezogen auf dessen Gewicht, ein Partikeldurchmesser $d^P \geq 160\ \mu m$ erhalten bleibt; und

- in der Verfahrensstufe E) wenigstens eine Teilmenge der Formkörper V bei erhöhter Temperatur unter Erhalt der geometrischen Katalysatorformkörper K thermisch behandelt, **dadurch gekennzeichnet, dass** man
- vorab der Verfahrensstufe E) die in der Verfahrensstufe D) erzeugten Formkörper V in einer zusätzlichen Trennstufe als Verfahrensstufe F) in nicht intakte Formkörper V- sowie in intakte Formkörper V⁺ auftrennt, die Formkörper V⁺ der Verfahrensstufe E) zuführt und

- in der Verfahrensstufe G) nicht intakte Formkörper V- unter Ausbildung eines feinteiligen Haufwerks H, dessen Partikeldurchmesser $d_{50}^H$ die Bedingung $1\ \mu m \leq d_{50}^H \leq 150\ \mu m$ erfüllt und das zu höchstens 10 Gew.-% seines Gesamtgewichts Partikel mit einem Partikeldurchmesser $d^H \geq 160\ \mu m$ enthält, zerkleinert und das feinteilige Haufwerk H kontinuierlich oder absatzweise zur zusätzlichen Einarbeitung in das feinteilige Gemisch M* mit der Maßgabe in die Verfahrensstufe B) rückführt, dass der Gehalt des feinteiligen Gemischs M* an feinteiligem Haufwerk H, bezogen auf das Gesamtgewicht des feinteiligen Gemischs M*, 20 Gew.-% nicht überschreitet.

2. Verfahren nach Anspruch 1, **dadurch gekennzeichnet, dass** die Auftrennung der Formkörper V in der Verfahrensstufe F durch eine Siebtrennung vorgenommen wird, bei der die intakten Formkörper V⁺ als Siebrückstand verbleiben und der Siebdurchgang die Bruchstücke von nicht intakten Formkörpern V- umfasst.

3. Verfahren nach Anspruch 1 oder 2, **dadurch gekennzeichnet, dass** in wenigstens einer der verschiedenen Verfahrensstufen B) bis G) die in dieser wenigstens einen Verfahrensstufe herrschende Gasatmosphäre abgesaugt und wenigstens einer mechanischen Trennoperation unterworfen wird, mit der in der Gasatmosphäre enthaltene Feststoffpartikel FP abgetrennt, und kontinuierlich oder absatzweise in die Verfahrensstufe B) rückgeführt und mit der Maßgabe in das feinteilige Gemisch M* eingearbeitet werden, dass der Gehalt des Gemischs M* an solchen rückgeführten Feststoffpartikeln FP, bezogen auf das Gesamtgewicht des Gemischs M*, 10 Gew.-% nicht überschreitet.

4. Verfahren nach Anspruch 3, **dadurch gekennzeichnet, dass** der Gehalt des Gemischs M* an rückgeführten Feststoffpartikeln FP 5 Gew.-% nicht überschreitet.

**5.** Verfahren nach Anspruch 3 oder 4, **dadurch gekennzeichnet, dass** die wenigstens eine mechanische Trennoperation die Filtration ist.

**6.** Verfahren nach einem der Ansprüche 1 bis 5, **dadurch gekennzeichnet, dass** die geometrischen Katalysatorformkörper K als Aktivmasse ein Multielementoxid enthalten, in welchem das Element Mo dasjenige von Sauerstoff verschiedene Element E ist, das von allen von Sauerstoff verschiedenen Elementen E des Multielementoxids mit der größten molaren Häufigkeit im Multielementoxid enthalten ist.

**7.** Verfahren nach einem der Ansprüche 1 bis 6, **dadurch gekennzeichnet, dass** die geometrischen Katalysatorformkörper K als Aktivmasse ein Multielementoxid enthalten, das, bezogen auf die molare Gesamtmenge seiner von molekularem Sauerstoff verschiedenen Elemente E, zu wenigstens 30 mol-% das Element Mo enthält.

**8.** Verfahren nach einem der Ansprüche 1 bis 7, **dadurch gekennzeichnet, dass** die geometrischen Katalysatorformkörper K als Aktivmasse ein Multielementoxid enthalten, das wenigstens eines der Alkalimetalle K, Na und Cs als Element E enthält.

**9.** Verfahren nach einem der Ansprüche 1 bis 8, **dadurch gekennzeichnet, dass** $1 \ \mu m \leq d_{50}^{M} \leq 100 \ \mu m$ ist.

**10.** Verfahren nach einem der Ansprüche 1 bis 9, **dadurch gekennzeichnet, dass** im Rahmen der Erzeugung der feinteiligen Mischung M wenigstens drei voneinander verschiedene Quellen Q von voneinander verschiedenen Elementen E in wässrigem Medium miteinander vermischt werden.

**11.** Verfahren nach einem der Ansprüche 1 bis 10, **dadurch gekennzeichnet, dass** das zu geometrischen Formkörpern V pressagglomerierte Pulver P oder P* wenigstens ein Ion aus der Gruppe bestehend aus Ammonium-, Formiat-, Acetat- und Nitrationen enthält, und der Gehalt $G^W$ des Pulvers P oder P* an Wasser, bezogen auf das Gesamtgewicht der in ihm enthaltenen Gesamtmenge an Ammonium-, Formiat-, Acetat- und Nitrationen ≤ 60 Gew.-% beträgt.

**12.** Verfahren nach Anspruch 11, **dadurch gekennzeichnet, dass** $G^W$ ≤ 50 Gew.-% beträgt.

**13.** Verfahren nach Anspruch 11, **dadurch gekennzeichnet, dass** $G^W$ ≤ 40 Gew.-% beträgt.

**14.** Verfahren nach einem der Ansprüche 11 bis 13, **dadurch gekennzeichnet, dass** das Pulver P oder P* sauer ist.

**15.** Verfahren nach Anspruch 14, **dadurch gekennzeichnet, dass** das Pulver P oder P* Nitrationen enthält.

**16.** Verfahren nach einem der Ansprüche 1 bis 15, **dadurch gekennzeichnet, dass** die Längstausdehnung L der in der Verfahrensstufe B) erzeugten Agglomerate ≥ 0,5 cm beträgt.

**17.** Verfahren nach einem der Ansprüche 1 bis 15. **dadurch gekennzeichnet, dass** die Längstausdehnung L der in der Verfahrensstufe B) erzeugten Agglomerate ≥ 1 cm beträgt.

**18.** Verfahren nach einem der Ansprüche 1 bis 17. **dadurch gekennzeichnet, dass** die Pressagglomeration in der Verfahrensstufe B) mit einer Walzenpresse durchgeführt wird.

**19.** Verfahren nach einem der Ansprüche 1 bis 18, **dadurch gekennzeichnet, dass** P2 ≥ 3 • P1 ist.

**20.** Verfahren nach einem der Ansprüche 1 bis 18, **dadurch gekennzeichnet, dass** P2 ≥ 4 • P1 ist.

**21.** Verfahren nach einem der Ansprüche 1 bis 20, **dadurch gekennzeichnet, dass** der Formkörper V eine ringförmige Geometrie aufweist mit einem Außendurchmesser A und einer Höhe H von 2 bis 10 mm und einer Wandstärke von 1 bis 3 mm.

**22.** Verfahren nach einem der Ansprüche 1 bis 21, **dadurch gekennzeichnet, dass** die Partikeldurchmesser $d^P$ ≤ 1 mm betragen.

**23.** Verfahren nach einem der Ansprüche 1 bis 22, **dadurch gekennzeichnet, dass** bei wenigstens 90 Gew.-%, bezogen auf das Gesamtgewicht des Pulvers P, der Partikel des Pulvers P der Partikeldurchmesser $d^P \geq 200 \mu$m ist.

24. Verfahren nach einem der Ansprüche 1 bis 23, **dadurch gekennzeichnet, dass** die Partikeldurchmesser $d^H \leq 100$ $\mu$m betragen.

25. Verfahren nach einem der Ansprüche 1 bis 24, **dadurch gekennzeichnet, dass** der Pressdruck P1 0,1 bis 5 k N/cm² ist.

26. Verfahren nach einem der Ansprüche 1 bis 25, **dadurch gekennzeichnet, dass** die Förderung des Pulvers P aus der Verfahrensstufe C) in die Verfahrensstufe D) saugpneumatisch oder druckpneumatisch erfolgt.

27. Verfahren nach einem der Ansprüche 1 bis 26, **dadurch gekennzeichnet, dass** der Gehalt des feinteiligen Gemisches M* an feinteiligem Haufwerk H, bezogen auf das Gesamtgewicht des feinteiligen Gemisches M*, 15 Gew.-% nicht überschreitet.

28. Verfahren nach einem der Ansprüche 1 bis 26, **dadurch gekennzeichnet, dass** der Gehalt des feinteiligen Gemisches M* an feinteiligem Haufwerk H, bezogen auf das Gesamtgewicht des feinteiligen Gemischs M*, 10 Gew.-% nicht überschreitet.

29. Verfahren nach einem der Ansprüche 1 bis 28, **dadurch gekennzeichnet, dass** der Gehalt des feinteiligen Gemisches M* an feinteiligem Haufwerk H, bezogen auf das Gesamtgewicht des feinteiligen Gemischs M*, wenigstens 1 Gew.-% beträgt.

30. Verfahren nach einem der Ansprüche 1 bis 28, **dadurch gekennzeichnet, dass** der Gehalt des feinteiligen Gemisches M* an feinteiligem Haufwerk H, bezogen auf das Gesamtgewicht des feinteiligen Gemischs M*, wenigstens 3 Gew.-% beträgt.

31. Verfahren nach einem der Ansprüche 1 bis 28, **dadurch gekennzeichnet, dass** der Gehalt des feinteiligen Gemisches M* an feinteiligem Haufwerk H, bezogen auf das Gesamtgewicht des feinteiligen Gemischs M*, wenigstens 5 Gew.-% beträgt.

32. Verfahren nach einem der Ansprüche 1 bis 31, **dadurch gekennzeichnet, dass** sich an ein Verfahren nach einem der Ansprüche 1 bis 31 ein Verfahren der heterogen katalysierten partiellen Gasphasenoxidation einer organischen Verbindung anschließt, bei dem als Katalysator wenigstens ein geometrischer Katalysatorformkörper K erhalten nach dem Verfahren gemäß Anspruch 1 bis 31 eingesetzt wird.

33. Verfahren nach Anspruch 32, **dadurch gekennzeichnet, dass** das Verfahren der heterogen katalysierten partiellen Gasphasenoxidation die Partialoxidation von Propen zu Acrolein, von iso-Buten zu Methacrolein, von Propen zu Acrylnitril, von iso-Buten zu Methacrylnitril, von Acrolein zu Acrylsäure oder von Methacrolein zu Methacrylsäure ist.

## Claims

1. A process for continuously producing geometric shaped catalyst bodies K which comprise, as an active material, a multielement oxide which comprises, as elements E other than oxygen, the element Mo, at least one of the two elements Bi and V, and at least one further element from the group consisting of Co, Ni, Fe, Cu and the alkali metals, in process stages A) to G), in which

- in process stage A), with the aid of sources Q of the elements E, a finely divided mixture M is obtained with the proviso that at most 10% by weight of the total weight of the particles present in the finely divided mixture M have a particle diameter of $d^M \geq 160 \mu$m and the particle diameter $d_{50}^M$ of the particles of the finely divided mixture M satisfies the condition $1 \mu m \leq d_{50}^M \leq 150 \mu m$;
- in process stage B), the finely divided mixture M*, which consists either only of the finely divided mixture M or of a mixture of the finely divided mixture M and fines F which are obtained in the next process stage C) and are recycled into process stage B) continuously or batchwise from process stage C), is compacted by press agglomeration in which the maximum pressure applied is P1 to agglomerates A whose longest dimension is $\geq 3$ mm;
- in process stage C), the agglomerates A are comminuted and the particulate material formed in the comminution is separated by sieving into a powder P whose particle diameters $d^P$ are $\leq 2$ mm and, to an extent of at least 90% by weight, based on the weight of the powder P, 160 $\mu$m, as sieve oversize, and into fines F as sieve undersize, and the fines F are recycled continuously or batchwise into process stage B to obtain finely divided mixture M*;

- in process stage D), the powder P conducted into it or a mixture P* consisting of the powder P conducted into process stage D) and shaping assistants is used to obtain, by press agglomeration in which the maximum pressure applied is P2 and satisfies the relationship $P2 \geq 2 \cdot P1$, geometric shaped bodies V with the proviso that

- when the powder P is conveyed into process stage D) and when shaping assistants are mixed into the powder P, a particle diameter $d^P \geq 160$ $\mu$m is maintained overall in at least 40% by weight of the particles of the powder P, based on the weight thereof; and

- in process stage E), at least a portion of the shaped bodies V is treated thermally at elevated temperature to obtain the geometric shaped catalyst bodies K, wherein

- prior to process stage E), the shaped bodies V obtained in process stage D) are separated in an additional separation stage as process stage F) into non-intact shaped bodies V- and into intact shaped bodies V$^+$, the shaped bodies V$^+$ are fed to process stage E) and

- in process stage G), non-intact shaped bodies V$^-$ are comminuted to form a finely divided aggregate H whose particle diameter $d_{50}^H$ satisfies the condition $1 \ \mu m \ \leq d_{50}^H \ \leq \ 150 \ \mu m$ and which comprises particles having a particle diameter $d^H \geq 160$ $\mu$m to an extent of at most 10% by weight of its total weight, and the finely divided aggregate H is recycled continuously or batchwise to the additional incorporation into the finely divided mixture M* into process stage B) with the proviso that the content of finely divided aggregate H in the finely divided mixture M*, based on the total weight of the finely divided mixture M*, does not exceed 20% by weight.

2. The process according to claim 1, wherein the separation of the shaped bodies V in process stage F is undertaken by a sieve separation in which the intact shaped bodies V$^+$ remain as the sieve residue and the fragments of non-intact shaped bodies V$^-$ pass through the sieve.

3. The process according to claim 1 or 2, wherein, in at least one of the different process stages B) to G), the gas atmosphere which exists in this at least one process stage is sucked out and subjected to at least one mechanical separating operation, with which solid particles FP present in the gas atmosphere are removed, and recycled continuously or batchwise into process stage B) and incorporated into the finely divided mixture M* with the proviso that the content of such recycled solid particles FP in the mixture M*, based on the total weight of the mixture M*, does not exceed 10% by weight.

4. The process according to claim 3, wherein the content of recycled solid particles FP in the mixture M* does not exceed 5% by weight.

5. The process according to claim 3 or 4, wherein the at least one mechanical separating operation is filtration.

6. The process according to any one of claims 1 to 5, wherein the geometric shaped catalyst bodies K comprise, as an active material, a multielement oxide in which the element Mo is that element E other than oxygen which, of all elements E other than oxygen in the multielement oxide, is present with the greatest molar frequency in the multielement oxide.

7. The process according to any one of claims 1 to 6, wherein the geometric shaped catalyst bodies K comprise, as an active material, a multielement oxide which, based on the molar total amount of its elements E other than molecular oxygen, comprises the element Mo to an extent of at least 30 mol%.

8. The process according to any one of claims 1 to 7, wherein the geometric shaped catalyst bodies K comprise, as an active material, a multielement oxide which comprises at least one of the alkali metals K, Na and Cs as the element E.

9. The process according to any one of claims 1 to 8, wherein $1 \ \mu m \ \leq d_{50}^M \ \leq \ 100 \ \mu m .$

10. The process according to any one of claims 1 to 9, wherein, in the course of obtaining the finely divided mixture M, at least three different sources Q of different elements E are mixed with one another in an aqueous medium.

11. The process according to any one of claims 1 to 10, wherein the powder P or P* press agglomerated to geometric shaped bodies V comprises at least one ion from the group consisting of ammonium, formate, acetate and nitrate ions, and the water content $G^W$ of the powder P or P*, based on the total weight of the total amount of ammonium, formate, acetate and nitrate ions present therein, is $\leq 60\%$ by weight.

**12.** The process according to claim 11, wherein $G^W \leq 50\%$ by weight.

**13.** The process according to claim 11, wherein $G^W \leq 40\%$ by weight.

**14.** The process according to any one of claims 11 to 13, wherein the powder P or P* is acidic.

**15.** The process according to claim 14, wherein the powder P or P* comprises nitrate ions.

**16.** The process according to any one of claims 1 to 15, wherein the longest dimension L of the agglomerates obtained in process stage B) is $\geq 0.5$ cm.

**17.** The process according to any one of claims 1 to 15, wherein the longest dimension L of the agglomerates obtained in process stage B) is $\geq 1$ cm.

**18.** The process according to any one of claims 1 to 17, wherein the press agglomeration in process stage B) is performed with a roller press.

**19.** The process according to any one of claims 1 to 18, wherein $P2 \geq 3 \cdot pa1$.

**20.** The process according to any one of claims 1 to 18, wherein $P2 \geq 4 \cdot P1$.

**21.** The process according to any one of claims 1 to 20, wherein the shaped body V has an annular geometry with an external diameter A and a height H of 2 to 10 mm and a wall thickness of 1 to 3 mm.

**22.** The process according to any one of claims 1 to 21, wherein the particle diameters $d^P$ are $\leq 1$ mm.

**23.** The process according to any one of claims 1 to 22, wherein the particle diameter $d^P$ is $\geq 200$ $\mu$m in at least 90% by weight, based on the total weight of the powder P, of the particles of the powder P.

**24.** The process according to any one of claims 1 to 23, wherein the particle diameters $d^H$ are $\leq 100$ $\mu$m.

**25.** The process according to any one of claims 1 to 24, wherein the pressure P1 is 0.1 to 5 kN/cm$^2$.

**26.** The process according to any one of claims 1 to 25, wherein the powder P is conveyed from process stage C) into process stage D) by suction pneumatics or pressure pneumatics.

**27.** The process according to any of claims 1 to 26, wherein the content in the finely divided mixture M* of finely divided aggregate H, based on the total weight of the finely divided mixture M*, does not exceed 15% by weight.

**28.** The process according to any of claims 1 to 26, wherein the content in the finely divided mixture M* of finely divided aggregate H, based on the total weight of the finely divided mixture M*, does not exceed 10% by weight.

**29.** The process according to any of claims 1 to 28, wherein the content in the finely divided mixture M* of finely divided aggregate H, based on the total weight of the finely divided mixture M*, is at least 1% by weight.

**30.** The process according to any of claims 1 to 28, wherein the content in the finely divided mixture M* of finely divided aggregate H, based on the total weight of the finely divided mixture M*, is at least 3% by weight.

**31.** The process according to any of claims 1 to 28, wherein the content in the finely divided mixture M* of finely divided aggregate H, based on the total weight of the finely divided mixture M*, is at least 5% by weight.

**32.** The process according to any of claims 1 to 31, wherein a process according to any of claims 1 to 31 is followed by a process for heterogeneously catalyzed partial gas phase oxidation of an organic compound, in which the catalyst used is at least one geometric shaped catalyst body K obtained by the process according to claims 1 to 31.

**33.** The process according to claim 32, wherein the process for heterogeneously catalyzed partial gas phase oxidation is the partial oxidation of propene to acrolein, of isobutene to methacrolein, of propene to acrylonitrile, of isobutene to methacrylonitrile, of acrolein to acrylic acid or of methacrolein to methacrylic acid.

## Revendications

**1.** Procédé pour la préparation continue de corps façonnés catalytiques K géométriques, qui contiennent, comme masse active, un oxyde d'éléments multiples qui contient, comme éléments E différents de l'oxygène, l'élément Mo, au moins un des deux éléments Bi et V ainsi qu'au moins un autre élément du groupe constitué par Co, Ni, Fe, Cu et les métaux alcalins, en étapes de procédé A) à G), dans lequel

- dans l'étape de procédé A), à l'aide de sources Q des éléments E, on génère un mélange M finement divisé sous réserve qu'au plus 10% en poids du poids total des particules contenues dans le mélange M finement divisé présentent un diamètre de particule $d^M \geq 160\ \mu m$ et que le diamètre de particule $d^M_{50}$ des particules du

mélange M finement divisé remplisse la condition $1\ \mu m\ \leq\ d^M_{50}\ \leq\ 150\ \mu m$ ;

- dans l'étape de procédé B), on compacte le mélange M* finement divisé, qui est constitué soit uniquement par le mélange M finement divisé, soit par un mélange du mélange M finement divisé et des fines F produites dans l'étape de procédé C) consécutive et recyclées par lots ou en continu de l'étape de procédé C) dans l'étape de procédé B), par agglomération par compression, lors de laquelle la pression de compression la plus élevée utilisée est P1, en agglomérats A dont la dimension la plus longue est $\geq 3$ mm ;
- dans l'étape de procédé C), on broie les agglomérats A et on sépare le produit granuleux formé lors du broyage par tamisage en une poudre P, dont les diamètres de particule $d^P$ sont $\leq 2$ mm et à raison d'au moins 90% en poids, par rapport au poids de la poudre P, $\geq 160\ \mu m$, comme refus de tamis, et en fines F, comme tamisat inférieur et on recycle les fines F, en continu ou par lots, dans l'étape de procédé B) pour la génération du mélange M* finement divisé ;
- dans l'étape de procédé D), on génère, à partir de la poudre P introduite dans celle-ci ou à partir d'un mélange P*, constitué par la poudre P introduite dans l'étape de procédé D) et par des adjuvants de façonnage, par agglomération par compression, lors de laquelle la pression de compression la plus élevée utilisée est P2 et la relation $P2 \geq 2 \cdot P1$ est satisfaite, des corps façonnés V sous réserve que
- lors de l'introduction de la poudre P dans l'étape de procédé D) et lors du mélange d'adjuvants de façonnage dans la poudre P, un diamètre de particule $d^P \geq 160\ \mu m$ soit conservé au total pour au moins 40% en poids des particules de la poudre P, par rapport à son poids ; et
- dans l'étape de procédé E), on traite au moins une quantité partielle des corps façonnés V thermiquement à température augmentée avec obtention des corps façonnés catalytiques K géométriques, **caractérisé en ce qu'**on
- sépare, avant l'étape de procédé E), les corps façonnés V générés dans l'étape de procédé D), dans une étape de séparation supplémentaire en tant qu'étape de procédé F), en corps façonnés V⁻ non intacts et en corps façonnés V⁺ intacts, et on introduit les corps façonnés V⁺ dans l'étape de procédé E) et
- dans l'étape de procédé G), on broie les corps façonnés V⁻ non intacts avec formation de débris H finement divisés, dont le diamètre de particule $d^H_{50}$ satisfait à la condition $1\ \mu m\ \leq\ d^H_{50}\ \leq\ 150\ \mu m$ et qui contient, à raison d'au plus 10% en poids de son poids total, de particules présentant un diamètre de particule $d^H \geq 160\ \mu m$, et on recycle les débris H finement divisés en continu ou par lots, en vue d'une incorporation supplémentaire dans le mélange M* finement divisé, dans l'étape de procédé B) sous réserve que la teneur du mélange M* finement divisé en débris H finement divisés, par rapport au poids total du mélange M* finement divisé, ne dépasse pas 20% en poids.

**2.** Procédé selon la revendication 1, **caractérisé en ce que** la séparation des corps façonnés V dans l'étape de procédé F) est réalisée par une séparation par tamisage lors de laquelle les corps façonnés V⁺ intacts restent comme refus de tamis et le tamisat comprend les fragments de corps façonnés V⁻ non intacts.

**3.** Procédé selon la revendication 1 ou 2, **caractérisé en ce que** dans au moins une des différentes étapes de procédé B) à G), l'atmosphère gazeuse qui règne dans cette au moins une étape de procédé est aspirée et soumise à au moins une opération de séparation mécanique, par laquelle des particules solides FP contenues dans l'atmosphère gazeuse sont séparées et recyclées en continu ou par lots dans l'étape de procédé B) et incorporées dans le mélange M* finement divisé, sous réserve que la teneur du mélange M* en ces particules solides FP recyclées, par rapport au poids total du mélange M*, ne dépasse pas 10% en poids.

**4.** Procédé selon la revendication 3, **caractérisé en ce que** la teneur du mélange M* en particules solides FP recyclées ne dépasse pas 5% en poids.

**5.** Procédé selon la revendication 3 ou 4, **caractérisé en ce que** ladite au moins une opération de séparation mécanique est une filtration.

**6.** Procédé selon l'une quelconque des revendications 1 à 5, **caractérisé en ce que** les corps façonnés catalytiques K géométriques contiennent, comme masse active, un oxyde d'éléments multiples, dans lequel l'élément Mo est l'élément E différent de l'oxygène qui est contenu dans l'oxyde d'éléments multiples à la fréquence molaire la plus élevée parmi tous les éléments E différents de l'oxygène de l'oxyde d'éléments multiples.

**7.** Procédé selon l'une quelconque des revendications 1 à 6, **caractérisé en ce que** les corps façonnés catalytiques K géométriques contiennent, comme masse active, un oxyde d'éléments multiples qui contient, par rapport à la quantité molaire totale de ses éléments E différents de l'oxygène moléculaire, à raison d'au moins 30% en mole l'élément Mo.

**8.** Procédé selon l'une quelconque des revendications 1 à 7, **caractérisé en ce que** les corps façonnés catalytiques K géométriques contiennent, comme masse active, un oxyde d'éléments multiples qui contient au moins un des métaux alcalins K, Na et Cs comme élément E.

**9.** Procédé selon l'une quelconque des revendications 1 à 8, **caractérisé en ce que** $1 \ \mu m \leq d^M_{50} \leq 100 \ \mu m$.

**10.** Procédé selon l'une quelconque des revendications 1 à 9, **caractérisé en ce que** dans le cadre de la génération du mélange M finement divisé, on mélange les unes avec les autres au moins trois sources Q, différentes les unes des autres, d'éléments E, différents les uns des autres, en milieu aqueux.

**11.** Procédé selon l'une quelconque des revendications 1 à 10, **caractérisé en ce que** la poudre P ou P* agglomérée par compression en corps façonnés V géométriques contient au moins un ion du groupe constitué par les ions d'ammonium, de formiate, d'acétate et de nitrate, et la teneur $G^W$ de la poudre P ou P* en eau, par rapport au poids total de la quantité totale d'ions d'ammonium, de formiate, d'acétate et de nitrate qu'elle contient, est $\leq 60\%$ en poids.

**12.** Procédé selon la revendication 11, **caractérisé en ce que** $G^W$ est $\leq 50\%$ en poids.

**13.** Procédé selon la revendication 11, **caractérisé en ce que** $G^W$ est $\leq 40\%$ en poids.

**14.** Procédé selon l'une quelconque des revendications 11 à 13, **caractérisé en ce que** la poudre P ou P* est acide.

**15.** Procédé selon la revendication 14, **caractérisé en ce que** la poudre P ou P* contient des ions nitrate.

**16.** Procédé selon l'une quelconque des revendications 1 à 15, **caractérisé en ce que** la dimension L la plus longue des agglomérats générés dans l'étape de procédé B) est $\geq 0,5$ cm.

**17.** Procédé selon l'une quelconque des revendications 1 à 15, **caractérisé en ce que** la dimension L la plus longue des agglomérats générés dans l'étape de procédé B) est $\geq 1$ cm.

**18.** Procédé selon l'une quelconque des revendications 1 à 17, **caractérisé en ce que** l'agglomération par compression dans l'étape de procédé B) est réalisée à l'aide d'une presse à rouleaux.

**19.** Procédé selon l'une quelconque des revendications 1 à 18, **caractérisé en ce que** P2 $\geq 3 \cdot$P1.

**20.** Procédé selon l'une quelconque des revendications 1 à 18, **caractérisé en ce que** P2 $\geq 4 \cdot$P1.

**21.** Procédé selon l'une quelconque des revendications 1 à 20, **caractérisé en ce que** le corps façonné V présente une géométrie annulaire, dotée d'un diamètre externe A et d'une hauteur H de 2 à 10 mm et d'une épaisseur de paroi de 1 à 3 mm.

22. Procédé selon l'une quelconque des revendications 1 à 21, **caractérisé en ce que** les diamètres de particule $d^P$ sont ≤ 1 mm.

23. Procédé selon l'une quelconque des revendications 1 à 22, **caractérisé en ce que**, pour au moins 90% en poids, par rapport au poids total de la poudre P, des particules de la poudre P, le diamètre de particule $d^P$ est ≥ 200 µm.

24. Procédé selon l'une quelconque des revendications 1 à 23, **caractérisé en ce que** les diamètres de particule $d^H$ sont ≤ 100 µm.

25. Procédé selon l'une quelconque des revendications 1 à 24, **caractérisé en ce que** la pression de compression P1 est de 0,1 à 5 kN/cm$^2$.

26. Procédé selon l'une quelconque des revendications 1 à 25, **caractérisé en ce que** le transport de la poudre P de l'étape de procédé C) dans l'étape de procédé D) a lieu de manière pneumatique par aspiration ou de manière pneumatique sous pression.

27. Procédé selon l'une quelconque des revendications 1 à 26, **caractérisé en ce que** la teneur du mélange M* finement divisé en débris H finement divisés, par rapport au poids total du mélange M* finement divisé, ne dépasse pas 15% en poids.

28. Procédé selon l'une quelconque des revendications 1 à 26, **caractérisé en ce que** la teneur du mélange M* finement divisé en débris H finement divisés, par rapport au poids total du mélange M* finement divisé, ne dépasse pas 10% en poids.

29. Procédé selon l'une quelconque des revendications 1 à 28, **caractérisé en ce que** la teneur du mélange M* finement divisé en débris H finement divisés, par rapport au poids total du mélange M* finement divisé, est d'au moins 1% en poids.

30. Procédé selon l'une quelconque des revendications 1 à 28, **caractérisé en ce que** la teneur du mélange M* finement divisé en débris H finement divisés, par rapport au poids total du mélange M* finement divisé, est d'au moins 3% en poids.

31. Procédé selon l'une quelconque des revendications 1 à 28, **caractérisé en ce que** la teneur du mélange M* finement divisé en débris H finement divisés, par rapport au poids total du mélange M* finement divisé, est d'au moins 5% en poids.

32. Procédé selon l'une quelconque des revendications 1 à 31, **caractérisé en ce qu'**un procédé selon l'une quelconque des revendications 1 à 31 est suivi d'un procédé d'oxydation partielle en phase gazeuse catalysée par voie hétérogène d'un composé organique, lors duquel on utilise, comme catalyseur, au moins un corps façonné catalytique K géométrique obtenu selon le procédé selon la revendication 1 à 31.

33. Procédé selon la revendication 32, **caractérisé en ce que** le procédé de l'oxydation partielle en phase gazeuse catalysée par voie hétérogène est l'oxydation partielle de propène en acroléine, d'isobutène en méthacroléine, de propène en acrylonitrile, d'isobutène en méthacrylonitrile, d'acroléine en acide acrylique ou de méthacroléine en acide méthacrylique.

**In der Beschreibung aufgeführte Patentdokumente**

- EP 184790 A
- US 20050263926 A
- JP 10029097 A
- US 7147011 B
- DE 102007028332 A
- WO 2005049200 A1
- DE 102008040093 A
- DE 102008040094 A
- WO 2008014839 A
- EP 1726358 A
- DE 10360396 A
- DE 102008042064
- DE 102008042061
- DE 102008042060
- DE 102008040093
- DE 102008040094
- WO 2005030393 A
- EP 467144 A
- EP 1060792 A
- DE 19855913 A
- WO 0168245 A
- WO 2005497012 A
- DE 02005035978 A1
- DE 102005037678 A
- WO 0378059 A
- WO 03078310 A
- DE 19922113 A
- WO 0224620 A
- WO 02062737 A
- DE 102007025869 A
- DE 102007017080 A
- US 20050131253 A
- WO 2435860 A
- WO 1117245 A
- WO 2007017431 A
- DE 102007004961 A
- DE 10046957 A
- WO 0053557 A
- WO 0053558 A
- DE 19910506 A
- EP 1106598 A
- WO 0136364 A
- DE 19927624 A
- DE 19948248 A
- DE 19948523 A
- DE 19948241 A
- EP 700714 A
- DE 10313213 A
- DE 10313209 A
- DE 102004003212 A
- DE 102005013039 A
- DE 102007003778 A
- DE 102007003778
- DE 19835247 A
- DE 10051419 A
- DE 10046672 A
- US 61122129 B

**In der Beschreibung aufgeführte Nicht-Patentliteratur**

- *Chem.-Ing.-Tech.,* 1984, vol. 56 (12), 897-907
- **PAUL SCHMIDT.** Sieben und Siebmaschinen, Grundlagen und Anwendung. Wiley VCH, 2003
- *Chem.-Ing.-Tech.,* 1984, vol. 56 (12), 902
- *Chem.-Ing.-Techn.,* 1984, vol. 56 (12), 897-907
- **PAUL SCHMIDT et al.** Sieben und Siebmaschinen. Wiley-VCH GmbH & Co. KGaA, 2003
- **H. BRUNNER ; D. SCHUTTE.** *Chem. Ing. Techn.,* 1965, vol. 89, 437